# EUROPEAN PATENT APPLICATION

(11) **EP 4 245 375 A2**
(43) Date of publication of application: **20.09.2023**
(21) Application number: 23174625.6
(22) Date of filing: 30.05.2018
(51) Int. Cl.: A61P 35/00

(54) **COMPOSITIONS COMPRISING A COMBINATION OF AN ANTI-LAG-3 ANTIBODY, A PD-1 PATHWAY INHIBITOR, AND AN IMMUNOTHERAPEUTIC AGENT**

(30) Priority: 30.05.2017 US 201762512618 P; 01.06.2017 US 201762513812 P
(62) Divisional of application: 18734678.8
(71) Applicant: Bristol-Myers Squibb Company, Princeton, NJ 08543 (US)
(72) Inventor: KORMAN, Alan J., Piedmont, 94611 (US); LONBERG, Nils, Woodside, 94062 (US); SELBY, Mark J., San Francisco, 94131 (US); JACKSON, Jeffrey, Princeton, 08543 (US)
(74) Representative: Mewburn Ellis LLP

(57) **Abstract**

Provided are methods for clinical treatment of malignant tumors (e.g., advanced solid tumors) using a combination of an anti-LAG-3 antibody, an anti-PD-1 antibody, and an immunotherapeutic agent.

## Description

### CROSS-REFERENCE TO RELATED APPLICATIONS

This application claims priority to U.S. Provisional Application Nos. 62/512,618, filed May 30, 2017 and 62/513,812, filed June 1, 2017, which are incorporated herein by reference in their entireties.

### FIELD OF THE INVENTION

The present disclosure provides methods for treating a malignant tumor (e.g., advanced solid tumors) with a pharmaceutical composition comprising a combination of an anti-LAG-3 antibody, a PD-1 pathway inhibitor, and an immunotherapeutic agent.

### BACKGROUND OF THE INVENTION

Human cancers harbor numerous genetic and epigenetic alterations, generating neoantigens potentially recognizable by the immune system (Sjoblom et al., Science 314(5797):268-274 (2006)). The adaptive immune system, comprised of T and B lymphocytes, has powerful anti-cancer potential, with a broad capacity and exquisite specificity to respond to diverse tumor antigens. Further, the immune system demonstrates considerable plasticity and a memory component. The successful harnessing of all these attributes of the adaptive immune system would make immunotherapy unique among all cancer treatment modalities.

Until recently, cancer immunotherapy had focused substantial effort on approaches that enhance anti-tumor immune responses by adoptive-transfer of activated effector cells, immunization against relevant antigens, or providing non-specific immune-stimulatory agents such as cytokines. In the past decade, however, intensive efforts to develop specific immune checkpoint pathway inhibitors have begun to provide new immunotherapeutic approaches for treating cancer, including the development of an antibody (antibody), ipilimumab (YERVOY^{®}), that binds to and inhibits CTLA-4 for the treatment of patients with advanced melanoma (Hodi et al., N Engl J Med 363:711-723 (2010)) and the development of antibodies such as nivolumab and pembrolizumab (formerly lambrolizumab; USAN Council Statement, (2013)) that bind specifically to the Programmed Death -1 (PD-1) receptor and block the inhibitory PD-1/PD-1 ligand pathway (Topalian et al., N Engl J Med 366:2443-54 (2012a); Topalian et al., Curr Opin Immunol 24:207-12 (2012b); Topalian et al., J Clin Oncol 32(10): 1020-30 (2014); Hamid et al., N Engl J Med 369:134-144 (2013); Hamid and Carvajal, Expert Opin Biol Ther 13(6):847-61 (2013); and McDermott and Atkins, Cancer 2(5):662-73 (2013)).

Immune tolerance observed in the setting of tumor development and tumor recurrence, however, seems to be mediated by the co-expression of various T cell negative regulatory receptors, not solely from LAG-3. Data from chronic viral infection models (Blackburn et al., Nat. Immunol 10:29-37 (2009), Grosso et al., J. Clin. Invest. 117:3383-3392 (2007), and Lyford-Pike et al., Cancer Res. 73(6):1733-41 (2013)), knock-out mice (Woo et al., Cancer Res. 72:917-927 (2012); Okazaki et al., J. Exp Med. 208:395-407 (2011), and Bettini et al., J. Immunol. 187:3493-3498 (2011)), tumor recurrence models (Goding et al., J. Immunol. 190(9):4899-4909 (2013)) and, to a more limited extent, human cancer patients (Matsuzaki et al., Proc. Natl. Acad. Sci., USA. 107:7875-7880 (2010), and Gandhi M K, et al., Blood. 108:2280-2289 (2006)) support a model wherein T cells that are continuously exposed to antigen become progressively inactivated through a process termed "exhaustion." Exhausted T cells are characterized by the expression of T cell negative regulatory receptors, predominantly CTLA-4, PD-1, and LAG-3, whose action is to limit the cell's ability to proliferate, produce cytokines, and kill target cells and/or to increase Treg activity. Accordingly, a combination therapy comprising an anti-PD-1 antibody and an anti-LAG-3 antibody has had promising results in certain types of cancers. (U.S. Publ. No. 2016/0222116 A1).

Lymphocyte activation gene-3 (LAG-3; CD223) is a type I transmembrane protein that is expressed on the cell surface of activated CD4+ and CD8+ T cells and subsets of NK and dendritic cells (Triebel et al., J. Exp. Med. 171:1393-1405 (1990); Workman et al., J. Immunol. 182(4):1885-91 (2009)). LAG-3 is closely related to CD4, which is a coreceptor for T helper cell activation. Both molecules have 4 extracellular Ig-like domains and require binding to their ligand, major histocompatibility complex (MHC) class II, for their functional activity. In contrast to CD4, LAG-3 is only expressed on the cell surface of activated T cells and its cleavage from the cell surface terminates LAG-3 signaling. LAG-3 can also be found as a soluble protein but it does not bind to MHC class II and its function is unknown.

It has been reported that LAG-3 plays an important role in promoting regulatory T cell (Treg) activity and in negatively regulating T cell activation and proliferation (Workman et al., J. Immunol. 174:688-695 (2005)). Both natural and induced Treg express increased LAG-3, which is required for their maximal suppressive function (Camisaschi et al., J. Immunol. 184:6545-6551 (2010) and Huang et al., Immunity. 21:503-513 (2004)). Furthermore, ectopic expression of LAG-3 on CD4+ effector T cells reduced their proliferative capacity and conferred on them regulatory potential against third party T cells (Huang et al., Immunity. 21:503-513 (2004)). Recent studies have also shown that high LAG-3 expression on exhausted lymphocytic choriomeningitis virus (LCMV)-specific CD8+ T cells contributes to their unresponsive state and limits CD8+ T cell antitumor responses (Blackburn et al., Nat. Immunol. 10:29-37 (2009) and Grosso et al., J. Clin. Invest. 117:3383-3392 (2007)). In fact, LAG-3 maintained tolerance to self and tumor antigens via direct effects on CD8+ T cells in 2 murine models (Grosso et al., J. Clin. Invest. 117:3383-3392 (2007)).

Programmed Cell Death 1 (PD-1) is a cell surface signaling receptor that plays a critical role in the regulation of T cell activation and tolerance (Keir et al., Annu Rev Immunol 26:677-704 (2008)). It is a type I transmembrane protein and together with BTLA, CTLA-4, ICOS and CD28, comprise the CD28 family of T cell co-stimulatory receptors. PD-1 is primarily expressed on activated T cells, B cells, and myeloid cells (Dong et al., Nat Med. 5: 1365-1369 (1999)). It is also expressed on natural killer (NK) cells (Terme et al., Cancer Res 71:5393-5399 (2011)). Binding of PD-1 by its ligands, PD-L1 and PD-L2, results in phosphorylation of the tyrosine residue in the proximal intracellular immune receptor tyrosine inhibitory domain, followed by recruitment of the phosphatase SHP-2, eventually resulting in down-regulation of T cell activation. One important role of PD-1 is to limit the activity of T cells in peripheral tissues at the time of an inflammatory response to infection, thus limiting the development of autoimmunity (Pardoll Nat Rev Cancer 12:252-264 (2012)). Evidence of this negative regulatory role comes from the finding that PD-1-deficient mice develop lupus-like autoimmune diseases including arthritis and nephritis, along with cardiomyopathy (Nishimura H, et al., Immunity, 1999; 11:141-151; and Nishimura H, et al., Science, 2001; 291:319-322). In the tumor setting, the consequence is the development of immune resistance within the tumor microenvironment. PD-1 is highly expressed on tumor-infiltrating lymphocytes, and its ligands are up-regulated on the cell surface of many different tumors (Dong H, et al., Nat Med 2002; 8:793-800). Multiple murine cancer models have demonstrated that binding of ligand to PD-1 results in immune evasion. In addition, blockade of this interaction results in anti-tumor activity (Topalian S L, et al. NEJM 2012; 366(26):2443-2454; Hamid O, et al., NEJM 2013; 369:134-144). Moreover, it has been shown that inhibition of the PD-1/PD-L1 interaction mediates potent antitumor activity in preclinical models (U.S. Pat. Nos. 8,008,449 and 7,943,743).

Patients with certain malignant tumors (e.g., metastatic or refractory solid tumors) have very poor prognosis (Rosenberg S A, et al., Cancer immunotherapy in Cancer: Principles & Practice of Oncology (Eds DeVita V T, Lawrence T S and Rosenberg S A) 2011; 332-344 (Lippincott Williams & Wilkins, Philadelphia Pa.)). Despite advances in multimodal therapy, increases in overall survival in this patient population have been limited. Accordingly, it is an object of the present invention to provide improved methods (e.g., a composition comprising a combination of an anti-PD-1 antibody, an anti-LAG-3 antibody, and an immunotherapeutic agent) for treating subjects with such tumors (e.g., advanced refractory solid tumors).

### SUMMARY OF THE INVENTION

The present disclosure provides a method for treating a subject afflicted with a malignant tumor comprising administering to the subject a therapeutically effective amount of (a) LAG-3 inhibitor, (b) a PD-1 pathway inhibitor; and (c) an immunotherapeutic agent, in combination.

In certain embodiments, the LAG-3 inhibitor is an anti-LAG-3 antibody or an antigen binding fragment thereof. In one embodiment, the anti-LAG-3 antibody is a bispecific antibody. In another embodiment, wherein the anti-LAG-3 antibody or antigen binding fragment thereof comprises (a) a heavy chain variable region CDR1 comprising the sequence set forth in SEQ ID NO:7; (b) a heavy chain variable region CDR2 comprising the sequence set forth in SEQ ID NO:8; (c) a heavy chain variable region CDR3 comprising the sequence set forth in SEQ ID NO:9; (d) a light chain variable region CDR1 comprising the sequence set forth in SEQ ID NO:10; (e) a light chain variable region CDR2 comprising the sequence set forth in SEQ ID NO: 11; and (f) a light chain variable region CDR3 comprising the sequence set forth in SEQ ID NO:12. In some embodiments, the anti-LAG-3 antibody or antigen binding fragment thereof comprises heavy and light chain variable regions comprising the sequences set forth in SEQ ID NOs:3 and 5, respectively. In one embodiment, the anti-LAG-3 antibody is BMS 986016, MK-4280 (28G-10), REGN3767, GSK2831781, IMP731 (H5L7BW), BAP050, IMP-701 (LAG-5250), IMP321, TSR-033, LAG525, BI 754111, or FS-118.

In certain embodiments, the LAG-3 inhibitor is a soluble LAG-3 polypeptide. In one embodiment, the soluble LAG-3 polypeptide is a fusion polypeptide. In another embodiment, soluble LAG-3 polypeptide comprises a ligand binding fragment of the LAG-3 extracellular domain. In some embodiments, the ligand binding fragment of the LAG-3 extracellular domain comprises an amino acid sequence with at least 90%, at least 95%, at least 98%, or at least 99% sequence identity to SEQ ID NO:44. In certain embodiments, the soluble LAG-3 polypeptide further comprises an Fc domain.

In one embodiment, the PD-1 pathway inhibitor is an anti-PD-1 antibody or antigen binding fragment thereof. In certain embodiments, the anti-PD-1 antibody is pembrolizumab (KEYTRUDA; MK-3475), pidilizumab (CT-011), nivolumab (OPDIVO; BMS-936558), PDR001, MEDI0680 (AMP-514), TSR-042, REGN2810, JS001, AMP-224 (GSK-2661380), PF-06801591, BGB-A317, BI 754091, or SHR-1210.

In one embodiment, the PD-1 pathway inhibitor is an anti-PD-L1 antibody or antigen binding fragment thereof. In certain embodiments, the anti-PD-L1 antibody is atezolizumab (TECENTRIQ; RG7446; MPDL3280A; RO5541267), durvalumab (MEDI4736), BMS-936559, avelumab (bavencio), LY3300054, CX-072 (Proclaim-CX-072), FAZ053, KN035, orMDX-1105.

In one embodiment, the PD-1 pathway inhibitor is a small molecule drug. In certain embodiments, the PD-1 pathway inhibitor is CA-170. In another embodiment, the PD-1 pathway inhibitor is a cell based therapy. In one embodiment, the cell based therapy is a MiHA-loaded PD-L1/L2-silenced dendritic cell vaccine. In other embodiments, the cell based therapy is an anti-programmed cell death protein 1 antibody expressing pluripotent killer T lymphocyte, an autologous PD-1-targeted chimeric switch receptor-modified T lymphocyte, or a PD-1 knockout autologous T lymphocyte.

In one embodiment, the PD-1 pathway inhibitor is an anti-PD-L2 antibody or antigen binding fragment thereof. In another embodiment, the anti-PD-L2 antibody is rHIgM12B7.

In one embodiment, the PD-1 pathway inhibitor is a soluble PD-1 polypeptide. In certain embodiments, the soluble PD-1 polypeptide is a fusion polypeptide. In some embodiments, the soluble PD-1 polypeptide comprises a ligand binding fragment of the PD-1 extracellular domain. In other embodiments, the soluble PD-1 polypeptide comprises a ligand binding fragment of the PD-1 extracellular domain. In one embodiment, the ligand binding fragment of the PD-1 extracellular domain comprises an amino acid sequence with at least 90%, at least 95%, .at least 98%, or at least 99% sequence identity to SEQ ID NO:29. In another embodiment, the soluble PD-1 polypeptide further comprises an Fc domain.

In one embodiment, the immunotherapeutic agent is a modulator of CTLA-4 activity, a modulator of CD28 activity, a modulator of CD80 activity, a modulator of CD86 activity, a modulator of 4-1BB activity, an modulator of OX40 activity, a modulator of KIR activity, a modulator of Tim-3 activity, a modulator of CD27 activity, a modulator of CD40 activity, a modulator of GITR activity, a modulator of TIGIT activity, a modulator of CD20 activity, a modulator of CD96 activity, a modulator of IDO1 activity, a modulator of STING activity, a modulator of GARP activity, a modulator of A2aR activity, a modulator of CEACAM1 activity, a modulator of CEA activity, a modulator of CD47 activity, a modulator of PVRIG activity, a modulator of TDO activity, a modulator of VISTA activity, a cytokine, a chemokine, an interferon, an interleukin, a lymphokine, a member of the tumor necrosis factor (TNF) family, or an immunostimulatory oligonucleotide.

In one embodiment, the immunotherapeutic agent is an immune checkpoint inhibitor. In certain embodiments, the immune checkpoint inhibitor is a CTLA-4 antagonist, a CD80 antagonist, a CD86 antagonist, a Tim-3 antagonist, a TIGIT antagonist, a CD20 antagonist, a CD96 antagonist, a IDO1 antagonist, a STING antagonist, a GARP antagonist, a CD40 antagonist, A2aR antagonist, a CEACAM1 (CD66a) antagonist, a CEA antagonist, a CD47 antagonist a PVRIG antagonist, a TDO antagonist, a VISTA antagonist, or a KIR antagonist.

In one embodiment, the immune checkpoint inhibitor is a CTLA-4 antagonist. In certain embodiments, the CTLA-4 antagonist is an anti-CTLA-4 antibody or antigen binding fragment thereof. In some embodiments, the anti-CTLA-4 antibody is ipilimumab (YERVOY), tremelimumab (ticilimumab; CP-675,206), AGEN-1884, or ATOR-1015.

In one embodiment, the CTLA-4 antagonist is a soluble CTLA-4 polypeptide. In one embodiment, the soluble CTLA-4 polypeptide is abatacept (Orencia), belatacept (Nulojix), RG2077, or RG-1046. In another embodiment, the CTLA-4 antagonist is a cell based therapy. In some embodiments, the CTLA-4 antagonist is an anti-CTLA4 mAb RNA/GITRL RNA-transfected autologous dendritic cell vaccine or an anti-CTLA-4 mAb RNA-transfected autologous dendritic cell vaccine.

In one embodiment, the immune checkpoint inhibitor is a KIR antagonist. In certain embodiments, the KIR antagonist is an anti-KIR antibody or antigen binding fragment thereof. In some embodiments, the anti-KIR antibody is lirilumab (1-7F9, BMS-986015, IPH 2101) or IPH4102.

In one embodiment, the immune checkpoint inhibitor is TIGIT antagonist. In one embodiment, the TIGIT antagonist is an anti-TIGIT antibody or antigen binding fragment thereof. In certain embodiments, the anti-TIGIT antibody is BMS-986207, AB 154, COM902 (CGEN-15137), or OMP-313M32.

In one embodiment, the immune checkpoint inhibitor is Tim-3 antagonist. In certain embodiments, the Tim-3 antagonist is an anti-Tim-3 antibody or antigen binding fragment thereof. In some embodiments, the anti-Tim-3 antibody is TSR-022 or LY3321367.

In one embodiment, the immune checkpoint inhibitor is a IDO1 antagonist. In another embodiment, the IDO1 antagonist is indoximod (NLG8189; 1-methyl-_{D}-TRP), epacadostat (INCB-024360, INCB-24360), KHK2455, PF-06840003, navoximod (RG6078, GDC-0919, NLG919), BMS-986205 (F001287), or pyrrolidine-2,5-dione derivatives.

In one embodiment, the immune checkpoint inhibitor is a STING antagonist. In certain embodiments, the STING antagonist is 2' or 3'-mono-fluoro substituted cyclic-di-nucleotides; 2'3'-di-fluoro substituted mixed linkage 2',5' - 3',5' cyclic-di-nucleotides; 2'-fluoro substituted, bis-3',5' cyclic-di-nucleotides; 2',2"-diF-Rp,Rp,bis-3',5' cyclic-di-nucleotides; or fluorinated cyclic-di-nucleotides.

In one embodiment, the immune checkpoint inhibitor is CD20 antagonist. In some embodiments, the CD20 antagonist is an anti-CD20 antibody or antigen binding fragment thereof. In one embodiment, the anti-CD20 antibody is rituximab (RITUXAN; IDEC-102; IDEC-C2B8), ABP 798, ofatumumab, or obinutuzumab.

In one embodiment, the immune checkpoint inhibitor is CD80 antagonist. In certain embodiments, the CD80 antagonist is an anti-CD80 antibody or antigen binding fragment thereof. In one embodiment, the anti-CD80 antibody is galiximab or AV 1142742.

In one embodiment, the immune checkpoint inhibitor is a GARP antagonist. In some embodiments, the GARP antagonist is an anti-GARP antibody or antigen binding fragment thereof. In certain embodiments, the anti-GARP antibody is ARGX-115.

In one embodiment, the immune checkpoint inhibitor is a CD40 antagonist. In certain embodiments, the CD40 antagonist is an anti-CD40 antibody for antigen binding fragment thereof. In some embodiments, the anti-CD40 antibody is BMS3h-56, lucatumumab (HCD122 and CHIR-12.12), CHIR-5.9, or dacetuzumab (huS2C6, PRO 64553, RG 3636, SGN 14, SGN-40). In another embodiment, the CD40 antagonist is a soluble CD40 ligand (CD40-L). In one embodiment, the soluble CD40 ligand is a fusion polypeptide. In one embodiment, the soluble CD40 ligand is a CD40-L/FC2 or a monomeric CD40-L.

In one embodiment, the immune checkpoint inhibitor is an A2aR antagonist. In some embodiments, the A2aR antagonist is a small molecule. In certain embodiments, the A2aR antagonist is CPI-444, PBF-509, istradefylline (KW-6002), preladenant (SCH420814), tozadenant (SYN115), vipadenant (BIIB014), HTL-1071, ST1535, SCH412348, SCH442416, SCH58261, ZM241385, or AZD4635.

In one embodiment, the immune checkpoint inhibitor is a CEACAM1 antagonist. In some embodiments, the CEACAM1 antagonist is an anti-CEACAM1 antibody or antigen binding fragment thereof. In one embodiment, the anti-CEACAM1 antibody is CM-24 (MK-6018).

In one embodiment, the immune checkpoint inhibitor is a CEA antagonist. In one embodiment, the CEA antagonist is an anti-CEA antibody or antigen binding fragment thereof. In certain embodiments, the anti-CEA antibody is cergutuzumab amunaleukin (RG7813, RO-6895882) or RG7802 (RO6958688).

In one embodiment, the immune checkpoint inhibitor is a CD47 antagonist. In some embodiments, the CD47 antagonist is an anti-CD47 antibody or antigen binding fragment thereof. In certain embodiments, the anti-CD47 antibody is HuF9-G4, CC-90002, TTI-621, ALX148, NI-1701, NI-1801, SRF231, or Effi-DEM.

In one embodiment, the immune checkpoint inhibitor is a PVRIG antagonist. In certain embodiments, the PVRIG antagonist is an anti-PVRIG antibody or antigen binding fragment thereof. In one embodiment, the anti-PVRIG antibody is COM701 (CGEN-15029).

In one embodiment, the immune checkpoint inhibitor is a TDO antagonist. In one embodiment, the TDO antagonist is a 4-(indol-3-yl)-pyrazole derivative, a 3-indol substituted derivative, or a 3-(indol-3-yl)-pyridine derivative. In another embodiment, the immune checkpoint inhibitor is a dual IDO and TDO antagonist. In one embodiment, the dual IDO and TDO antagonist is a small molecule.

In one embodiment, the immune checkpoint inhibitor is a VISTA antagonist. In some embodiments, the VISTA antagonist is CA-170 or JNJ-61610588.

In one embodiment, the immunotherapeutic agent is an immune checkpoint enhancer or stimulator. In one embodiment, the immune checkpoint enhancer or stimulator is a CD28 agonist, a 4-1BB agonist, an OX40 agonist, a CD27 agonist, a CD80 agonist, a CD86 agonist, a CD40 agonist, an ICOS agonist, a CD70 agonist, or a GITR agonist.

In one embodiment, the immune checkpoint enhancer or stimulator is an OX40 agonist. In certain embodiments, the OX40 agonist is an anti-OX40 antibody or antigen binding fragment thereof. In some embodiments, the anti-OX40 antibody is tavolixizumab (MEDI-0562), pogalizumab (MOXR0916, RG7888), GSK3174998, ATOR-1015, MEDI-6383, MEDI-6469, BMS 986178, PF-04518600, or RG7888 (MOXR0916). In another embodiment, the OX40 agonist is a cell based therapy. In certain embodiments, the OX40 agonist is a GINAKIT cell (iC9-GD2-CD28-OX40-expressing T lymphocytes).

In one embodiment, the immune checkpoint enhancer or stimulator is a CD40 agonist. In some embodiments, the CD40 agonist is an anti-CD40 antibody or antigen binding fragment thereof. In one embodiment, the anti-CD40 antibody is ADC-1013 (JNJ-64457107), RG7876 (RO-7009789), HuCD40-M2, APX005M (EPI-0050), or Chi Lob 7/4. In another embodiment, the CD40 agonist is a soluble CD40 ligand (CD40-L). In one embodiment, the soluble CD40 ligand is a fusion polypeptide. In certain embodiments, the soluble CD40 ligand is a trimeric CD40-L (A VREND^{®}).

In one embodiment, the immune checkpoint enhancer or stimulator is a GITR agonist. In certain embodiments, the GITR agonist is an anti-GITR antibody or antigen binding fragment thereof. In one embodiment, the anti-GITR antibody is BMS-986156, TRX518, GWN323, INCAGN01876, or MEDI1873. In one embodiment, the GITR agonist is a soluble GITR ligand (GITRL). In some embodiments, the soluble GITR ligand is a fusion polypeptide. In another embodiment, the GITR agonist is a cell based therapy. In one embodiment, the cell based therapy is an anti-CTLA4 mAb RNA/GITRL RNA-transfected autologous dendritic cell vaccine or a GITRL RNA-transfected autologous dendritic cell vaccine.

In one embodiment, the immune checkpoint enhancer or stimulator a 4-1BB agonist. In some embodiments, the 4-1BB agonist is an anti-4-1BB antibody or antigen binding fragment thereof. In one embodiment, the anti-4-1BB antibody is urelumab or PF-05082566.

In one embodiment, the immune checkpoint enhancer or stimulator is a CD80 agonist or a CD86 agonist. In some embodiments, the CD80 agonist or the CD86 agonist is a soluble CD80 or CD86 ligand (CTLA-4). In certain embodiments, the soluble CD80 or CD86 ligand is a fusion polypeptide. In one embodiment, the CD80 or CD86 ligand is CTLA4-Ig (CTLA4-IgG4m, RG2077, or RG1046) or abatacept (ORENCIA, BMS-188667). In other embodiments, the CD80 agonist or the CD86 agonist is a cell based therapy. In one embodiment, the cell based therapy is MGN1601 (an allogeneic renal cell carcinoma vaccine).

In one embodiment, the immune checkpoint enhancer or stimulator is a CD28 agonist. In some embodiments, the CD28 agonist is an anti-CD28 antibody or antigen binding fragment thereof. In certain embodiments, the anti-CD28 antibody is TGN1412.

In one embodiment, the CD28 agonist is a cell based therapy. In certain embodiments, the cell based therapy is JCAR015 (anti-CD19-CD28-zeta modified CAR CD3+ T lymphocyte); CD28CAR/CD137CAR-expressing T lymphocyte; allogeneic CD4+ memory Th1-like T cells/microparticle-bound anti-CD3/anti-CD28; anti-CD19/CD28/CD3zeta CAR gammaretroviral vector-transduced autologous T lymphocytes KTE-C19; anti-CEA IgCD28TCR-transduced autologous T lymphocytes; anti-EGFRvIII CAR-transduced allogeneic T lymphocytes; autologous CD123CAR-CD28-CD3zeta-EGFRt-expressing T lymphocytes; autologous CD171-specific CAR-CD28 zeta-4-1-BB-EGFRt-expressing T lymphocytes; autologous CD19CAR-CD28-CD3zeta-EGFRt-expressing Tcm-enriched T cells; autologous PD-1-targeted chimeric switch receptor-modified T lymphocytes (chimera with CD28); CD19CAR-CD28-CD3zeta-EGFRt-expressing Tcm-enriched T lymphocytes; CD19CAR-CD28-CD3zeta-EGFRt-expressing Tn/mem-enriched T lymphocytes; CD19CAR-CD28zeta-4-1BB-expressing allogeneic T lymphocytes; CD19CAR-CD3zeta-4-1BB-CD28-expressing autologous T lymphocytes; CD28CAR/CD137CAR-expressing T lymphocytes; CD3/CD28 costimulated vaccine-primed autologous T lymphocytes; or iC9-GD2-CD28-OX40-expressing T lymphocytes.

In one embodiment, the immune checkpoint enhancer or stimulator is a CD27 agonist. In certain embodiments, the CD27 agonist is an anti-CD27 antibody or antigen binding fragment thereof. In one embodiment, the anti-CD27 antibody is varlilumab (CDX-1127).

In one embodiment, the immune checkpoint enhancer or stimulator is a CD70 agonist. In some embodiments, the CD70 agonist is an anti-CD70 antibody or antigen binding fragment thereof. In one embodiment, the anti-CD70 antibody is ARGX-110.

In one embodiment, the immune checkpoint enhancer or stimulator is an ICOS agonist. In certain embodiments, the ICOS agonist is an anti-ICOS antibody or antigen binding fragment thereof. In some embodiments, the anti-ICOS antibody is BMS986226, MEDI-570, GSK3359609, or JTX-2011. In other embodiments, the ICOS agonist is a soluble ICOS ligand. In some embodiments, the soluble ICOS ligand is a fusion polypeptide. In one embodiment, the soluble ICOS ligand is AMG 750.

In one embodiment, the immunotherapeutic agent is an anti-CD73 antibody or antigen binding fragment thereof. In certain embodiments, the anti-CD73 antibody is MEDI9447.

In one embodiment, the immunotherapeutic agent is a TLR9 agonist. In one embodiment, the TLR9 agonist is agatolimod sodium.

In one embodiment, the immunotherapeutic agent is a cytokine. In certain embodiments, the cytokine is a chemokine, an interferon, an interleukin, lymphokine, or a member of the tumor necrosis factor family. In some embodiments, the cytokine is IL-2, IL-15, or interferon-gamma.

In one embodiment, the immunotherapeutic agent is a TGF-β antagonist. In some embodiments, the TGF-β antagonist is fresolimumab (GC-1008); NIS793; IMC-TR1 (LY3022859); ISTH0036; trabedersen (AP 12009); recombinant transforming growth factor-beta-2; autologous HPV-16/18 E6/E7-specific TGF-beta-resistant T lymphocytes; or TGF-beta-resistant LMP-specific cytotoxic T-lymphocytes.

In one embodiment, the immunotherapeutic agent is an iNOS antagonist. In some embodiments, the iNOS antagonist is N-Acetyle-cysteine (NAC), aminoguanidine, L-nitroarginine methyl ester, or S,S-1,4-phenylene-bis(1,2-ethanediyl)bis-isothiourea).

In one embodiment, the immunotherapeutic agent is a SHP-1 antagonist.

In one embodiment, the immunotherapeutic agent is a CSF1R (colony stimulating factor 1 receptor) antagonist. In certain embodiments, the CSF1R antagonist is an anti-CSF1R antibody or antigen binding fragment thereof. In some embodiments, the anti-CSF1R antibody is emactuzumab.

In one embodiment, the immunotherapeutic agent is an agonist of a TNF family member. In some embodiments, the agonist of the TNF family member is ATOR 1016, ABBV-621, or Adalimumab.

In one embodiment, the immunotherapeutic agent is aldesleukin, tocilizumab, or MEDI5083.

In one embodiment, the immunotherapeutic agent is a CD160 (NK1) agonist. In certain embodiments, the CD160 (NK1) agonist is an anti-CD160 antibody or antigen binding fragment thereof. In one embodiment, the anti-CD160 antibody is BY55.

In one embodiment, the LAG-3 inhibitor, PD-1 pathway inhibitor, and the immunotherapeutic agent are formulated for intravenous administration. In some embodiments, the LAG-3 inhibitor, PD-1 pathway inhibitor, and the immunotherapeutic agent are formulated together. In another embodiment, the LAG-3 inhibitor, PD-1 pathway inhibitor, and the immunotherapeutic agent are formulated separately.

In one embodiment, the malignant tumor is selected from the group consisting of a liver cancer, bone cancer, pancreatic cancer, skin cancer, oral cancer, cancer of the head or neck, breast cancer, lung cancer - including small cell and non-small cell lung cancer, cutaneous or intraocular malignant melanoma, renal cancer, uterine cancer, ovarian cancer, colorectal cancer, colon cancer, rectal cancer, cancer of the anal region, stomach cancer, testicular cancer, uterine cancer, carcinoma of the fallopian tubes, carcinoma of the endometrium, carcinoma of the cervix, carcinoma of the vagina, carcinoma of the vulva, non-Hodgkin's lymphoma, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, cancers of the childhood, lymphocytic lymphoma, cancer of the bladder, cancer of the kidney or ureter, carcinoma of the renal pelvis, neoplasm of the central nervous system (CNS), primary CNS lymphoma, tumor angiogenesis, spinal axis tumor, brain stem glioma, pituitary adenoma, Kaposi's sarcoma, epidermoid cancer, squamous cell cancer, environmentally induced cancers including those induced by asbestos, hematologic malignancies including, for example, multiple myeloma, B-cell lymphoma, Hodgkin lymphoma/primary mediastinal B-cell lymphoma, non-Hodgkin's lymphomas, acute myeloid lymphoma, chronic myelogenous leukemia, chronic lymphoid leukemia, follicular lymphoma, diffuse large B-cell lymphoma, Burkitt's lymphoma, immunoblastic large cell lymphoma, precursor B-lymphoblastic lymphoma, mantle cell lymphoma, acute lymphoblastic leukemia, mycosis fungoides, anaplastic large cell lymphoma, T-cell lymphoma, and precursor T-lymphoblastic lymphoma, and any combination thereof.

In one embodiment, the malignant tumor is non-small cell lung cancer (NSCLC), a virally-related cancer related tumor, or gastric adenocarcinoma. In some embodiments, the malignant tumor is melanoma, gastric cancer, gastroesophageal junction cancer, non-small cell lung cancer, bladder cancer, head and neck squamous cell carcinoma, or renal cell cancer. In one embodiment, wherein the tumor is lung cancer, melanoma, squamous cell carcinoma of the head and neck, renal cancer, gastric cancer, or hepatocellular carcinoma.

In one embodiment, the anti-LAG-3 antibody or antigen binding fragment thereof and the immunotherapeutic agent are administered as a first line of treatment. In another embodiment, the LAG-3 inhibitor, PD-1 pathway inhibitor, and the immunotherapeutic agent are administered as a second line of treatment. In certain embodiments, the malignant tumor is refractory to first line treatment.

In one embodiment, the method for treating a subject afflicted with a malignant tumor as described above further comprises the administration of at least one additional therapeutic agent. In certain embodiments, the at least one additional therapeutic agent is a chemotherapeutic agent.

Other features and advantages of the instant disclosure will be apparent from the following detailed description and examples, which should be construed as limiting.

### DETAILED DESCRIPTION OF THE INVENTION

### I. Terms

A "patient" as used herein includes any patient who is afflicted with a cancer (e.g., melanoma). The terms "subject" and "patient" are used interchangeably herein

As used herein, the term "administering" refers to the physical introduction of a composition comprising a therapeutic agent (e.g., combination of an anti-PD-1 antibody, an anti-LAG-3 antibody, and an additional immunotherapeutic agent) to a subject, using any of the various methods and delivery systems known to those skilled in the art. Routes of administration include intravenous, intramuscular, subcutaneous, intraperitoneal, spinal or other parenteral routes of administration, for example by injection or infusion. The phrase "parenteral administration" as used herein means modes of administration other than enteral and topical administration, usually by injection, and includes, without limitation, intravenous, intramuscular, intraarterial, intrathecal, intralymphatic, intralesional, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion, as well as *in vivo* electroporation. Other non-parenteral routes include a topical, epidermal or mucosal route of administration, for example, intranasally, vaginally, rectally, sublingually or topically. Administering can also be performed, for example, once, a plurality of times, and/or over one or more extended periods.

As used herein, "effective treatment" refers to treatment producing a beneficial effect, e.g., amelioration of at least one symptom of a disease or disorder. A beneficial effect can take the form of an improvement over baseline, i.e., an improvement over a measurement or observation made prior to initiation of therapy according to the method. A beneficial effect can also take the form of arresting, slowing, retarding, or stabilizing of a deleterious progression of a marker of solid tumor. Effective treatment may refer to alleviation of at least one symptom of a solid tumor. Such effective treatment may, e.g., reduce patient pain, reduce the size and/or number of lesions, may reduce or prevent metastasis of a tumor, and/or may slow tumor growth.

The term "effective amount" refers to an amount of an agent that provides the desired biological, therapeutic, and/or prophylactic result. That result can be reduction, amelioration, palliation, lessening, delaying, and/or alleviation of one or more of the signs, symptoms, or causes of a disease, or any other desired alteration of a biological system. In reference to solid tumors, an effective amount comprises an amount sufficient to cause a tumor to shrink and/or to decrease the growth rate of the tumor (such as to suppress tumor growth) or to prevent or delay other unwanted cell proliferation. In some embodiments, an effective amount is an amount sufficient to delay tumor development. In some embodiments, an effective amount is an amount sufficient to prevent or delay tumor recurrence. An effective amount can be administered in one or more administrations. The effective amount of the drug or composition may: (i) reduce the number of cancer cells; (ii) reduce tumor size; (iii) inhibit, retard, slow to some extent and may stop cancer cell infiltration into peripheral organs; (iv) inhibit (i.e., slow to some extent and may stop tumor metastasis; (v) inhibit tumor growth; (vi) prevent or delay occurrence and/or recurrence of tumor; and/or (vii) relieve to some extent one or more of the symptoms associated with the cancer. In one example, an "effective amount" is the amount of anti-LAG-3 antibody and the amount of anti-PD-1 antibody, in combination, clinically proven to affect a significant decrease in cancer or slowing of progression of cancer, such as an advanced solid tumor. As used herein, the terms "fixed dose", "flat dose" and "flat-fixed dose" are used interchangeably and refer to a dose that is administered to a patient without regard for the weight or body surface area (BSA) of the patient. The fixed or flat dose is therefore not provided as a mg/kg dose, but rather as an absolute amount of the agent (e.g., the anti-LAG-3 antibody and/or anti-PD-1 antibody).

As used herein, the term "immunotherapy" refers to the treatment of a subject afflicted with, or at risk of contracting or suffering a recurrence of, a disease by a method comprising inducing, enhancing, suppressing or otherwise modifying an immune response. "Treatment" or "therapy" of a subject refers to any type of intervention or process performed on, or the administration of an active agent (e.g., composition comprising a combination of an anti-PD-1 antibody, an anti-LAG-3 antibody, and an additional immunotherapeutic agent) to the subject with the objective of reversing, alleviating, ameliorating, inhibiting, slowing down or preventing the onset, progression, development, severity or recurrence of a symptom, complication or condition, or biochemical indicia associated with a disease.

As used herein, the terms "cell based therapy," "cell therapy," "cellular therapy," or "cytotherapy" refer to the transplantation of delivery of cellular material into a patient for the purpose of treating a disease or disorder (e.g., malignant tumor). The cellular material can be a cellular fragment or an intact, living cell (e.g., T lymphocytes, dendritic cells, or stem cells).

The use of the term "fixed dose" with regard to a composition of the invention means that two or more different antibodies in a single composition are present in the composition in particular (fixed) ratios with each other. In some embodiments, the fixed dose is based on the weight (*e*.*g*., mg) of the antibodies. In certain embodiments, the fixed dose is based on the concentration (*e*.*g*., mg/ml) of the antibodies. In some embodiments, the ratio is at least about 1: 1, about 1:2, about 1:3, about 1:4, about 1:5, about 1:6, about 1:7, about 1:8, about 1:9, about 1:10, about 1:15, about 1:20, about 1:30, about 1:40, about 1:50, about 1:60, about 1:70, about 1:80, about 1:90, about 1:100, about 1:120, about 1:140, about 1:160, about 1:180, about 1:200, about 200:1, about 180:1, about 160:1, about 140:1, about 120:1, about 100:1, about 90:1, about 80:1, about 70:1, about 60:1, about 50:1, about 40:1, about 30:1, about 20:1, about 15:1, about 10:1, about 9:1, about 8:1, about 7:1, about 6:1, about 5:1, about 4:1, about 3:1, or about 2:1 mg first antibody to mg second antibody. For example, the 3:1 ratio of a first antibody and a second antibody can mean that a vial can contain about 240 mg of the first antibody and 80 mg of the second antibody or about 3 mg/ml of the first antibody and 1 mg/ml of the second antibody.

The use of the term "flat dose" with regard to the composition of the invention means a dose that is administered to a patient without regard for the weight or body surface area (BSA) of the patient. The flat dose is therefore not provided as a mg/kg dose, but rather as an absolute amount of the agent (e.g., the anti-LAG-3 antibody and/or anti-PD-1 antibody). For example, a 60 kg person and a 100 kg person would receive the same dose of the composition (*e*.*g*., 240 mg of an anti-PD-1 antibody and 80 mg of an anti-LAG-3 antibody in a single fixed dosing formulation vial containing both 240 mg of an anti-PD-1 antibody and 80 mg of an anti- LAG-3 antibody (or two fixed dosing formulation vials containing 120 mg of an anti-PD-1 antibody and 40 mg of an anti-LAG-3 antibody, etc.)).

The term "weight based dose" as referred to herein means that a dose that is administered to a patient is calculated based on the weight of the patient. For example, when a patient with 60 kg body weight requires 3 mg/kg of an anti-LAG-3 antibody in combination with 3 mg/kg of an anti-PD-1 antibody, one can draw the appropriate amounts of the anti-LAG-3 antibody (*i.e.,* 180 mg) and the anti-PD-1 antibody (*i.e.,* 180 mg) at once from a 1:1 ratio fixed dosing formulation of an anti-LAG3 antibody and an anti-PD-1 antibody.

An "antibody" (Ab) shall include, without limitation, a glycoprotein immunoglobulin which binds specifically to an antigen and comprises at least two heavy (H) chains and two light (L) chains interconnected by disulfide bonds, or an antigen-binding portion thereof. Each heavy chain is comprised of a heavy chain variable region (abbreviated herein as V_{H}) and a heavy chain constant region (abbreviated herein as CH). In certain antibodies, *e*.*g*., naturally occurring IgG antibodies, the heavy chain constant region is comprised of a hinge and three domains, CH1, CH2 and CH3. In certain antibodies, *e*.*g*., naturally occurring IgG antibodies, each light chain is comprised of a light chain variable region (abbreviated herein as V_{L}) and a light chain constant region. The light chain constant region is comprised of one domain (abbreviated herein as CL). The V_{H} and V_{L} regions can be further subdivided into regions of hypervariability, termed complementarity determining regions (CDR), interspersed with regions that are more conserved, termed framework regions (FR). Each V_{H} and V_{L} is composed of three CDRs and four FRs, arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies can mediate the binding of the immunoglobulin to host tissues or factors, including various cells of the immune system (*e*.*g*., effector cells) and the first component (C1q) of the classical complement system. A heavy chain may have the C-terminal lysine or not. Unless specified otherwise herein, the amino acids in the variable regions are numbered using the Kabat numbering system and those in the constant regions are numbered using the EU system.

An immunoglobulin can be from any of the known isotypes, including IgA, secretory IgA, IgD, IgE, IgG, and IgM. The IgG isotype is divided in subclasses in certain species: IgG1, IgG2, IgG3 and IgG4 in humans, and IgG1, IgG2a, IgG2b and IgG3 in mice. "Isotype" refers to the antibody class or subclass (e.g., IgM or IgG1) that is encoded by the heavy chain constant region genes. The term "antibody" includes, by way of example, monoclonal and polyclonal antibodies; chimeric and humanized antibodies; human or nonhuman antibodies; wholly synthetic antibodies; and single chain antibodies. A nonhuman antibody may be humanized by recombinant methods to reduce its immunogenicity in man. Where not expressly stated, and unless the context indicates otherwise, the term "antibody" includes monospecific, bispecific, or multi-specific antibodies, as well as a single chain antibody. In embodiments, the antibody is a bispecific antibody. In other embodiments, the antibody is a monospecific antibody.

As used herein, an "IgG antibody" has the structure of a naturally occurring IgG antibody, *i*.*e*., it has the same number of heavy and light chains and disulfide bonds as a naturally occurring IgG antibody of the same subclass. For example, an anti-ICOS IgG1, IgG2, IgG3 or IgG4 antibody consists of two heavy chains (HCs) and two light chains (LCs), wherein the two heavy chains and light chains are linked by the same number and location of disulfide bridges that occur in naturally occurring IgG1, IgG2, IgG3 and IgG4 antibodies, respectively (unless the antibody has been mutated to modify the disulfide bonds).

An "isolated antibody" refers to an antibody that is substantially free of other antibodies having different antigenic specificities (*e*.*g*., an isolated antibody that binds specifically to PD-1 is substantially free of antibodies that bind specifically to antigens other than PD-1). An isolated antibody that binds specifically to PD-1 may, however, have cross-reactivity to other antigens, such as PD-1 molecules from different species. Moreover, an isolated antibody may be substantially free of other cellular material and/or chemicals.

The antibody may be an antibody that has been altered (e.g., by mutation, deletion, substitution, conjugation to a non-antibody moiety). For example, an antibody may include one or more variant amino acids (compared to a naturally occurring antibody) which change a property (e.g., a functional property) of the antibody. For example, numerous such alterations are known in the art which affect, e.g., half-life, effector function, and/or immune responses to the antibody in a patient. The term antibody also includes artificial polypeptide constructs which comprise at least one antibody-derived antigen binding site.

The term "monoclonal antibody" ("mAb") refers to a non-naturally occurring preparation of antibody molecules of single molecular composition, *i*.*e*., antibody molecules whose primary sequences are essentially identical, and which exhibits a single binding specificity and affinity for a particular epitope. A monoclonal antibody is an example of an isolated antibody. MAbs may be produced by hybridoma, recombinant, transgenic or other techniques known to those skilled in the art.

A "human" antibody (HuMAb) refers to an antibody having variable regions in which both the framework and CDR regions are derived from human germline immunoglobulin sequences. Furthermore, if the antibody contains a constant region, the constant region also is derived from human germline immunoglobulin sequences. The human antibodies of the invention can include amino acid residues not encoded by human germline immunoglobulin sequences (*e*.*g*., mutations introduced by random or site-specific mutagenesis *in vitro* or by somatic mutation *in vivo*). However, the term "human antibody," as used herein, is not intended to include antibodies in which CDR sequences derived from the germline of another mammalian species, such as a mouse, have been grafted onto human framework sequences. The terms "human" antibodies and "fully human" antibodies and are used synonymously.

A "humanized antibody" refers to an antibody in which some, most or all of the amino acids outside the CDR domains of a non-human antibody are replaced with corresponding amino acids derived from human immunoglobulins. In one embodiment of a humanized form of an antibody, some, most or all of the amino acids outside the CDR domains have been replaced with amino acids from human immunoglobulins, whereas some, most or all amino acids within one or more CDR regions are unchanged. Small additions, deletions, insertions, substitutions or modifications of amino acids are permissible as long as they do not abrogate the ability of the antibody to bind to a particular antigen. A "humanized" antibody retains an antigenic specificity similar to that of the original antibody.

A "chimeric antibody" refers to an antibody in which the variable regions are derived from one species and the constant regions are derived from another species, such as an antibody in which the variable regions are derived from a mouse antibody and the constant regions are derived from a human antibody.

An "anti-antigen" antibody refers to an antibody that binds specifically to the antigen. For example, an anti-PD-1 antibody binds specifically to PD-1 and an anti-CTLA-4 antibody binds specifically to CTLA-4.

An "antigen-binding portion" of an antibody (also called an "antigen-binding fragment") refers to one or more fragments of an antibody that retain the ability to bind specifically to the antigen bound by the whole antibody. It has been shown that the antigen-binding function of an antibody can be performed by fragments or portions of a full-length antibody. Examples of binding fragments encompassed within the term "antigen-binding portion" or "antigen-binding fragment" of an antibody, e.g., an anti-ICOS antibody described herein, include:
(1) a Fab fragment (fragment from papain cleavage) or a similar monovalent fragment consisting of the VL, VH, LC and CH1 domains;
(2) a F(ab')2 fragment (fragment from pepsin cleavage) or a similar bivalent fragment comprising two Fab fragments linked by a disulfide bridge at the hinge region;
(3) a Fd fragment consisting of the VH and CH1 domains;
(4) a Fv fragment consisting of the VL and VH domains of a single arm of an antibody,
(5) a single domain antibody (dAb) fragment (Ward et al., (1989) Nature 341:544-46), which consists of a VH domain;
(6) a bi-single domain antibody which consists of two VH domains linked by a hinge (dual-affinity re-targeting antibodies (DARTs));
(7) a dual variable domain immunoglobulin;
(8) an isolated complementarity determining region (CDR); and
(9) a combination of two or more isolated CDRs, which can optionally be joined by a synthetic linker. Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules (known as single chain Fv (scFv); see e.g., Bird et al. (1988) Science 242:423-426; and Huston et al. (1988) Proc. Natl. Acad. Sci. USA 85:5879-5883). Such single chain antibodies are also intended to be encompassed within the term "antigen-binding portion" or "antigen-binding fragment" of an antibody. These antibody fragments are obtained using conventional techniques known to those with skill in the art, and the fragments are screened for utility in the same manner as are intact antibodies. Antigen-binding portions can be produced by recombinant DNA techniques, or by enzymatic or chemical cleavage of intact immunoglobulins.

The term "LAG-3", "LAG3", or "Lymphocyte Activation Gene-3" refers to Lymphocyte Activation Gene-3. The term LAG-3 as used herein includes human LAG-3 (hLAG-3), variants, isoforms, and species homologs of hLAG-3, and analogs having at least one common epitope with hLAG-3. The term LAG-3 as used herein includes variants, isoforms, homologs, orthologs and paralogs. For example, antibodies specific for a human LAG-3 protein may, in certain cases, cross-react with a LAG-3 protein from a species other than human. In other embodiments, the antibodies specific for a human LAG-3 protein may be completely specific for the human LAG-3 protein and may not exhibit species or other types of cross-reactivity, or may cross-react with LAG-3 from certain other species, but not all other species (e.g., cross-react with monkey LAG-3 but not mouse LAG-3). The term "human LAG-3" refers to human sequence LAG-3, such as the complete amino acid sequence of human LAG-3 having Genbank Accession No. NP_002277 (SEQ ID NO: 13). The term "mouse LAG-3" refers to mouse sequence LAG-3, such as the complete amino acid sequence of mouse LAG-3 having Genbank Accession No. NP_032505. LAG-3 is also known in the art as, for example, CD223. The human LAG-3 sequence may differ from human LAG-3 of Genbank Accession No. NP_002277 by having, e.g., conserved mutations or mutations in non-conserved regions and the LAG-3 has substantially the same biological function as the human LAG-3 of Genbank Accession No. NP_002277 (SEQ ID NO: 44). For example, a biological function of human LAG-3 is having an epitope in the extracellular domain of LAG-3 that is specifically bound by an antibody of the instant disclosure or a biological function of human LAG-3 is binding to MHC Class II molecules.

A particular human LAG-3 sequence will generally be at least 90% identical in amino acid sequence to human LAG-3 of GenBank Accession No. NP_002277 and contains amino acid residues that identify the amino acid sequence as being human when compared to LAG-3 amino acid sequences of other species (e.g., murine). In certain cases, a human LAG-3 can be at least 95%, or even at least 96%, 97%, 98%, or 99% identical in amino acid sequence to LAG-3 of GenBank Accession No. NP_002277. In certain embodiments, a human LAG-3 sequence will display no more than 10 amino acid differences from the LAG-3 sequence of GenBank Accession No. NP_002277. In certain embodiments, the human LAG-3 can display no more than 5, or even no more than 4, 3, 2, or 1 amino acid difference from the LAG-3 sequence of GenBank Accession No. NP_002277. Percent identity can be determined as described herein.

As used herein, the terms "Programmed Death 1," "Programmed Cell Death 1," "Protein PD-1," "PD-1," "PD1," "PDCD1," "hPD-1" and "hPD-I" are used interchangeably, and include variants, isoforms, species homologs of human PD-1, and analogs having at least one common epitope with PD-1. The complete PD-1 sequence can be found under GenBank Accession Nos. U64863 (SEQ ID NO:29) and AAC51773.1 (SEQ ID NO: 45).

The protein Programmed Death 1 (PD-1) is an inhibitory member of the CD28 family of receptors, that also includes CD28, CTLA-4, ICOS and BTLA. PD-1 is expressed on activated B cells, T cells, and myeloid cells (Agata et al., supra; Okazaki et al. (2002) Curr. Opin. Immunol. 14: 391779-82; Bennett et al. (2003) J Immunol 170:711-8). The initial members of the family, CD28 and ICOS, were discovered by functional effects on augmenting T cell proliferation following the addition of monoclonal antibodies (Hutloff et al. Nature (1999); 397:263-266; Hansen et al. Immunogenics (1980); 10:247-260). PD-1 was discovered through screening for differential expression in apototic cells (Ishida et al. EMBO J (1992); 11:3887-95). The other members of the family, CTLA-4 and BTLA, were discovered through screening for differential expression in cytotoxic T lymphocytes and TH1 cells, respectively. CD28, ICOS and CTLA-4 all have an unpaired cysteine residue allowing for homodimerization. In contrast, PD-1 is suggested to exist as a monomer, lacking the unpaired cysteine residue characteristic in other CD28 family members.

The PD-1 gene is a 55 kDa type I transmembrane protein that is part of the Ig gene superfamily (Agata et al. (1996) Int Immunol 8:765-72). PD-1 contains a membrane proximal immunoreceptor tyrosine inhibitory motif (ITIM) and a membrane distal tyrosine-based switch motif (ITSM) (Thomas, M. L. (1995) J Exp Med 181:1953-6; Vivier, E and Daeron, M (1997) Immunol Today 18:286-91). Although structurally similar to CTLA-4, PD-1 lacks the MYPPPY motif that is critical for B7-1 and B7-2 binding. Two ligands for PD-1 have been identified, PD-L1 and PD-L2, that have been shown to downregulate T cell activation upon binding to PD-1 (Freeman et al. (2000) J Exp Med 192:1027-34; Latchman et al. (2001) Nat Immunol 2:261-8; Carter et al. (2002) Eur J Immunol 32:634-43). Both PD-L1 and PD-L2 are B7 homologs that bind to PD-1, but do not bind to other CD28 family members. PD-L1 is abundant in a variety of human cancers (Dong et al. (2002) Nat. Med. 8:787-9). The interaction between PD-1 and PD-L1 results in a decrease in tumor infiltrating lymphocytes, a decrease in T-cell receptor mediated proliferation, and immune evasion by the cancerous cells (Dong et al. (2003) J. Mol. Med. 81:281-7; Blank et al. (2005) Cancer Immunol. Immunother. 54:307-314; Konishi et al. (2004) Clin. Cancer Res. 10:5094-100). Immune suppression can be reversed by inhibiting the local interaction of PD-1 with PD-L1, and the effect is additive when the interaction of PD-1 with PD-L2 is blocked as well (Iwai et al. (2002) Proc. Nat'l. Acad. Sci. USA 99:12293-7; Brown et al. (2003) J. Immunol. 170:1257-66).

Consistent with PD-1 being an inhibitory member of the CD28 family, PD-1 deficient animals develop various autoimmune phenotypes, including autoimmune cardiomyopathy and a lupus-like syndrome with arthritis and nephritis (Nishimura et al. (1999) Immunity 11:141-51; Nishimura et al. (2001) Science 291:319-22). Additionally, PD-1 has been found to play a role in autoimmune encephalomyelitis, systemic lupus erythematosus, graft-versus-host disease (GVHD), type I diabetes, and rheumatoid arthritis (Salama et al. (2003) J Exp Med 198:71-78; Prokunina and Alarcon-Riquelme (2004) Hum Mol Genet 13:R143; Nielsen et al. (2004) Lupus 13:510). In a murine B cell tumor line, the ITSM of PD-1 was shown to be essential to block BCR-mediated Ca2+-flux and tyrosine phosphorylation of downstream effector molecules (Okazaki et al. (2001) PNAS 98:13866-71).

"Programmed Death Ligand-1 (PD-L1)" is one of two cell surface glycoprotein ligands for PD-1 (the other being PD-L2) that downregulate T cell activation and cytokine secretion upon binding to PD-1. The term "PD-L1" as used herein includes human PD-L1 (hPD-L1), variants, isoforms, and species homologs of hPD-L1, and 5 analogs having at least one common epitope with hPD-L1. The complete hPD-L1 sequence can be found under GenBank Accession No. Q9NZQ7.

The terms "Programmed Death Ligand-2" and "PD-L2" as used herein include human PD-L2 (hPD-L2), variants, isoforms, and species homologs of hPD-L2, and analogs having at least one common epitope with hPD-L2. The complete hPD-L2 sequence can be found under GenBank Accession No. Q9BQ51.

A "cancer" refers a broad group of various diseases characterized by the uncontrolled growth of abnormal cells in the body. Unregulated cell division and growth results in the formation of malignant tumors that invade neighboring tissues and may also metastasize to distant parts of the body through the lymphatic system or bloodstream. A "cancer" or "cancer tissue" can include a tumor.

The term "tumor" as used herein refers to any mass of tissue that results from excessive cell growth or proliferation, either benign (non-cancerous) or malignant (cancerous), including pre-cancerous lesions.

The term "and/or" where used herein is to be taken as specific disclosure of each of the two specified features or components with or without the other. Thus, the term "and/or" as used in a phrase such as "A and/or B" herein is intended to include "A and B," "A or B," "A" (alone), and "B" (alone). Likewise, the term "and/or" as used in a phrase such as "A, B, and/or C" is intended to encompass each of the following aspects: A, B, and C; A, B, or C; A or C; A or B; B or C; A and C; A and B; B and C; A (alone); B (alone); and C (alone).

It is understood that wherever aspects are described herein with the language "comprising," otherwise analogous aspects described in terms of "consisting of" and/or "consisting essentially of" are also provided.

The terms "about" or "comprising essentially of" refer to a value or composition that is within an acceptable error range for the particular value or composition as determined by one of ordinary skill in the art, which will depend in part on how the value or composition is measured or determined, i.e., the limitations of the measurement system. For example, "about" or "comprising essentially of" can mean within 1 or more than 1 standard deviation per the practice in the art. Alternatively, "about" or "comprising essentially of" can mean a range of up to 10% or 20% (*i.e.,* ±10% or ±20%). For example, about 3mg can include any number between 2.7 mg and 3.3 mg (for 10%) or between 2.4 mg and 3.6 mg (for 20%). Furthermore, particularly with respect to biological systems or processes, the terms can mean up to an order of magnitude or up to 5-fold of a value. When particular values or compositions are provided in the application and claims, unless otherwise stated, the meaning of "about" or "comprising essentially of" should be assumed to be within an acceptable error range for that particular value or composition.

As described herein, any concentration range, percentage range, ratio range or integer range is to be understood to include the value of any integer within the recited range and, when appropriate, fractions thereof (such as one-tenth and one-hundredth of an integer), unless otherwise indicated.

Unless defined otherwise, all technical and scientific terms used herein have the same meaning as commonly understood by one of ordinary skill in the art to which this disclosure is related. For example, the Concise Dictionary of Biomedicine and Molecular Biology, Juo, Pei-Show, 2nd ed., 2002, CRC Press; The Dictionary of Cell and Molecular Biology, 5th ed., 2013, Academic Press; and the Oxford Dictionary Of Biochemistry And Molecular Biology, 2006, Oxford University Press, provide one of skill with a general dictionary of many of the terms used in this disclosure.

Units, prefixes, and symbols are denoted in their Système International de Unites (SI) accepted form. Numeric ranges are inclusive of the numbers defining the range. The headings provided herein are not limitations of the various aspects of the disclosure, which can be had by reference to the specification as a whole. Accordingly, the terms defined immediately below are more fully defined by reference to the specification in its entirety.

Various aspects of the invention are described in further detail in the following subsections.

### IIa. Anti-LAG-3 Antibodies

Anti-human-LAG-3 antibodies (or VH/VL domains derived therefrom) suitable for use in the invention can be generated using methods well known in the art. Alternatively, art recognized anti-LAG-3 antibodies can be used. For example, the anti-human LAG-3 antibody described in US2011/0150892 A1, the teachings of which are hereby incorporated by reference, and referred to as monoclonal antibody 25F7 (also known as "25F7" and "LAG3.1) can be used. Other art recognized anti-LAG-3 antibodies that can be used include IMP731 (H5L7BW) described in US 2011/007023, MK-4280 (28G-10) described in WO2016028672, REGN3767 described in Journal for ImmunoTherapy of Cancer, (2016) Vol. 4, Supp. Supplement 1 Abstract Number: P195, BAP050 described in WO2017/019894, IMP-701 (LAG-525), IMP321 (eftilagimod alpha)), Sym022, TSR-033, MGD013, BI754111, FS118, AVA-017 and GSK2831781. These and other anti-LAG-3 antibodies useful in the claimed invention can be found in, for example: WO2016/028672, WO2017/106129, WO2017/062888, WO2009/044273, WO2018/069500, WO2016/126858, WO2014/179664, WO2016/200782, WO2015/200119, WO2017/019846, WO2017/198741, WO2017/220555, WO2017/220569, WO2018/071500, WO2017/015560, WO2017/025498, WO2017/087589, WO2017/087901, WO2018/083087, WO2017/149143, WO2017/219995, US2017/0260271, WO2017/086367, WO/2017/086419, WO2018/034227, and WO2014/140180. The contents of each of these references are herein incorporated by reference.

Antibodies that compete with any of the above-referenced art-recognized antibodies for binding to LAG-3 also can be used.

An exemplary anti-LAG-3 antibody is BMS-986016 comprising heavy and light chains comprising the sequences shown in SEQ ID NOs:1 and 2, respectively, or antigen binding fragments and variants thereof, as described in US Pat. No. 9,505,839, which is herein incorporated by reference.

In other embodiments, the antibody has the heavy and light chain CDRs or variable regions of BMS-986016. Accordingly, in one embodiment, the antibody comprises CDR1, CDR2, and CDR3 domains of the VH region of BMS-986016 having the sequence set forth in SEQ ID NO:3, and CDR1, CDR2 and CDR3 domains of the VL region of BMS-986016 having the sequence set forth in SEQ ID NO:5. In another embodiment, the antibody comprises CDR1, CDR2 and CDR3 domains comprising the sequences set forth in SEQ ID NOs:7, 8, and 9, respectively, and CDR1, CDR2 and CDR3 domains comprising the sequences set forth in SEQ ID NOs:10, 11, and 12, respectively. In another embodiment, the antibody comprises VH and/or VL regions comprising the amino acid sequences set forth in SEQ ID NO:3 and/or SEQ ID NO: 5, respectively. In another embodiment, the antibody comprises heavy chain variable (VH) and/or light chain variable (VL) regions encoded by the nucleic acid sequences set forth in SEQ ID NO:4 and/or SEQ ID NO:6, respectively. In another embodiment, the antibody competes for binding with and/or binds to the same epitope on LAG-3 as the above-mentioned antibodies. In another embodiment, the antibody binds an epitope of human LAG-3 comprising the amino acid sequence PGHPLAPG (SEQ ID NO:14). In another embodiment, the antibody binds an epitope of human LAG-3 comprising the amino acid sequence HPAAPSSW (SEQ ID NO:15) or PAAPSSWG (SEQ ID NO:16).

In another embodiment, the antibody has at least about 90% variable region amino acid sequence identity with the above-mentioned antibodies (e.g., at least about 90%, 95% or 99% variable region identity with SEQ ID NO:3 or SEQ ID NO:5).

In embodiments, the anti-LAG-3 antibody is a bispecific antibody. In embodiments, the anti-LAG-3 antibody is a bispecific antibody that binds both PD-1 and LAG-3.

### Ib. Anti-PD-1 Antibodies

Human monoclonal antibodies (HuMAbs) that bind specifically to PD-1 with high affinity have been disclosed in U.S. Patent Nos. 8,008,449 and 8,779,105. Other anti-PD-1 mAbs have been described in, for example, U.S. Patent Nos. 6,808,710, 7,488,802, 8,168,757 and 8,354,509, and PCT Publication No. WO 2012/145493. Each of the anti-PD-1 HuMAbs disclosed in U.S. Patent No. 8,008,449 has been demonstrated to exhibit one or more of the following characteristics: (a) binds to human PD-1 with a K_{D} of 1 × 10⁻⁷ M or less, as determined by surface plasmon resonance using a Biacore biosensor system; (b) does not substantially bind to human CD28, CTLA-4 or ICOS; (c) increases T-cell proliferation in a Mixed Lymphocyte Reaction (MLR) assay; (d) increases interferon-γ production in an MLR assay; (e) increases IL-2 secretion in an MLR assay; (f) binds to human PD-1 and cynomolgus monkey PD-1; (g) inhibits the binding of PD-L1 and/or PD-L2 to PD-1; (h) stimulates antigen-specific memory responses; (i) stimulates Ab responses; and (j) inhibits tumor cell growth *in vivo.* Anti-PD-1 antibodies useful for the present invention include mAbs that bind specifically to human PD-1 and exhibit at least one, preferably at least five, of the preceding characteristics.

In one embodiment, the anti-PD-1 antibody is nivolumab. Nivolumab (also known as "OPDIVO^{®}"; BMS-936558; formerly designated 5C4, BMS-936558, MDX-1106, or ONO-4538) is a fully human IgG4 (S228P) PD-1 immune checkpoint inhibitor antibody that selectively prevents interaction with PD-1 ligands (PD-L1 and PD-L2), thereby blocking the down-regulation of antitumor T-cell functions (U.S. Patent No. 8,008,449; Wang et al., 2014 Cancer Immunol Res. 2(9):846-56). In another embodiment, the anti-PD-1 antibody or fragment thereof cross-competes with nivolumab. In other embodiments, the anti-PD-1 antibody or fragment thereof binds to the same epitope as nivolumab. In certain embodiments, the anti-PD-1 antibody has the same CDRs as nivolumab.

In another embodiment, the anti-PD-1 antibody is pembrolizumab. Pembrolizumab is a humanized monoclonal IgG4 (S228P) antibody directed against human cell surface receptor PD-1 (programmed death-1 or programmed cell death-1). Pembrolizumab is described, for example, in U.S. Patent Nos. 8,354,509 and 8,900,587.

Anti-human-PD-1 antibodies (or VH and/or VL domains derived therefrom) suitable for use in the invention can be generated using methods well known in the art. Alternatively, art recognized anti-PD-1 antibodies can be used. For example, monoclonal antibodies 5C4 (referred to herein as Nivolumab or BMS-936558), 17D8, 2D3, 4H1, 4A11, 7D3, and 5F4, described in WO 2006/121168, the teachings of which are hereby incorporated by reference, can be used. Other known PD-1 antibodies include lambrolizumab (MK-3475) described in WO 2008/156712, and AMP-514 described in WO 2012/145493, the teachings of which are hereby incorporated by reference. Further known anti-PD-1 antibodies and other PD-1 inhibitors include those described in WO 2009/014708, WO 03/099196, WO 2009/114335 and WO 2011/161699, the teachings of which are hereby incorporated by reference. In one embodiment, the anti-PD-1 antibody is REGN2810. In one embodiment, the anti-PD-1 antibody is PDR001. Another known anti-PD-1 antibody is pidilizumab (CT-011). Antibodies or antigen binding fragments thereof that compete with any of these antibodies or inhibitors for binding to PD-1 also can be used.

Other anti-PD-1 monoclonal antibodies have been described in, for example, U.S. Patent Nos. 6,808,710, 7,488,802, 8,168,757 and 8,354,509, US Publication No. 2016/0272708, and PCT Publication Nos. WO 2012/145493, WO 2008/156712, WO 2015/112900, WO 2012/145493, WO 2015/112800, WO 2014/206107, WO 2015/35606, WO 2015/085847, WO 2014/179664, WO 2017/020291, WO 2017/020858, WO 2016/197367, WO 2017/024515, WO 2017/025051, WO 2017/123557, WO 2016/106159, WO 2014/194302, WO 2017/040790, WO 2017/133540, WO 2017/132827, WO 2017/024465, WO 2017/025016, WO 2017/106061, WO 2017/19846, WO 2017/024465, WO 2017/025016, WO 2017/132825, and WO 2017/133540, each of which are herein incorporated by reference.

In some embodiments, the anti-PD-1 antibody is selected from the group consisting of nivolumab (also known as OPDIVO^{®}, 5C4, BMS-936558, MDX-1106, and ONO-4538), pembrolizumab (Merck; also known as KEYTRUDA^{®}, lambrolizumab, and MK-3475; *see* WO2008/156712), PDR001 (Novartis; *see* WO 2015/112900), MEDI-0680 (AstraZeneca; also known as AMP-514; *see* WO 2012/145493), cemiplimab (Regeneron; also known as REGN-2810; *see* WO 2015/112800), JS001 (TAIZHOU JUNSHI PHARMA; *see* Si-Yang Liu et al., J. Hematol. Oncol. 10:136 (2017)), BGB-A317 (Beigene; *see* WO 2015/35606 and US 2015/0079109), INCSHR1210 (Jiangsu Hengrui Medicine; also known as SHR-1210; *see* WO 2015/085847; Si-Yang Liu et al., J. Hematol. Oncol. 10:136 (2017)), TSR-042 (Tesaro Biopharmaceutical; also known as ANB011; *see* WO2014/179664), GLS-010 (Wuxi/Harbin Gloria Pharmaceuticals; also known as WBP3055; *see* Si-Yang Liu et al., J. Hematol. Oncol. 10:136 (2017)), AM-0001 (Armo), STI-1110 (Sorrento Therapeutics; *see* WO 2014/194302), AGEN2034 (Agenus; *see* WO 2017/040790), MGA012 (Macrogenics, *see* WO 2017/19846), and IBI308 (Innovent; *see* WO 2017/024465, WO 2017/025016, WO 2017/132825, and WO 2017/133540), which references are herein incorporated by reference.

In another embodiment, the anti-PD-1 antibody or antigen binding fragment thereof cross-competes with pembrolizumab. In some embodiments, the anti-PD-1 antibody or antigen binding fragment thereof binds to the same epitope as pembrolizumab. In certain embodiments, the anti-PD-1 antibody or antigen binding fragment thereof has the same CDRs as pembrolizumab. In another embodiment, the anti-PD-1 antibody is pembrolizumab. Pembrolizumab (also known as "KEYTRUDA^{®}", lambrolizumab, and MK-3475) is a humanized monoclonal IgG4 antibody directed against human cell surface receptor PD-1 (programmed death-1 or programmed cell death-1). Pembrolizumab is described, for example, in U.S. Patent Nos. 8,354,509 and 8,900,587; *see* also http://www.cancer.gov/drugdictionary?cdrid=695789 (last accessed: May 25, 2017). Pembrolizumab has been approved by the FDA for the treatment of relapsed or refractory melanoma.

In other embodiments, the anti-PD-1 antibody or antigen binding fragment thereof cross-competes with MEDI0608. In still other embodiments, the anti-PD-1 antibody or antigen binding fragment thereof binds to the same epitope as MEDI0608. In certain embodiments, the anti-PD-1 antibody has the same CDRs as MEDI0608. In other embodiments, the anti-PD-1 antibody is MEDI0608 (formerly AMP-514), which is a monoclonal antibody. MEDI0608 is described, for example, in U.S. Patent No. 8,609,089 or in http://www.cancer.gov/drugdictionary?cdrid=756047 (last accessed May 25, 2017).

In other embodiments, the anti-PD-1 antibody or antigen binding fragment thereof cross-competes with BGB-A317. In some embodiments, the anti-PD-1 antibody or antigen binding fragment thereof binds the same epitope as BGB-A317. In certain embodiments, the anti-PD-1 antibody or antigen binding fragment thereof has the same CDRs as BGB-A317. In certain embodiments, the anti-PD-1 antibody or antigen binding fragment thereof is BGB-A317, which is a humanized monoclonal antibody. BGB-A317 is described in U.S. Publ. No. 2015/0079109.

Anti-PD-1 antibodies useful for the disclosed compositions also include isolated antibodies that bind specifically to human PD-1 and cross-compete for binding to human PD-1 with nivolumab (*see, e.g.,* U.S. Patent Nos. 8,008,449 and 8,779,105; Int'l Pub. No. WO 2013/173223). The ability of antibodies to cross-compete for binding to an antigen indicates that these antibodies bind to the same epitope region of the antigen and sterically hinder the binding of other cross-competing antibodies to that particular epitope region. These cross-competing antibodies are expected to have functional properties very similar to those of nivolumab by virtue of their binding to the same epitope region of PD-1. Cross-competing antibodies can be readily identified based on their ability to cross-compete with nivolumab in standard PD-1 binding assays such as Biacore analysis, ELISA assays or flow cytometry (*see, e.g.,* Int'l Pub. No. WO 2013/173223).

Anti-PD-1 antibodies usable in the disclosed methods also include isolated antibodies that bind specifically to human PD-1 and cross-compete for binding to human PD-1 with any anti-PD-1 antibody disclosed herein, e.g., nivolumab (*see, e.g.,* U.S. Patent No. 8,008,449 and 8,779,105; WO 2013/173223), which are herein incorporated by reference. In some embodiments, the anti-PD-1 antibody binds the same epitope as any of the anti-PD-1 antibodies described herein, e.g., nivolumab. The ability of antibodies to cross-compete for binding to an antigen indicates that these monoclonal antibodies bind to the same epitope region of the antigen and sterically hinder the binding of other cross-competing antibodies to that particular epitope region. These cross-competing antibodies are expected to have functional properties very similar those of the reference antibody, e.g., nivolumab, by virtue of their binding to the same epitope region of PD-1. Cross-competing antibodies can be readily identified based on their ability to cross-compete with nivolumab in standard PD-1 binding assays such as Biacore analysis, ELISA assays or flow cytometry (*see, e.g.,* WO 2013/173223).

In certain embodiments, antibodies or antigen binding fragments thereof that cross-compete for binding to human PD-1 with, or bind to the same epitope region of human PD-1 as, nivolumab are mAbs. For administration to human subjects, these cross-competing antibodies can be chimeric antibodies, or humanized or human antibodies. Such chimeric, humanized or human mAbs can be prepared and isolated by methods well known in the art.

Anti-PD-1 antibodies useful for the compositions of the disclosed invention also include antigen-binding portions of the above antibodies. It has been amply demonstrated that the antigen-binding function of an antibody can be performed by fragments of a full-length antibody. Examples of binding fragments encompassed within the term "antigen-binding portion" of an antibody include (i) a Fab fragment, a monovalent fragment consisting of the VL, VH, CL and CH1 domains; (ii) a F(ab')₂ fragment, a bivalent fragment comprising two Fab fragments linked by a disulfide bridge at the hinge region; (iii) a Fd fragment consisting of the VH and CH1 domains; and (iv) a Fv fragment consisting of the VL and VH domains of a single arm of an antibody.

Anti-PD-1 antibodies suitable for use in the disclosed compositions are antibodies that bind to PD-1 with high specificity and affinity, block the binding of PD-L1 and or PD-L2, and inhibit the immunosuppressive effect of the PD-1 signaling pathway. In certain embodiments, the anti-PD-1 antibody or antigen-binding portion thereof cross-competes with nivolumab for binding to human PD-1. In other embodiments, the anti-PD-1 antibody or antigen-binding portion thereof is a chimeric, humanized or human monoclonal antibody or a portion thereof. In certain embodiments, the antibody is a humanized antibody. In other embodiments, the antibody is a human antibody. Antibodies of an IgG1, IgG2, IgG3 or IgG4 isotype can be used.

In certain embodiments, the anti-PD-1 antibody or antigen binding fragment thereof comprises a heavy chain constant region which is of a human IgG1 or IgG4 isotype. In certain other embodiments, the sequence of the IgG4 heavy chain constant region of the anti-PD-1 antibody or antigen binding fragment thereof contains an S228P mutation which replaces a serine residue in the hinge region with the proline residue normally found at the corresponding position in IgG1 isotype antibodies. This mutation, which is present in nivolumab, prevents Fab arm exchange with endogenous IgG4 antibodies, while retaining the low affinity for activating Fc receptors associated with wild-type IgG4 antibodies (Wang *et al.*, 2014). In yet other embodiments, the antibody comprises a light chain constant region which is a human kappa or lambda constant region. In other embodiments, the anti-PD-1 antibody or antigen binding fragment thereof is a mAb or an antigen-binding portion thereof. In certain embodiments of any of the therapeutic methods described herein comprising administration of an anti-PD-1 antibody, the anti-PD-1 antibody is nivolumab. In other embodiments, the anti-PD-1 antibody is pembrolizumab. In other embodiments, the anti-PD-1 antibody is chosen from the human antibodies 17D8, 2D3, 4H1, 4A11, 7D3 and 5F4 described in U.S. Patent No. 8,008,449. In still other embodiments, the anti-PD-1 antibody is MEDI0608 (formerly AMP-514), AMP-224, or Pidilizumab (CT-011).

In embodiments, the anti-PD-1 antibody is a bispecific antibody. In embodiments, the anti-PD-1 antibody is a bispecific antibody that binds both PD-1 and LAG-3.

### IIc. Anti-PD-L1 Antibodies

Anti-human-PD-L1 antibodies (or VH and/or VL domains derived therefrom) suitable for use in the invention can be generated using methods well known in the art. Examples of anti-PD-L1 antibodies useful in the methods of the present disclosure include the antibodies disclosed in US Patent No. 9,580,507, incorporated herein by reference. Anti-PD-L1 human monoclonal antibodies disclosed in U.S. Patent No. 9,580,507 have been demonstrated to exhibit one or more of the following characteristics: (a) bind to human PD-L1 with a KD of 1 × 10⁻⁷ M or less, as determined by surface plasmon resonance using a Biacore biosensor system; (b) increase T-cell proliferation in a Mixed Lymphocyte Reaction (MLR) assay; (c) increase interferon-γ production in an MLR assay; (d) increase IL-2 secretion in an MLR assay; (e) stimulate antibody responses; and (f) reverse the effect of T regulatory cells on T cell effector cells and/or dendritic cells. Anti-PD-L1 antibodies usable in the present invention include monoclonal antibodies that bind specifically to human PD-L1 and exhibit at least one, in some embodiments, at least five, of the preceding characteristics.

A recognized anti-PD-L1 antibodies can be used. For example, human anti-PD-L1 antibodies disclosed in U.S. Pat. No. 7,943,743, the contents of which are hereby incorporated by reference, can be used. Such anti-PD-L1 antibodies include 3G10, 12A4 (also referred to as BMS-936559), 10A5, 5F8, 10H10, 1B12, 7H1, 11E6, 12B7, and 13G4. Other art recognized anti-PD-L1 antibodies which can be used include those described in, for example, U.S. Pat. Nos. 7,635,757 and 8,217,149, U.S. Publication No. 2009/0317368, and PCT Publication Nos. WO 2011/066389 and WO 2012/145493, the teachings of which also are hereby incorporated by reference. Other examples of an anti-PD-L1 antibody include atezolizumab (TECENTRIQ; RG7446), or durvalumab (IMFINZI; MEDI4736) or avelumab (Bavencio). Antibodies or antigen binding fragments thereof that compete with any of these art-recognized antibodies or inhibitors for binding to PD-L1 also can be used.

In certain embodiments, the anti-PD-L1 antibody is BMS-936559 (formerly 12A4 or MDX-1105) (*see, e.g.,* U.S. Patent No. 7,943,743; WO 2013/173223). In other embodiments, the anti-PD-L1 antibody is MPDL3280A (also known as RG7446 and atezolizumab) (*see, e.g.,* Herbst et al. 2013 J Clin Oncol 31(suppl):3000; U.S. Patent No. 8,217,149), MEDI4736 (Khleif, 2013, In: Proceedings from the European Cancer Congress 2013; September 27-October 1, 2013; Amsterdam, The Netherlands. Abstract 802), or MSB0010718C (also called Avelumab; *see* US 2014/0341917). In certain embodiments, antibodies that cross-compete for binding to human PD-L1 with, or bind to the same epitope region of human PD-L1 as the above-references PD-L1 antibodies are mAbs. For administration to human subjects, these cross-competing antibodies can be chimeric antibodies, or can be humanized or human antibodies. Such chimeric, humanized or human mAbs can be prepared and isolated by methods well known in the art. In certain embodiments, the anti-PD-L1 antibody is selected from the group consisting of BMS-936559 (also known as 12A4, MDX-1105; see, e.g., U.S. Patent No. 7,943,743 and WO 2013/173223), atezolizumab (Roche; also known as TECENTRIQ^{®}; MPDL3280A, RG7446; see US 8,217,149; see, also, Herbst et al. (2013) J Clin Oncol 31(suppl):3000), durvalumab (AstraZeneca; also known as IMFINZI^{™}, MEDI-4736; see WO 2011/066389), avelumab (Pfizer; also known as BAVENCIO^{®}, MSB-0010718C; see WO 2013/079174), STI-1014 (Sorrento; see WO2013/181634), CX-072 (Cytomx; see WO2016/149201), KN035 (3D Med/Alphamab; see Zhang et al., Cell Discov. 7:3 (March 2017), LY3300054 (Eli Lilly Co.; see, e.g., WO 2017/034916), and CK-301 (Checkpoint Therapeutics; see Gorelik et al., AACR:Abstract 4606 (Apr 2016)).

In certain embodiments, the PD-L1 antibody is atezolizumab (TECENTRIQ^{®}). Atezolizumab is a fully humanized IgG1 monoclonal anti-PD-L1 antibody.

In certain embodiments, the PD-L1 antibody is durvalumab (IMFINZI^{™}). Durvalumab is a human IgG1 kappa monoclonal anti-PD-L1 antibody.

In certain embodiments, the PD-L1 antibody is avelumab (BAVENCIO^{®}). Avelumab is a human IgG1 lambda monoclonal anti-PD-L1 antibody.

In other embodiments, the anti-PD-L1 monoclonal antibody is selected from the group consisting of 28-8, 28-1, 28-12, 29-8, 5H1, and any combination thereof.

Anti-PD-L1 antibodies usable in the disclosed methods also include isolated antibodies that bind specifically to human PD-L1 and cross-compete for binding to human PD-L1 with any anti-PD-L1 antibody disclosed herein, e.g., atezolizumab, durvalumab, and/or avelumab. In some embodiments, the anti-PD-L1 antibody binds the same epitope as any of the anti-PD-L1 antibodies described herein, e.g., atezolizumab, durvalumab, and/or avelumab. The ability of antibodies to cross-compete for binding to an antigen indicates that these antibodies bind to the same epitope region of the antigen and sterically hinder the binding of other cross-competing antibodies to that particular epitope region. These cross-competing antibodies are expected to have functional properties very similar those of the reference antibody, *e*.*g*., atezolizumab and/or avelumab, by virtue of their binding to the same epitope region of PD-L1. Cross-competing antibodies can be readily identified based on their ability to cross-compete with atezolizumab and/or avelumab in standard PD-L1 binding assays such as Biacore analysis, ELISA assays or flow cytometry (*see, e.g.,* WO 2013/173223).

In certain embodiments, the antibodies that cross-compete for binding to human PD-L1 with, or bind to the same epitope region of human PD-L1 antibody as, atezolizumab, durvalumab, and/or avelumab, are monoclonal antibodies. For administration to human subjects, these cross-competing antibodies are chimeric antibodies, engineered antibodies, or humanized or human antibodies. Such chimeric, engineered, humanized or human monoclonal antibodies can be prepared and isolated by methods well known in the art.

Anti-PD-L1 antibodies usable in the methods of the disclosed invention also include antigen-binding portions of the above antibodies. It has been amply demonstrated that the antigen-binding function of an antibody can be performed by fragments of a full-length antibody.

Anti-PD-L1 antibodies suitable for use in the disclosed methods or compositions are antibodies that bind to PD-L1 with high specificity and affinity, block the binding of PD-1, and inhibit the immunosuppressive effect of the PD-1 signaling pathway. In any of the compositions or methods disclosed herein, an anti-PD-L1 "antibody" includes an antigen-binding portion or fragment that binds to PD-L1 and exhibits the functional properties similar to those of whole antibodies in inhibiting receptor binding and up-regulating the immune system. In certain embodiments, the anti-PD-L1 antibody or antigen-binding portion thereof cross-competes with atezolizumab, durvalumab, and/or avelumab for binding to human PD-L1.

### IId. Immunotherapeutic Agents

"Immunotherapeutic agent" and "immuno-oncology drugs" as used in the present disclosure include any agent, compound, or biologic that is capable of modulating the host's immune system. The immunotherapeutic agent can be an immune checkpoint inhibitor an immune checkpoint enhancer or stimulator. The immunotherapeutic agents described herein can be used in combination with one or more additional immunotherapeutic agents (e.g., anti-PD-1 antibody and anti-LAG-3 antibody).

In one embodiment, the immunotherapeutic agent is an immune checkpoint inhibitor. Non-limiting examples of such immunotherapeutic agents include:
(i) a CTLA-4 (CD152) antagonist (e.g., YERVOY^{®} (ipilimumab) (U.S. Patent No. 6,984,720); tremelimumab (formerly ticilimumab and CP-675,206); AGEN-1884; and ATOR-1015 (an anti-OX40 and anti-CTLA-4 bispecific antibody)) Human monoclonal antibodies that bind specifically to CTLA-4 with high affinity have been disclosed in U.S. Patent Nos. 6,984,720. Other anti-CTLA-4 monoclonal antibodies have been described in, for example, U.S. Patent Nos. 5,977,318, 6,051,227, 6,682,736, and 7,034,121 and International Publication Nos. WO 2012/122444, WO 2007/113648, WO 2016/196237, and WO 2000/037504.
(ii) TIM-3 (HAVCR2) antagonist (e.g., TSR-022 and LY3321367);
(iii) TIGIT (T cell immunoreceptor with Ig and ITIM domains) antagonist (e.g., BMS-986207, OMP-313M32, COM902 (CGEN-15137), and AB154);
(iv) an IDO1 (indoleamine-2,3-dioxygenase 1) antagonist (e.g., indoximod (NLG8189, 1-methyl-D-TRP), epacadostat (INCB-024360), KHK2455, PF-06840003 (PCT Publication No. WO 2016/181348 A1), pyrrolididine-2,5-dione derivatives (PCT Publication No. WO 2015/173764 A1), navoximod (RG6078, GDC-0919, NLG919), and BMS-986205 (F001287));
(v) KIR (killer-cell immunoglobulin-like receptor) antagonist (e.g., lirilumab (I-7F9, BMS-980615, or IPH2101) and IPH4102 (an anti-KIR3DL2 monoclonal antibody);
(vi) TDO (tryptophan 2,3-dioxygenase) antagonist (e.g., 4-(indol-3-yl)-pyrazole derivatives (U.S. Patent No. 9,126,984 B2 and U.S. Publication No. 2016/0263087 A1); 3-indol substituted derivatives (PCT Publication Nos. WO 2015140717 A1, WO 2017025868 A1, WO 2016147144 A1), 3-(indol-3-yl)-pyridine derivatives (U.S. Publication No. 20150225367 A1 and PCT Publication No. WO 2015121812 A1);
(vii) dual IDO/TDO antagonist (e.g., small molecule dual IDO/TDO inhibitors as disclosed in PCT Publication Nos. WO 2015150097 A1, WO 2015082499 A2, WO 2016026772 A1, WO 2016071283 A1, WO 2016071293 A2, and WO 2017007700 A1);
(viii) CD40 antagonist (e.g., Lineage BMS3h-56 (U.S. Patent No. 9,475,879), lucatumumab (HCD122 and CHIR-12.12), CHIR-5.9, and dacetuzumab (huS2C6, PRO 64553, RG 3636, SGN 14, SGN-40));
(ix) adenosine A2a receptor (A2aR) antagonist (e.g., CPI-444, PBF-509, istradefylline (KW-6002), preladenant (SCH420814), tozadenant (SYN115), vipadenant (BIIB014), HTL-1071, ST1535, SCH412348, SCH442416, SCH58261, ZM241385, and AZD4635 (a small molecule A2aR inhibitor));
(x) VISTA (V-domain immunoglobulin (Ig)-containing suppressor of T-cell activation) antagonist (e.g., CA-170 (anti-PD-L1/L2 and anti-VISTA small molecule) and JNJ-61610588);
(xi) CEACAM1 (CD66a) antagonist (e.g., CM-24 (MK-6018));
(xii) CEA (carcinoembryonic antigen) antagonist (e.g., cergutuzumab amunaleukin (RG7813, RO-6895882), RG7802 (RO6958688));
(xiii) CD47 antagonist (e.g., HuF9-G4, CC-90002, TTI-621, ALX148, NI-1701, NI-1801, SRF231, and Effi-DEM);
(xiv) PVRIG (poliovirus receptor related immunoglobulin domain containing, CD122R) antagonist (e.g., COM701);
(xv) GARP (glycoprotein A repetitions predominant) antagonist (e.g., ARGX-115);
(xvi) a STING (stimulator of IFN genes) agonist (e.g., 2' or 3'-mono-fluoro substituted, or 2'3'-di-fluoro substituted mixed linkage 2',5' - 3',5' cyclic-di-nucleotides (PCT Publication No. WO 2017/075477 A1); 2'-fluoro substituted, bis-3',5' cyclic-di-nucleotides and 2',2"-diF-Rp,Rp,bis-3',5' cyclic-di-nucleotides (PCT Publication No. WO 2016/145102 A1); and Fluorinated cyclic-di-nucleotides (PCT Publication No. WO 2016/096174 A1);
(xvii) CD20 agonist (e.g., RITUXAN^{®} and ABP 798);
(xviii) CD80 antagonist (e.g., galiximab (IDEC-114) and AV 1142742 (RhuDex);
(xix) CD86 antagonist; and
(xx) CD96 antagonist.

In another embodiment, the immunotherapeutic agent is an immune checkpoint stimulator or enhancer. Non-limiting examples of such immunotherapeutic agents include:
(i) a CD28 agonist (e.g., TGN1412 (an anti-CD28 antibody) and JCAR015 (an anti-CD19-CD28-zeta modified chimeric antigen receptor));
(ii) a CD80 or CD86 agonist (e.g., CTLA4-Ig fusion construct (CTLA-4-IgG4m, RG2077, or RG1046); ORENCIA^{®} (abatacept or BMS-188667); and MGN1601;
(iii) ICOS or ICOS-ligand agonist (e.g., BMS986226, MEDI-570, GSK3359609, JTX-2011, and AMG 570);
(iv) 4-1BB (CD137) agonist (e.g., urelumab and PF-05082566);
(v) OX40 (CD134 or TNFRS4) agonist (e.g., tavolixizumab (MEDI-0562); pogalizumab (MOXR0916, RG7888); GSK3174998; ATOR-1015 (an anti-OX40 and anti-CTLA-4 bispecific antibody); MEDI-6383; MEDI-6469; BMS 986178; PF-04518600; and GINAKIT cells (iC9-GD2-CD28-OX40-expressing T lymphocytes));
(vi) CD27 agonist (e.g., varilumab (CDX-1127));
(vii) CD40 agonist (e.g., ADC-1013 (JNJ-64457107), RG7876 (RO-7009789), HuCD40-M2; APX005M (EPI-0050) (U.S. Patent No. 9,556,278); Chi Lob 7/4 (IgG1 chimeric agonist CD40 monoclonal antibody));
(viii) CD70 agonist (e.g., ARGX-110); and
(ix) GITR agonist (e.g., BMS-986156, TRX518, GWN323, INCAGN01876, and MEDI1873).

In one embodiment, the immunotherapeutic agent is a cytokine, such as a chemokine, an interferon (e.g., interferon-gamma), an interleukin (e.g., aldesleukin (recombinant analog of IL-2 with immunoregulatory and antineoplastic activities), tocilizumab (anti-IL-6 receptor antibody)); a lymphokine, or a member of the tumor necrosis factor (TNF) family (e.g., ATOR-1016, ABBV-621, and adalimumab). Examples of other immunotherapeutic agents include: CSF1R (colony stimulating factor 1 receptor, CD115) antagonist (e.g., emactuzumab); Toll-like receptor 9 (TLR9) agonist (e.g., agatolimod sodium); CD160 (NK1) agonist (e.g., BY55); CD73 antagonist (5'-nucleotidase or ecto-5'-nucleotidase ) (e.g., MEDI9447); iNOS (inducible NO synthase, NOS2) antagonist (e.g., N-Acetyle-cysteine (NAC), aminoguanidine, L-nitroarginine methyl ester, S,S-1,4-phenylene-bis(1,2-ethanediyl)bis-isothiourea); and SHP-1 (Src homology 2 domain-containing protein tyrosine phosphatase 1) antagonist (*see* Watson et al., Biochem Soc Trans 44(2): 356-362 (2016)).

### III. Pharmaceutical Compositions

Pharmaceutical compositions suitable for administration to human patients are typically formulated for parenteral administration, e.g., in a liquid carrier, or suitable for reconstitution into liquid solution or suspension for intravenous administration.

In general, such compositions typically comprise a pharmaceutically acceptable carrier. As used herein, the term "pharmaceutically acceptable" means approved by a government regulatory agency or listed in the U.S. Pharmacopeia or another generally recognized pharmacopeia for use in animals, particularly in humans. The term "carrier" refers to a diluent, adjuvant, excipient, or vehicle with which the compound is administered. Such pharmaceutical carriers can be sterile liquids, such as water and oils, including those of petroleum, animal, vegetable or synthetic origin, such as peanut oil, soybean oil, mineral oil, sesame oil, glycerol polyethylene glycol ricinoleate, and the like. Water or aqueous solution saline and aqueous dextrose and glycerol solutions may be employed as carriers, particularly for injectable solutions (e.g., comprising an anti-PD-1 antibody, an anti-LAG-3 antibody, and/or another immunotherapeutic agent). Liquid compositions for parenteral administration can be formulated for administration by injection or continuous infusion. Routes of administration by injection or infusion include intravenous, intraperitoneal, intramuscular, intrathecal and subcutaneous. In one embodiment, the composition comprising an anti-PD-1 antibody, an anti-LAG-3 antibody, and an immunotherapeutic agent are administered intravenously (e.g., in separate formulations or together (in the same formulation or in separate formulations)).

### IV. Patient Populations

Provided herein are clinical methods for treating solid tumors cancer (e.g., advanced refractory solid tumors) in human patients using a combination of an anti-LAG-3 antibody, a PD-1 pathway inhibitor, and an additional immunotherapeutic agent.

Examples of cancers that may be treated using the methods of the invention, include liver cancer, bone cancer, pancreatic cancer, skin cancer, cancer of the head or neck, oral cancer breast cancer, lung cancer - including small cell and non-small cell lung cancer, cutaneous or intraocular malignant melanoma, renal cancer, uterine cancer, ovarian cancer, colorectal cancer, colon cancer, rectal cancer, cancer of the anal region, stomach cancer, testicular cancer, uterine cancer, carcinoma of the fallopian tubes, carcinoma of the endometrium, carcinoma of the cervix, carcinoma of the vagina, carcinoma of the vulva, non-Hodgkin's lymphoma, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, solid tumors of childhood, lymphocytic lymphoma, cancer of the bladder, cancer of the kidney or ureter, carcinoma of the renal pelvis, neoplasm of the central nervous system (CNS), primary CNS lymphoma, tumor angiogenesis, spinal axis tumor, brain stem glioma, pituitary adenoma, Kaposi's sarcoma, epidermoid cancer, squamous cell cancer, environmentally induced cancers including those induced by asbestos, hematologic malignancies including, for example, multiple myeloma, B-cell lymphoma, Hodgkin lymphoma/primary mediastinal B-cell lymphoma, non-Hodgkin's lymphomas, acute myeloid lymphoma, chronic myelogenous leukemia, chronic lymphoid leukemia, follicular lymphoma, diffuse large B-cell lymphoma, Burkitt's lymphoma, immunoblastic large cell lymphoma, precursor B-lymphoblastic lymphoma, mantle cell lymphoma, acute lymphoblastic leukemia, mycosis fungoides, anaplastic large cell lymphoma, T-cell lymphoma, and precursor T-lymphoblastic lymphoma, and any combinations of said cancers. The present invention is also applicable to treatment of metastatic cancers.

In one embodiment, the human patient suffers from non-small cell lung cancer (NSCLC) or a virally-related cancer (e.g., a human papilloma virus (HPV)-related tumor) or gastric adenocarcinoma. In a particular embodiment, the HPV-related tumor is HPV+ head and neck cancer (HNC). In another particular embodiment, the gastric adenocarcinoma is associated with Epstein-Barr virus (EBV) infection.

Patients can be tested or selected for one or more of the above described clinical attributes prior to, during or after treatment.

### V. Combination Therapy

Combination therapies provided herein involve administration of an anti-LAG-3 antibody, a PD-1 pathway inhibitor, and another immunotherapeutic agent that blocks an inhibitory immune receptor (e.g., a receptor, which upon binding to its natural ligand, inhibits/neutralizes activity, such as cytotoxic activity), to treat subjects having malignant tumors (e.g., advanced refractory solid tumors).

In one embodiment, the invention provides an anti-LAG-3 antibody, an anti-PD-1 antibody, and another immunotherapeutic agent, in combination, according to a defined clinical dosage regimen, to treat subjects having a malignant tumor (e.g., an advanced refractory solid tumor). In a particular embodiment, the anti-LAG-3 antibody is BMS-986016. In another embodiment, the anti-PD-1 antibody is BMS-936558. In another embodiment, dosage regimens are adjusted to provide the optimum desired response (e.g., an effective response).

As used herein, adjunctive or combined administration (coadministration) includes simultaneous administration of the compounds in the same or different dosage form, or separate administration of the compounds (e.g., sequential administration). Thus, the anti-LAG-3 antibody, the PD-1 pathway inhibitor, and the immunotherapeutic agent can be simultaneously administered in a single formulation. Alternatively, the anti-LAG-3 antibody, the PD-1 pathway inhibitor, and the immunotherapeutic agent can be formulated for separate administration and are administered concurrently or sequentially (e.g., one antibody is administered within about 30 minutes prior to administration of the second antibody), and in any order.

For example, the anti-PD-1 antibody can be administered first, followed by (e.g., immediately followed by) the administration of the anti-LAG-3 antibody and/or the immunotherapeutic agent. In one embodiment, the PD-1 pathway inhibitor is administered prior to administration of the anti-LAG-3 antibody and/or the immunotherapeutic agent. In another embodiment, the PD-1 pathway inhibitor is administered after administration of the anti-LAG-3 antibody and/or the immunotherapeutic agent. In another embodiment, the anti-LAG-3 antibody, the PD-1 pathway inhibitor, and the immunotherapeutic agent are administered concurrently. Such concurrent or sequential administration preferably results in all three components being simultaneously present in treated patients.

### VI. Treatment Protocols

Suitable treatment protocols for treating a malignant tumor in a human patient include, for example, administering to the patient an effective amount of each of:
(a) an anti-LAG-3 antibody, such as one comprising CDR1, CDR2 and CDR3 domains of the heavy chain variable region having the sequence set forth in SEQ ID NO:3, and CDR1, CDR2 and CDR3 domains of the light chain variable region having the sequence set forth in SEQ ID NO:5,
(b) an anti-PD-1 antibody, such as one comprising CDR1, CDR2 and CDR3 domains of the heavy chain variable region having the sequence set forth in SEQ ID NO: 19, and CDR1, CDR2 and CDR3 domains of the light chain variable region having the sequence set forth in SEQ ID NO:21,
(c) an immunotherapeutic agent,
wherein the method comprises at least one administration cycle, wherein the cycle is a period of eight weeks, wherein for each of the at least one cycles, at least four doses of the anti-LAG-3 antibody are administered at a flat dose of about 1, 3, 10, 20, 50, 80, 100, 130, 150, 180, 200, 240 or 280 mg, at least four doses of the anti-PD-1 antibody are administered at flat dose of about 50, 80, 100, 130, 150, 180, 200, 240 or 280 mg, and at least four doses of the immunotherapeutic agent are administered at a flat dose of about. In another embodiment, four doses of the anti-LAG-3 antibody are administered at a dose of 0.01, 0.03, 0.25, 0.1, 0.3, 1 or 3, 5, 8 or 10 mg/kg body weight, four doses of the anti-PD-1 antibody are administered at a dose of 0.1, 0.3, 1, 3, 5, 8 or 10 mg/kg body weight, and four doses of the immunotherapeutic agent are administered at a flat dose of about.

In one embodiment, the dose of the anti-LAG-3 antibody, the PD-1 pathway inhibitor, and/or the immunotherapeutic agent is calculated per body weight, e.g., mg/kg body weight. In another embodiment, the dose of the anti-LAG-3 antibody, the PD-1 pathway inhibitor, and/or the immunotherapeutic agent is a flat-fixed dose. In another embodiment, the dose of the anti-LAG-3 antibody, the PD-1 pathway inhibitor, and/or the immunotherapeutic agent is varied over time. For example, the anti-LAG-3 antibody, the PD-1 antibody, and/or the immunotherapeutic agent may be initially administered at a high dose and may be lowered over time. In another embodiment, the anti-LAG-3 antibody, the PD-1 pathway inhibitor, and/or the immunotherapeutic agent is initially administered at a low dose and increased over time.

In another embodiment, the amount of the anti-LAG-3 antibody, the PD-1 pathway inhibitor, and/or the immunotherapeutic agent administered is constant for each dose. In another embodiment, the amount of antibody and/or immunotherapeutic agent administered varies with each dose. For example, the maintenance (or follow-on) dose of the antibody and/or immunotherapeutic agent can be higher or the same as the loading dose which is first administered. In another embodiment, the maintenance dose of the antibody and/or immunotherapeutic agent can be lower or the same as the loading dose.

In another embodiment, the anti-LAG-3 antibody, the PD-1 pathway inhibitor, and/or the immunotherapeutic agent are formulated for intravenous administration. In one embodiment, the anti-PD-1 antibody is administered on Days 1, 15, 29, and 43 of each cycle. In another embodiment, the anti-LAG-3 antibody is administered on Days 1, 15, 29, and 43 of each cycle. In some embodiment, the therapeutic agent is administered on Days 1, 15, 29, and 43 of each cycle.

In other embodiments, the anti-LAG-3 antibody, the PD-1 pathway inhibitor, and/or the immunotherapeutic agent are administered once per week, once every or three two weeks, once per month or as long as a clinical benefit is observed or until there is a complete response, confirmed progressive disease or unmanageable toxicity.

In another embodiment, a cycle of administration is eight weeks, which can be repeated, as necessary. In another embodiment, the treatment consists of up to 12 cycles.

In another embodiment, 4 doses of the PD-1 pathway inhibitor are administered per eight week cycle. In another embodiment, 4 doses of the PD-1 pathway inhibitor are administered per eight week cycle. In some embodiment, 4 doses of the therapeutic agent are administered per eight week cycle.

In another embodiment, the PD-1 pathway inhibitor, the anti-LAG-3 antibody, and/or the immunotherapeutic agent are administered as a first line of treatment (e.g., the initial or first treatment). In another embodiment, the PD-1 pathway inhibitor, the anti-LAG-3 antibody, and/or the immunotherapeutic agent are administered as a second line of treatment (e.g., after the initial or first treatment, including after relapse and/or where the first treatment has failed).

In another aspect, the invention features any of the aforementioned embodiments, wherein the anti-PD-1 antibody is replaced by, or combined with, an anti-PD-L1 or anti-PD-L2 antibody.

### VII. Outcomes

Patients treated according to the methods disclosed herein preferably experience improvement in at least one sign of cancer. In one embodiment, improvement is measured by a reduction in the quantity and/or size of measurable tumor lesions. In another embodiment, lesions can be measured on chest x-rays or CT or MRI films. In another embodiment, cytology or histology can be used to evaluate responsiveness to a therapy.

In one embodiment, the patient treated exhibits a complete response (CR), a partial response (PR), stable disease (SD), immune-related complete disease (irCR), immune-related partial response (irPR), or immune-related stable disease (irSD). In another embodiment, the patient treated experiences tumor shrinkage and/or decrease in growth rate, i.e., suppression of tumor growth. In another embodiment, unwanted cell proliferation is reduced or inhibited. In yet another embodiment, one or more of the following can occur: the number of cancer cells can be reduced; tumor size can be reduced; cancer cell infiltration into peripheral organs can be inhibited, retarded, slowed, or stopped; tumor metastasis can be slowed or inhibited; tumor growth can be inhibited; recurrence of tumor can be prevented or delayed; one or more of the symptoms associated with cancer can be relieved to some extent.

In other embodiments, administration of effective amounts of the anti-LAG-3 antibody, the PD-1 pathway inhibitor, and the immunotherapeutic agent according to any of the methods provided herein produces at least one therapeutic effect selected from the group consisting of reduction in size of a tumor, reduction in number of metastatic lesions appearing over time, complete remission, partial remission, or stable disease. In still other embodiments, the methods of treatment produce a comparable clinical benefit rate (CBR=CR+PR+SD^6 months) better than that achieved by an anti-LAG-3 antibody, a PD-1 pathway inhibitor, or an immunotherapeutic agent alone. In other embodiments, the improvement of clinical benefit rate is about 20% 20%, 30%, 40%, 50%, 60%, 70%, 80% or more compared to an anti-LAG-3 antibody, a PD-1 pathway inhibitor, or an immunotherapeutic agent alone.

### VIII. Kits and Unit Dosage Forms

Also provided herein are kits which include a pharmaceutical composition comprising an anti-LAG-3 antibody (e.g., BMS-986016), a PD-1 pathway inhibitor (e.g., BMS-936558), an immunotherapeutic agent, and a pharmaceutically-acceptable carrier, in a therapeutically effective amount adapted for use in the preceding methods. The kits optionally also can include instructions, e.g., comprising administration schedules, to allow a practitioner (e.g., a physician, nurse, or patient) to administer the composition contained therein to administer the composition to a patient having cancer (e.g., a solid tumor). The kit also can include a syringe.

Optionally, the kits include multiple packages of the single-dose pharmaceutical compositions each containing an effective amount of the anti-LAG-3 antibody, the PD-1 pathway inhibitor, and/or the immunotherapeutic agent for a single administration in accordance with the methods provided above. Instruments or devices necessary for administering the pharmaceutical composition(s) also may be included in the kits. For instance, a kit may provide one or more pre-filled syringes containing an effective amount of the anti-LAG-3 antibody, the anti-PD-1 antibody, and/or the immunotherapeutic agent.

In one embodiment, the present invention provides a kit for treating a malignant tumor in a human patient, the kit comprising, for example:
(a) a dose of an anti-LAG-3 antibody, such as one comprising CDR1, CDR2 and CDR3 domains of the heavy chain variable region having the sequence set forth in SEQ ID NO:3, and CDR1, CDR2 and CDR3 domains of the light chain variable region having the sequence set forth in SEQ ID NO:5;
(b) a dose of PD-1 pathway inhibitor, such as an antibody antibody comprising CDR1, CDR2 and CDR3 domains of the heavy chain variable region having the sequence set forth in SEQ ID NO: 19, and CDR1, CDR2 and CDR3 domains of the light chain variable region having the sequence set forth in SEQ ID NO:21;
(c) a dose of an immunotherapeutic agent; and
(d) instructions for using the anti-LAG-3 antibody, the anti-PD-1 pathway inhibitor, and the immunotherapeutic agent in the methods described herein.

The following examples are merely illustrative and should not be construed as limiting the scope of this disclosure in any way as many variations and equivalents will become apparent to those skilled in the art upon reading the present disclosure.

### EXAMPLES

### EXAMPLE 1

### Treatment of Malignant Tumor with Nivolumab Monotherapy v. Nivolumab + BMS 986016 (anti-LAG-3 antibody) + Immunotherapeutic Agent

To determine if there is an improvement in overall survival (OS) compared to nivolumab monotherapy, a pharmaceutical composition comprising a combination of nivolumab, BMS 986016, and an immunotherapeutic agent is tested in patients with recurrent metastatic tumors. A formal pairwise comparison of OS among experimental arms (i.e., nivolumab monotherapy v. nivolumab + BMS 986016 + immunotherapeutic agent combination therapy) is conducted.

The study also compares the progression-free survival (PFS) and the objective response rate (ORR), based on Blinded Independent Central Review (BICR) assessment of the combination of nivolumab, BMS 986016, and an immunotherapeutic agent ("combined therapy") to nivolumab monotherapy in subjects with recurrent metastatic tumor. Differences in PFS and ORR between the different arms are evaluated.

Other objectives of the study include: 1) assessing the overall safety and tolerability of the combined therapy compared to the nivolumab monotherapy; 2) characterizing pharmacokinetics of the combined therapy and explore exposure-safety and exposure-efficacy relationships; 3) characterizing the immunogenicity of the combined therapy; 4) characterizing immune correlates of the combined therapy; 5) assessing predictive tumor and peripheral biomarkers of clinical response to the combined therapy; and 6) assessing overall health status using the EQ-5D index and visual analogue scale in subjects treated with the combined therapy.

### Methods

### Study Design

The study is an open label, 2-arm, randomized study in adult (greater than or equal to 18 years of age) male and female subjects, with stage IV or recurrent non-small cell lung cancer (NSCLC), PD-L1 positive or negative, previously untreated for advanced disease.

Key inclusion criteria include: 1) ECOG Performance Status of greater than or equal to 1; 2) Patients with histologically confirmed Stage IV or recurrent NSCLC (per the 7th International Association for the Study of Lung Cancer classification squamous or non-squamous histology), with no prior systemic anticancer therapy (including EGFR and ALK inhibitors) given as primary therapy for advanced or metastatic disease; and 3) Measurable disease by CT or MRI per RECIST 1.1 criteria.

Key exclusion criteria include: 1) Subjects with known EGFR mutations which are sensitive to available targeted inhibitor therapy; 2) Subjects with known ALK translocations which are sensitive to available targeted inhibitor therapy; 3) Subjects with untreated CNS metastases; 4) Subjects with an active, known or suspected autoimmune disease (subjects with type I diabetes mellitus, hypothyroidism only requiring hormone replacement, skin disorders (such as vitiligo, psoriasis, or alopecia) not requiring systemic treatment, or conditions not expected to recur in the absence of an external trigger are permitted to enroll); and 5) Subjects with a condition requiring systemic treatment with either corticosteroids (> 10 mg daily prednisone equivalent) or other immunosuppressive medications within 14 days of randomization (inhaled or topical steroids, and adrenal replacement steroid > 10 mg daily prednisone equivalent, are permitted in the absence of active autoimmune disease).

Subjects are randomized 1:1:1:1, and stratified by histology (Squamous versus Non-squamous) and PD-L1 status. PD-L1 status is determined by immunohistochemical (IHC) staining of PD-L1 protein in a tumor sample submitted prior to randomization. Subjects are identified as PD-L1 positive if greater than or equal to 5% tumor cell membrane staining in a minimum of a hundred evaluable tumor cells is observed, or PD-L1 negative if less than 5% tumor cell membrane staining in a minimum of a hundred evaluable tumor cells is observed.

Subjects receive open-label treatment from one of the two study arms. The dosing schedule is shown in **Table 1.**

**Table 1. Dosing Schedule***

| | **Week 1** | **Week 2** | **Week 3** | **Week 4** | **Week 5** | **Week 6** |
|---|---|---|---|---|---|---|
| **Arm A:** | **Day 1** | | **Day 1** | | **Day 1** | |
| Nivolumab 240mg^{a} q 2 weeks | Nivolumab | | Nivolumab | | Nivolumab | |
| **Arm B:** | **Day 1** | | | **Day 1** | | |
| Nivolumab 1 mg/kg + BMS 986016 1 mg/kg q 3weeks x4^{a} + Immunotherapeutic Agent 1 mg/kg | Nivolumab + BMS 986016 + Immunotherapeutic agent | | | Nivolumab + BMS 986016 + Immunotherapeutic agent | | |

| | | | | | | |
|---|---|---|---|---|---|---|
| * Both nivolumab and BMS 986016 may be administered as 30 minute infusions ^{a} continues until disease progression, discontinuation due to unacceptable toxicity, withdrawal of consent, or study closure | | | | | | |

On-study tumor assessment begins at Week 6 post randomization (± 7 days) and is performed every 6 weeks (± 7 days) until Week 48. After Week 48, tumor assessment is performed every 12 weeks (± 7 days) until progression or treatment discontinuation, whichever occurs later. Subjects receiving nivolumab or the combined therapy beyond investigator-assessed RECIST 1.1-defined progression must also continue tumor assessments until such treatment is discontinued. Enrollment will end after approximately 1200 subjects are randomized. The primary endpoint of the study is Overall Survival (OS). The duration of the study from start of enrollment to analysis of the primary OS endpoint is expected to be approximately 48 months.

### Study Arms

### Nivolumab Monotherapy (Arm A)

Nivolumab 240 mg is administered intravenously (IV) on day 1 of each cycle over 30 minutes every 2 weeks until disease progression, discontinuation due to unacceptable toxicity, withdrawal of consent or study closure. Treatment beyond initial investigator-assessed RECIST 1.1-defined progression is permitted if the subject has investigator-assessed clinical benefit and is tolerating treatment. Upon completion of dosing, subjects enter the Follow-up Phase.

### Nivolumab + BMS 986016 + Immunotherapeutic Agent Combined Therapy (Arm B)

Nivolumab 1 mg/kg is administered IV over 30 minutes combined with BMS 986016 1 mg/kg and immunotherapeutic agent administered IV over 30 minutes every 3 weeks for four cycles until disease progression, unacceptable toxicity, withdrawal of consent, or study closure. Treatment beyond initial investigator-assessed RECIST 1.1-defined progression is permitted if the subject has investigator-assessed clinical benefit and is tolerating treatment. Upon completion of dosing, subjects enter the Follow-up Phase.

### Post-Treatment Follow-up

The post-treatment follow-up begins when the decision to discontinue a subject from all treatment is made; this includes optional continuation maintenance therapy. Subjects who discontinue treatment for reasons other than disease progression will continue to have tumor assessments (if clinically feasible) until progression or the start of any subsequent therapy, whichever occurs first. Subjects are followed for drug-related toxicities until these toxicities resolve, return to baseline or are deemed irreversible. All adverse events are documented for a minimum of 100 days after the last dose of study medication. After completion of the first two follow-up visits, subjects are followed every 3 months for survival.

### Sample Size

Approximately 1200 subjects are randomized to the 4 treatment groups in a 1:1:1:1 ratio. The final analysis is conducted after 257 events occur in the control group, and these events will be monitored by the un-blinded independent statistician supporting the DMC. Assuming a 20% screening failure rate, it is estimated that approximately 1500 subjects will be enrolled in order to have 1200 subjects randomized, assuming a piecewise constant accrual rate (8 subjects/month during Months 1 to 2, 40 subjects/month during Months 3 to 4, 85 subjects/month during Months 5 to 6, 138 subjects/month during Months 7 to 8, 170 subjects/month after Month 8), it will take approximately 48 months to obtain the required number of death for the final OS analysis (14 months for accrual and 34 months for survival follow up).

### End Point

OS is a primary endpoint for this study. If OS superiority is demonstrated for at least one comparison, a gate keeping testing approach for the key secondary endpoints will be applied to additional experimental vs. control comparisons as described in the statistical analysis plan. Key secondary endpoints include PFS and ORR based on BICR assessments.

Each of the three primary OS analyses will be conducted using a two-sided log-rank test stratified by histology and PD-L1 status in all randomized subjects using Hochberg's procedure to address multiplicity. Hazard ratios (HR) and corresponding two-sided (1-adjusted α) % confidence intervals (CI) will be estimated using a Cox proportional hazard model, with treatment group as a single covariate, stratified by the above factors. OS curves, OS medians with 95% CIs, and OS rates at 12 and 24 months with 95% CIs will be estimated using Kaplan-Meier methodology. If OS superiority is demonstrated for at least one comparison, a gatekeeping testing approach for the key secondary endpoints will be applied to additional experimental vs. control comparisons as described in the statistical analysis plan. The key secondary endpoints will be tested in the following hierarchical order:
1) PFS (based on BICR assessments) analyses will be conducted using a two-sided log-rank test stratified by histology and PD-L1 status in all randomized subjects to compare each of the three experimental treatments to the control group. HRs and corresponding two-sided (1-adjusted α) % CIs will be estimated using a Cox proportional hazard model, with treatment group as a single covariate, stratified by the above factors. PFS curves, PFS medians with 95% CIs, and PFS rates at 6 and 12 months with 95% CIs will be estimated using Kaplan-Meier methodology.
2) ORR (based on BICR assessments) analyses will be conducted using a two-sided Cochran-Mantel-Haenszel (CMH) test stratified by PD-L1 status and histology to compare each of the three experiment treatments to the control group. Associated odds ratios and (1-adjusted α) % CI will also be calculated. Additionally, ORRs and their corresponding 95% exact CIs will be calculated using the Clopper-Pearson method for each of the four treatment groups.
3) Pairwise comparison of OS among experimental arms will be conducted using a two-sided log-rank test stratified by histology and PD-L1 status. HRs and corresponding two-sided (1-adjusted α) % CIs will be estimated using a Cox proportional hazard model, with treatment group as a single covariate, stratified by the above factors.

### Analyses

Analyses of PD-L1 expression will be descriptive. Distribution of PD-L1 expression will be examined based on overall population. Potential associations between PD-L1 expression and efficacy measures (ORR, OS and PFS) will be assessed. If there is an indication of a meaningful association, further evaluation will be conducted to explore PD-L1 expression as a predictive biomarker by estimating the interaction effect between PD-L1 expression and treatment.

The results show whether the combined therapy (nivolumab + BMS 986016 + immunotherapeutic agent) will improve overall survival (OS) compared with nivolumab monotherapy.

### SEQUENCES

SEQ ID NO:1 Heavy Chain Amino Acid Sequence; Anti-LAG-3 mAb (BMS-986016) 
QVQLQQWGAGLLKPSETLSLTCAVYGGSFSDYYWNWIRQPPGKGLEWIGEINHRGSTNSNPSLKS
RVTLSLDTSKNQFSLKLRSVTAADTAVYYCAFGYSDYEYNWFDPWGQGTLVTVSSASTKGPSVFP
LAPCSRSTSESTAALGCLVKDYFPEPVTVSWNSGALTSGVHTFPAVLQSSGLYSLSSVVTVPSSS
LGTKTYTCNVDHKPSNTKVDKRVESKYGPPCPPCPAPEFLGGPSVFLFPPKPKDTLMISRTPEVT
CVVVDVSQEDPEVQFNWYVDGVEVHNAKTKPREEQFNSTYRVVSVLTVLHQDWLNGKEYKCKVSN
KGLPSSIEKTISKAKGQPREPQVYTLPPSQEEMTKNQVSLTCLVKGFYPSDIAVEWESNGQPENN
YKTTPPVLDSDGSFFLYSRLTVDKSRWQEGNVFSCSVMHEALHNHYTQKSLSLSLGK
SEQ ID NO:2 Light Chain Amino Acid Sequence; Anti-LAG-3 mAb (BMS-986016) 
EIVLTQSPATLSLSPGERATLSCRASQSISSYLAWYQQKPGQAPRLLIYDASNRATGIPARFSGS
GSGTDFTLTISSLEPEDFAVYYCQQRSNWPLTFGQGTNLEIKRTVAAPSVFIFPPSDEQLKSGTA
SVVCLLNNFYPREAKVQWKVDNALQSGNSQESVTEQDSKDSTYSLSSTLTLSKADYEKHKVYACE
VTHQGLSSPVTKSFNRGEC
SEQ ID NO:3 Heavy Chain Variable Region (VH) Amino Acid Sequence; Anti-LAG-3 mAb (BMS-986016) 
QVQLQQWGAGLLKPSETLSLTCAVYGGSFSDYYWNWIRQPPGKGLEWIGEINHRGSTN
SNPSLKSRVTLSLDTSKNQFSLKLRSVTAADTAVYYCAFGYSDYEYNWFDPWGQGTLV
TVSS
SEQ ID NO:4 Heavy Chain Variable Region (VH) Nucleotide Sequence; Anti-LAG-3 mAb (BMS-986016) 
caggtgcagctacagcagtggggcgcaggactgttgaagccttcggagaccctgtccctcacctg
cgctgtctatggtgggtccttcagtgattactactggaactggatccgccagcccccagggaagg
ggctggagtggattggggaaatcaatcatcgtggaagcaccaactccaacccgtccctcaagagt
cgagtcaccctatcactagacacgtccaagaaccagttctccctgaagctgaggtctgtgaccgc
cgcggacacggctgtgtattactgtgcgtttggatatagtgactacgagtacaactggttcgacc
cctggggccagggaaccctggtcaccgtctcctca
SEQ ID NO:5 Light Chain Variable Region (VL) Amino Acid Sequence; Anti-LAG-3 mAb (BMS-986016) 
EIVLTQSPATLSLSPGERATLSCRASQSISSYLAWYQQKPGQAPRLLIYDASNRATGIPARFSGS
GSGTDFTLTISSLEPEDFAVYYCQQRSNWPLTFGQGTNLEIK
SEQ ID NO:6 Light Chain Variable Region (VL) Nucleotide Sequence; Anti-LAG-3 mAb (BMS-986016) 
gaaattgtgttgacacagtctccagccaccctgtctttgtctccaggggaaagagccaccctctc
ctgcagggccagtcagagtattagcagctacttagcctggtaccaacagaaacctggccaggctc
ccaggctcctcatctatgatgcatccaacagggccactggcatcccagccaggttcagtggcagt
gggtctgggacagacttcactctcaccatcagcagcctagagcctgaagattttgcagtttatta
ctgtcagcagcgtagcaactggcctctcacttttggccaggggaccaacctggagatcaaa
SEQ ID NO:7 Heavy Chain CDR1 Amino Acid Sequence; Anti-LAG-3 mAb (BMS-986016)
   DYYWN
SEQ ID NO:8 Heavy Chain CDR2 Amino Acid Sequence; Anti-LAG-3 mAb (BMS-986016)
   EINHRGSTNSNPSLKS
SEQ ID NO:9 Heavy Chain CDR3 Amino Acid Sequence; Anti-LAG-3 mAb (BMS-986016)
   GYSDYEYNWFDP
SEQ ID NO:10 Light Chain CDR1 Amino Acid Sequence; Anti-LAG-3 mAb (BMS-986016)
   RASQSISSYLA
SEQ ID NO:11 Light Chain CDR2 Amino Acid Sequence; Anti-LAG-3 mAb (BMS-986016)
   DASNRAT
SEQ ID NO:12 Light Chain CDR3 Amino Acid Sequence; Anti-LAG-3 mAb (BMS-986016)
   QQRSNWPLT
SEQ ID NO:13 Human LAG-3 Amino Acid Sequence 
MWEAQFLGLLFLQPLWVAPVKPLQPGAEVPVVWAQEGAPAQLPCSPTIPLQDLSLLRRAGVTWQH
QPDSGPPAAAPGHPLAPGHPAAPSSWGPRPRRYTVLSVGPGGLRSGRLPLQPRVQLDERGRQRG
DFSLWLRPARRADAGEYRAAVHLRDRALSCRLRLRLGQASMTASPPGSLRASDWVILNCSFSRPD
RPASVHWFRNRGQGRVPVRESPHHHLAESFLFLPQVSPMDSGPWGCILTYRDGFNVSIMYNLTVL
GLEPPTPLTVYAGAGSRVGLPCRLPAGVGTRSFLTAKWTPPGGGPDLLVTGDNGDFTLRLEDVSQ
AQAGTYTCHIHLQEQQLNATVTLAIITVTPKSFGSPGSLGKLLCEVTPVSGQERFVWSSLDTPSQ
RSFSGPWLEAQEAQLLSQPWQCQLYQGERLLGAAVYFTELSSPGAQRSGRAPGALPAGHLLLFLT
LGVLSLLLLVTGAFGFHLWRRQWRPRRFSALEQGIHPPQAQSKIEELEQEPEPEPEPEPEPEPEP
EPEQL
SEQ ID NO:14 LAG-3 Epitope
   PGHPLAPG
SEQ ID NO:15 LAG-3 Epitope
   HPAAPSSW
SEQ ID NO:16 LAG-3 Epitope
   PAAPSSWG
SEQ ID NO:17 Heavy Chain Amino Acid Sequence; Anti-PD-1 mAb (BMS936558) 
QVQLVESGGGVVQPGRSLRLDCKASGITFSNSGMHWVRQAPGKGLEWVAVIWYDGSKRYYADSVK
GRFTISRDNSKNTLFLQMNSLRAEDTAVYYCATNDDYWGQGTLVTVSSASTKGPSVFPLAPCSRS
 
TSESTAALGCLVKDYFPEPVTVSWNSGALTSGVHTFPAVLQSSGLYSLSSVVTVPSSSLGTKTYT
CNVDHKPSNTKVDKRVESKYGPPCPPCPAPEFLGGPSVFLFPPKPKDTLMISRTPEVTCVVVDVS
QEDPEVQFNWYVDGVEVHNAKTKPREEQFNSTYRVVSVLTVLHQDWLNGKEYKCKVSNKGLPSSI
EKTISKAKGQPREPQVYTLPPSQEEMTKNQVSLTCLVKGFYPSDIAVEWESNGQPENNYKTTPPV
LDSDGSFFLYSRLTVDKSRWQEGNVFSCSVMHEALHNHYTQKSLSLSLGK
SEQ ID NO:18 Light Chain Amino Acid Sequence; Anti-PD-1 mAb (BMS936558) 
EIVLTQSPATLSLSPGERATLSCRASQSVSSYLAWYQQKPGQAPRLLIYDASNRATGIPARFSGS
GSGTDFTLTISSLEPEDFAVYYCQQSSNWPRTFGQGTKVEIKRTVAAPSVFIFPPSDEQLKSGTA
SVVCLLNNFYPREAKVQWKVDNALQSGNSQESVTEQDSKDSTYSLSSTLLSKADYEKHKVYACEV
THQGLSSPVTKSFNRGEC
SEQ ID NO:19 Heavy Chain Variable Region (VH) Amino Acid Sequence; Anti-PD-1 mAb (BMS936558)
SEQ ID NO:20 Heavy Chain Variable Region (VH) Nucleotide Sequence; Anti-PD-1 mAb (BMS936558) 
caggtgcagctggtggagtctgggggaggcgtggtccagcctgggaggtccctgagactcgactg
taaagcgtctggaatcaccttcagtaactctggcatgcactgggtccgccaggctccaggcaagg
ggctggagtgggtggcagttatttggtatgatggaagtaaaagatactatgcagactccgtgaag
ggccgattcaccatctccagagacaattccaagaacacgctgtttctgcaaatgaacagcctgag
agccgaggacacggctgtgtattactgtgcgacaaacgacgactactggggccagggaaccctgg
tcaccgtctcctca
SEQ ID NO:21 Light Chain Variable Region (VL) Amino Acid Sequence; Anti-PD-1 mAb (BMS936558)
SEQ ID NO:22 Light Chain Variable Region (VL) Nucleotide Sequence; Anti-PD-1 mAb (BMS936558) 
gaaattgtgttgacacagtctccagccaccctgtctttgtctccaggggaaagagccaccctctc
ctgcagggccagtcagagtgttagtagttacttagcctggtaccaacagaaacctggccaggctc
ccaggctcctcatctatgatgcatccaacagggccactggcatcccagccaggttcagtggcagt
gggtctgggacagacttcactctcaccatcagcagcctagagcctgaagattttgcagtttatta
ctgtcagcagagtagcaactggcctcggacgttcggccaagggaccaaggtggaaatcaaa
SEQ ID NO:23 Heavy Chain CDR1 Amino Acid Sequence; Anti-PD-1 mAb (BMS936558)
   NSGMH
SEQ ID NO:24 Heavy Chain CDR2 Amino Acid Sequence; Anti-PD-1 mAb (BMS936558)
   VIWYDGSKRYYADSVKG
SEQ ID NO:25 Heavy Chain CDR3 Amino Acid Sequence; Anti-PD-1 mAb (BMS936558)
   NDDY
SEQ ID NO:26 Light Chain CDR1 Amino Acid Sequence; Anti-PD-1 mAb (BMS936558)
   RASQSVSSYLA
SEQ ID NO:27 Light Chain CDR2 Amino Acid Sequence; Anti-PD-1 mAb (BMS936558)
   DASNRAT
SEQ ID NO:28 Light Chain CDR3 Amino Acid Sequence; Anti-PD-1 mAb (BMS936558)
   QQSSNWPRT
SEQ ID NO:29 Complete Homo sapiens PD-1 sequence 
agtttcccttccgctcacctccgcctgagcagtggagaaggcggcactctggtggggctgctcca
ggcatgcagatcccacaggcgccctggccagtcgtctggcggtgctacaactgggctggcggcc
aggatggttcttagactccccagacaggccctggaacccccccaccttcttcccagccctgctcg
tggtgaccgaaggggacaacgccaccttcacctgcagcttctccaacacatcggagagcttcgtg
ctaaactggtaccgcatgagccccagcaaccagacggacaagctggccgccttccccgaggaccg
cagccagcccggccaggactgccgcttccgtgtcacacaactgcccaacgggcgtgacttccaca
tgagcgtggtcagggcccggcgcaatgacagcggcacctacctctgtggggccatctccctggcc
cccaaggcgcagatcaaagagagcctgcgggcagagctcagggtgacagagagaagggcagaagt
gcccacagcccaccccagcccctcacccaggccagccggccagttccaaaccctggtggttggtg
tcgtgggcggcctgctgggcagcctggtgctgctagtctgggtcctggccgtcatctgctcccgg
gccgcacgagggacaataggagccaggcgcaccggccagcccctgaaggaggacccctcagccgt
gcctgtgttctctgtggactatggggagctggatttccagtggcgagagaagaccccggagcccc
ccgtgccctgtgtccctgagcagacggagtatgccaccattgtctttcctagcggaatgggcacc
tcatcccccgcccgcagggggctcagccgacggccctcggagtgcccagccactgaggcctgagga
tggacactgctcttggcccctctgaccggcttccttggccaccagtgttctgcagaccctccacc
atgagcccgggtcagcgcatttcctcaggagaagcaggcagggtgcaggccattgcaggccgtcc
aggggctgagctgcctgggggcgaccggggctccagcctgcacctgcaccaggcacagccccacc
acaggactcatgtctcaatgcccacagtgagcccaggcagcaggtgtcaccgtcccctacagga
gggccagatgcagtcactgcttcaggtcctgccagcacagagctgcctgcgtccagctccctgaa
tctctgctgctgctgctgctgctgctgctgctgcctgcggcccggggctgaaggcgccgtggccc
tgcctgacgccccggagcctcctgcctgaacttgggggctggttggagatggccttggagcagcc
aaggtgcccctggcagtggcatcccgaaacgccctggacgcagggcccaagactgggcacaggag
tgggaggtacatggggctggggactccccaggagttatctgctccctgcaggcctagagaagttt
cagggaaggtcagaagagctcctggctgtggtgggcagggcaggaaacccctcccacctttacac
atgcccaggcagcacctcaggccctttgtggggcagggaagctgaggcagtaagcgggcaggcag
agctggaggcctttcaggccagccagcactctggcctcctgccgccgcattccaccccagcccct
cacaccactcgggagagggacatcctacggtcccaaggtcaggagggcagggctggggttgactc
aggcccctcccagctgtggccacctgggtgttgggagggcagaagtgcaggcacctagggccccc
catgtgcccaccctgggagctctccttggaacccattcctgaaattatttaaaggggttggccgg
gctcccaccagggcctgggtgggaaggtacaggcgttcccccggggcctagtacccccgcgtggc
ctatccactcctcacatccacacactgcacccccactcctggggcagggccaccagcatccaggc
ggccagcaggcacctgagtggctgggacaaggatcccccttccctgtggttctattatattata
attataattaaatatgagagcatgct
SEQ ID NO:30 Heavy Chain Nucleotide Sequence; Anti-LAG-3 mAb (BMS-986016) 
caggtgcagctacagcagtggggcgcaggactgttgaagccttcggagaccctgtccctcacctg
cgctgtctatggtgggtccttcagtgattactactggaactggatccgccagcccccagggaagg
ggctggagtggattggggaaatcaatcatcgtggaagcaccaactccaacccgtccctcaagagt
cgagtcaccctatcactagacacgtccaagaaccagttctccctgaagctgaggtctgtgaccgc
cgcggacacggctgtgtattactgtgcgtttggatatagtgactacgagtacaactggttcgacc
cctggggccagggaaccctggtcaccgtctcctcagctagcaccaagggcccatccgtcttcccc
ctggcgccctgctccaggagcacctccgagagcacagccgccctgggctgcctggtcaaggacta
cttccccgaaccggtgacggtgtcgtggaactcaggcgccctgaccagcggcgtgcacaccttcc
cggctgtcctacagtcctcaggactctactccctcagcagcgtggtgaccgtgccctccagcagc
ttgggcacgaagacctacacctgcaacgtagatcacaagcccagcaacaccaaggtggacaagag
agttgagtccaaatatggtcccccatgcccaccatgcccagcacctgagttcctggggggaccat
cagtcttcctgttccccccaaaacccaaggacactctcatgatctcccggacccctgaggtcacg
tgcgtggtggtggacgtgagccaggaagaccccgaggtccagttcaactggtacgtggatggcgt
ggaggtgcataatgccaagacaaagccgcgggaggagcagttcaacagcacgtaccgtgtggtca
gcgtcctcaccgtcctgcaccaggactggctgaacggcaaggagtacaagtgcaaggtctccaac
aaaggcctcccgtcctccatcgagaaaaccatctccaaagccaaagggcagccccgagagccaca
ggtgtacaccctgcccccatcccaggaggagatgaccaagaaccaggtcagcctgacctgcctgg
tcaaaggcttctaccccagcgacatcgccgtggagtgggagagcaatgggcagccggagaacaac
tacaagaccacgcctcccgtgctggactccgacggctccttcttcctctacagcaggctaaccgt
ggacaagagcaggtggcaggaggggaatgtcttctcatgctccgtgatgcatgaggctctgcaca
accactacacacagaagagcctctccctgtctctgggtaaatga
SEQ ID NO:31 Light Chain Nucleotide Sequence; Anti-LAG-3 mAb (BMS-986016) 
gaaattgtgttgacacagtctccagccaccctgtctttgtctccaggggaaagagccaccctctc
ctgcagggccagtcagagtattagcagctacttagcctggtaccaacagaaacctggccaggctc
ccaggctcctcatctatgatgcatccaacagggccactggcatcccagccaggttcagtggcagt
gggtctgggacagacttcactctcaccatcagcagcctagagcctgaagattttgcagtttatta
ctgtcagcagcgtagcaactggcctctcacttttggccaggggaccaacctggagatcaaacgta
cggtggctgcaccatctgtcttcatcttcccgccatctgatgagcagttgaaatctggaactgcc
tctgttgtgtgcctgctgaataacttctatcccagagaggccaaagtacagtggaaggtggataa
cgccctccaatcgggtaactcccaggagagtgtcacagagcaggacagcaaggacagcacctaca
gcctcagcagcaccctgacgctgagcaaagcagactacgagaaacacaaagtctacgcctgcgaa
gtcacccatcagggcctgagctcgcccgtcacaaagagcttcaacaggggagagtgttag
SEQ ID NO:32 Motif
   MYPPPY
SEQ ID NO: 33 Heavy Chain Amino Acid Sequence; Anti-ICOS mAb (BMS986226) 
EVQLVESGGGLVKPGGSLRLSCAASGFTFSDYFMHWVRQAPGKGLEWVGVIDTKSFNYATYYSDL
VKGRFTISRDDSKNTLYLQMNSLKTEDTAVYYCTATIAVPYYFDYWGQGTLVTVSSASTKGPSVF
PLAPSSKSTSGGTAALGCLVKDYFPEPVTVSWNSGALTSGVHTFPAVLQSSGLYSLSSVVTVPSS
SLGTQTYICNVNHKPSNTKVDKRVEPKSCDKTHTCPPCPAPELLGGPSVFLFPPKPKDTLMISRT
PEVTCVVVDVEHEDPEVKFNWYVDGVEVHNAKTKPREEQYNSTYRVVSVLTVLHQDWLNGKEYKC
KVSNKALPAPIEKTISKAKGQPREPQVYTLPPSREEMTKNQVSLTCLVKGFYPSDIAVEWESNGQ
PENNYKTTPPVLDSDGSFFLYSKLTVDKSRWQQGNVFSCSVMHEALHNHYTQKSLSLSPG
SEQ ID NO: 34 Light Chain Amino Acid Sequence; Anti-ICOS mAb (BMS986226) 
DIQMTQSPSSLSASVGDRVTITCQASQDISNYLSWYQQKPGKAPKLLIYYTNLLAEGVPSRFSGS
GSGTDFTFTISSLQPEDIATYYCQQYYNYRTFGPGTKVDIKRTVAAPSVFIFPPSDEQLKSGTAS
VVCLLNNFYPREAKVQWKVDNALQSGNSQESVTEQDSKDSTYSLSSTLTLSKADYEKHKVYACEV
THQGLSSPVTKSFNRGEC
SEQ ID NO: 35 Heavy Chain Variable Region (VH) Amino Acid Sequence; Anti-ICOS mAb (BMS986226) 
EVQLVESGGGLVKPGGSLRLSCAASGFTFSDYFMHWVRQAPGKGLEWVGVIDTKSFNYATYYSDL
VKGRFTISRDDSKNTLYLQMNSLKTEDTAVYYCTATIAVPYYFDYWGQGTLVTVSS
SEQ ID NO: 36 Light Chain Variable Region (VL) Amino Acid Sequence; Anti-ICOS mAb (BMS986226) 
DIQMTQSPSSLSASVGDRVTITCQASQDISNYLSWYQQKPGKAPKLLIYYTNLLAEGVPSRFSGS
GSGTDFTFTISSLQPEDIATYYCQQYYNYRTFGPGTKVDIK
SEQ ID NO: 37 Heavy Chain CDR1 Amino Acid Sequence; Anti-ICOS mAb (BMS986226)
   GFTFSDYFMH
SEQ ID NO: 38 Heavy Chain CDR2 Amino Acid Sequence; Anti-ICOS mAb (BMS986226)
   VIDTKSFNYATYYSDLVKG
SEQ ID NO: 39 Heavy Chain CDR3 Amino Acid Sequence; Anti-ICOS mAb (BMS986226)
   TIAVPYYFDY
SEQ ID NO: 40 Light Chain CDR1 Amino Acid Sequence; Anti-ICOS mAb (BMS986226)
   QASQDISNYLS
SEQ ID NO: 41 Light Chain CDR2 Amino Acid Sequence; Anti-ICOS mAb (BMS986226)
   YTNLLAE
SEQ ID NO: 42 Light Chain CDR3 Amino Acid Sequence; Anti-ICOS mAb (BMS986226)
   QQYYNYRT
SEQ ID NO: 43 Light Chain CDR3 Amino Acid Sequence; Anti-ICOS mAb (BMS986226)
   QQYYNYRT
SEQ ID NO: 44 Lymphocyte Activation Gene 3 Protein Amino Acid Sequence (Homo Sapiens, NP_002277) 
MWEAQFLGLLFLQPLWVAPVKPLQPGAEVPVVWAQEGAPAQLPCSPTIPLQDLSLLRRAGVTWQHQPDSG
PPAAAPGHPLAPGPHPAAPSSWGPRPRRYTVLSVGPGGLRSGRLPLQPRVQLDERGRQRGDFSLWLRPAR
 
RADAGEYRAAVHLRDRALSCRLRLRLGQASMTASPPGSLRASDWVILNCSFSRPDRPASVHWFRNRGQGR
VPVRESPHHHLAESFLFLPQVSPMDSGPWGCILTYRDGFNVSIMYNLTVLGLEPPTPLTVYAGAGSRVGL
PCRLPAGVGTRSFLTAKWTPPGGGPDLLVTGDNGDFTLRLEDVSQAQAGTYTCHIHLQEQQLNATVTLAI
ITVTPKSFGSPGSLGKLLCEVTPVSGQERFVWSSLDTPSQRSFSGPWLEAQEAQLLSQPWQCQLYQGERL
LGAAVYFTELSSPGAQRSGRAPGALPAGHLLLFLILGVLSLLLLVTGAFGFHLWRRQWRPRRFSALEQGI
HPPQAQSKIEELEQEPEPEPEPEPEPEPEPEPEPEPEQL
SEQ ID NO: 45 PD-1 Amino Acid Sequence (Homo Sapiens, AAC51773.1)

MQIPQAPWPVVWAVLQLGWRPGWFLDSPDRPWNPPTFFPALLVVTEGDNATFTCSFSNTSESFVLNWYRM
SPSNQTDKLAAFPEDRSQPGQDCRFRVTQLPNGRDFHMSVVRARRNDSGTYLCGAISLAPKAQIKESLRA
ELRVTERRAEVPTAHPSPSPRPAGQFQTLVVGVVGGLLGSLVLLVWVLAVICSRAARGTIGARRTGQPLK
EDPSAVPVFSVDYGELDFQWREKTPEPPVPCVPEQTEYATIVFPSGMGTSSPARRGSADGPRSAQPLRPE
DGHCSWPL

The present invention further relates to the following embodiments:
1. A method of treating a malignant tumor in a human patient comprising administering a therapeutically effective amount of:
   a LAG-3 inhibitor;
   a PD-1 pathway inhibitor; and
   an immunotherapeutic agent.
2. The method of embodiment 1, wherein the LAG-3 inhibitor is an anti-LAG-3 antibody or an antigen binding fragment thereof.
3. The method of embodiment 1 or 2, wherein the anti-LAG-3 antibody is a bispecific antibody.
4. The method of any one of embodiments 1 to 3, wherein the anti-LAG-3 antibody or antigen binding fragment thereof comprises (a) a heavy chain variable region CDR1 comprising the sequence set forth in SEQ ID NO:7; (b) a heavy chain variable region CDR2 comprising the sequence set forth in SEQ ID NO:8; (c) a heavy chain variable region CDR3 comprising the sequence set forth in SEQ ID NO:9; (d) a light chain variable region CDR1 comprising the sequence set forth in SEQ ID NO:10; (e) a light chain variable region CDR2 comprising the sequence set forth in SEQ ID NO:11; and (f) a light chain variable region CDR3 comprising the sequence set forth in SEQ ID NO:12.
5. The method of any one of embodiments 1 to 4, wherein the anti-LAG-3 antibody or antigen binding fragment thereof comprises heavy and light chain variable regions comprising the sequences set forth in SEQ ID NOs:3 and 5, respectively.
6. The method of any one of embodiments 1 to 5, wherein the anti-LAG-3 antibody is BMS 986016, MK-4280 (28G-10), REGN3767, GSK2831781, IMP731 (H5L7BW), BAP050, IMP-701 (LAG-5250), IMP321, TSR-033, LAG525, BI 754111, or FS-118.
7. The method of embodiment 1, wherein the LAG-3 inhibitor is a soluble LAG-3 polypeptide.
8. The method of embodiment 7, wherein the soluble LAG-3 polypeptide is a fusion polypeptide.
9. The method of embodiment 7 or 8, wherein the soluble LAG-3 polypeptide comprises a ligand binding fragment of the LAG-3 extracellular domain.
10. The method of embodiment 9, wherein the ligand binding fragment of the LAG-3 extracellular domain comprises an amino acid sequence with at least 90%, at least 95%, at least 98%, or at least 99% sequence identity to SEQ ID NO:44.
11. The method of any one of embodiments 7 to 10, wherein the soluble LAG-3 polypeptide further comprises an Fc domain.
12. The method of any one of embodiments 1 to 11, wherein the PD-1 pathway inhibitor is an anti-PD-1 antibody or antigen binding fragment thereof.
13. The method of embodiment 12, wherein the anti-PD-1 antibody is pembrolizumab (KEYTRUDA; MK-3475), pidilizumab (CT-011), nivolumab (OPDIVO; BMS-936558), PDR001, MEDI0680 (AMP-514), TSR-042, REGN2810, JS001, AMP-224 (GSK-2661380), PF-06801591, BGB-A317, BI 754091, or SHR-1210.
14. The method of any one of embodiments 1 to 11, wherein the PD-1 pathway inhibitor is an anti-PD-L1 antibody or antigen binding fragment thereof.
15. The method of embodiment 14, wherein the anti-PD-L1 antibody is atezolizumab (TECENTRIQ; RG7446; MPDL3280A; RO5541267), durvalumab (MEDI4736), BMS-936559, avelumab (bavencio), LY3300054, CX-072 (Proembodiment-CX-072), FAZ053, KN035, or MDX-1105.
16. The method of any one of embodiments 1 to 11, wherein the PD-1 pathway inhibitor is a small molecule drug.
17. The method of embodiment 16, wherein the PD-1 pathway inhibitor is CA-170.
18. The method of embodiment 16, wherein the PD-1 pathway inhibitor is a cell based therapy.
19. The method of embodiment 18, wherein the cell based therapy is a MiHA-loaded PD-L1/L2-silenced dendritic cell vaccine.
20. The method of embodiment 19, wherein the cell based therapy is an anti-programmed cell death protein 1 antibody expressing pluripotent killer T lymphocyte, an autologous PD-1-targeted chimeric switch receptor-modified T lymphocyte, or a PD-1 knockout autologous T lymphocyte.
21. The method of any one of embodiments 1 to 11, wherein the PD-1 pathway inhibitor is an anti-PD-L2 antibody or antigen binding fragment thereof.
22. The method of embodiment 21, wherein the anti-PD-L2 antibody is rHIgM12B7.
23. The method of embodiment 1 to 11, wherein the PD-1 pathway inhibitor is a soluble PD-1 polypeptide.
24. The method of embodiment 23, wherein the soluble PD-1 polypeptide is a fusion polypeptide.
25. The method of embodiment 23 or 24, wherein the soluble PD-1 polypeptide comprises a ligand binding fragment of the PD-1 extracellular domain.
26. The method of any one of embodiments 23 to 25, wherein the soluble PD-1 polypeptide comprises a ligand binding fragment of the PD-1 extracellular domain.
27. The method of embodiment 26, wherein the ligand binding fragment of the PD-1 extracellular domain comprises an amino acid sequence with at least 90%, at least 95%, .at least 98%, or at least 99% sequence identity to SEQ ID NO:45.
28. The method of any one of embodiments 23 to 27, wherein the soluble PD-1 polypeptide further comprises an Fc domain.
29. The method of any one of embodiments 1 to 28, wherein the immunotherapeutic agent is a modulator of CTLA-4 activity, a modulator of CD28 activity, a modulator of CD80 activity, a modulator of CD86 activity, a modulator of 4-1BB activity, an modulator of OX40 activity, a modulator of KIR activity, a modulator of Tim-3 activity, a modulator of CD27 activity, a modulator of CD40 activity, a modulator of GITR activity, a modulator of TIGIT activity, a modulator of CD20 activity, a modulator of CD96 activity, a modulator of IDOl activity, a modulator of STING activity, a modulator of GARP activity, a modulator of A2aR activity, a modulator of CEACAM1 activity, a modulator of CEA activity, a modulator of CD47 activity, a modulator of PVRIG activity, a modulator of TDO activity, a modulator of VISTA activity, a cytokine, a chemokine, an interferon, an interleukin, a lymphokine, a member of the tumor necrosis factor (TNF) family, or an immunostimulatory oligonucleotide.
30. The method of any one of embodiments 1 to 28, wherein the immunotherapeutic agent is an immune checkpoint inhibitor.
31. The method of embodiment 30, wherein the immune checkpoint inhibitor is a CTLA-4 antagonist, a CD80 antagonist, a CD86 antagonist, a Tim-3 antagonist, a TIGIT antagonist, a CD20 antagonist, a CD96 antagonist, a IDOl antagonist, a STING antagonist, a GARP antagonist, a CD40 antagonist, A2aR antagonist, a CEACAM1 (CD66a) antagonist, a CEA antagonist, a CD47 antagonist a PVRIG antagonist, a TDO antagonist, a VISTA antagonist, or a KIR antagonist.
32. The method of embodiment 31, wherein the immune checkpoint inhibitor is a CTLA-4 antagonist.
33. The method of embodiment 32, wherein the CTLA-4 antagonist is an anti-CTLA-4 antibody or antigen binding fragment thereof.
34. The method of embodiment 33, wherein the anti-CTLA-4 antibody is ipilimumab (YERVOY), tremelimumab (ticilimumab; CP-675,206), AGEN-1884, or ATOR-1015.
35. The method of embodiment 32, wherein the CTLA-4 antagonist is a soluble CTLA-4 polypeptide.
36. The method of embodiment 35, wherein the soluble CTLA-4 polypeptide is abatacept (Orencia), belatacept (Nulojix), RG2077, or RG-1046.
37. The method of embodiment 32, wherein the CTLA-4 antagonist is a cell based therapy.
38. The method of embodiment 37, wherein the CTLA-4 antagonist is an anti-CTLA4 mAb RNA/GITRL RNA-transfected autologous dendritic cell vaccine or an anti-CTLA-4 mAb RNA-transfected autologous dendritic cell vaccine.
39. The method of embodiment 31, wherein the immune checkpoint inhibitor is a KIR antagonist
40. The method of embodiment 39, wherein the KIR antagonist is an anti-KIR antibody or antigen binding fragment thereof.
41. The method of embodiment 40, wherein the anti-KIR antibody is lirilumab (1-7F9, BMS-986015, IPH 2101) or IPH4102.
42. The method of embodiment 31, wherein the immune checkpoint inhibitor is TIGIT antagonist.
43. The method of embodiment 42, wherein the TIGIT antagonist is an anti-TIGIT antibody or antigen binding fragment thereof.
44. The method of embodiment 43, wherein the anti-TIGIT antibody is BMS-986207, AB 154, COM902 (CGEN-15137), or OMP-313M32.
45. The method of embodiment 31, wherein the immune checkpoint inhibitor is Tim-3 antagonist.
46. The method of embodiment 45, wherein the Tim-3 antagonist is an anti-Tim-3 antibody or antigen binding fragment thereof.
47. The method of embodiment 46, wherein the anti-Tim-3 antibody is TSR-022 or LY3321367.
48. The method of embodiment 31, wherein the immune checkpoint inhibitor is a IDO1 antagonist.
49. The method of embodiment 48, wherein the IDO1 antagonist is indoximod (NLG8189; 1-methyl-_{D}-TRP), epacadostat (INCB-024360, INCB-24360), KHK2455, PF-06840003, navoximod (RG6078, GDC-0919, NLG919), BMS-986205 (F001287), or pyrrolidine-2,5-dione derivatives.
50. The method of embodiment 31, wherein the immune checkpoint inhibitor is a STING antagonist.
51. The method of embodiment 50, wherein the STING antagonist is 2' or 3'-mono-fluoro substituted cyclic-di-nucleotides; 2'3'-di-fluoro substituted mixed linkage 2',5' - 3',5' cyclic-di-nucleotides; 2'-fluoro substituted, bis-3',5' cyclic-di-nucleotides; 2',2"-diF-Rp,Rp,bis-3',5' cyclic-di-nucleotides; or fluorinated cyclic-di-nucleotides.
52. The method of embodiment 31, wherein the immune checkpoint inhibitor is CD20 antagonist.
53. The method of embodiment 52, wherein the CD20 antagonist is an anti-CD20 antibody or antigen binding fragment thereof.
54. The method of embodiment 53, wherein the anti-CD20 antibody is rituximab (RITUXAN; IDEC-102; IDEC-C2B8), ABP 798, ofatumumab, or obinutuzumab.
55. The method of embodiment 31, wherein the immune checkpoint inhibitor is CD80 antagonist.
56. The method of embodiment 55, wherein the CD80 antagonist is an anti-CD80 antibody or antigen binding fragment thereof.
57. The method of embodiment 56, wherein the anti-CD80 antibody is galiximab or AV 1142742.
58. The method of embodiment 31, wherein the immune checkpoint inhibitor is a GARP antagonist.
59. The method of embodiment 58, wherein the GARP antagonist is an anti-GARP antibody or antigen binding fragment thereof.
60. The method of embodiment 59, wherein the anti-GARP antibody is ARGX-115.
61. The method of embodiment 31, wherein the immune checkpoint inhibitor is a CD40 antagonist.
62. The method of embodiment 61, wherein the CD40 antagonist is an anti-CD40 antibody for antigen binding fragment thereof.
63. The method of embodiment 62, wherein the anti-CD40 antibody is BMS3h-56, lucatumumab (HCD122 and CHIR-12.12), CHIR-5.9, or dacetuzumab (huS2C6, PRO 64553, RG 3636, SGN 14, SGN-40).
64. The method of embodiment 61, wherein the CD40 antagonist is a soluble CD40 ligand (CD40-L).
65. The method of embodiment 64, wherein the soluble CD40 ligand is a fusion polypeptide.
66. The method of embodiment 64 or 65, wherein the soluble CD40 ligand is a CD40-L/FC2 or a monomeric CD40-L.
67. The method of embodiment 31, wherein the immune checkpoint inhibitor is an A2aR antagonist.
68. The method of embodiment 67, wherein the A2aR antagonist is a small molecule.
69. The method of embodiment 67 or 68, wherein the A2aR antagonist is CPI-444, PBF-509, istradefylline (KW-6002), preladenant (SCH420814), tozadenant (SYN115), vipadenant (BIIB014), HTL-1071, ST1535, SCH412348, SCH442416, SCH58261, ZM241385, or AZD4635.
70. The method of embodiment 31, wherein the immune checkpoint inhibitor is a CEACAM1 antagonist.
71. The method of embodiment 70, wherein the CEACAM1 antagonist is an anti-CEACAM1 antibody or antigen binding fragment thereof.
72. The method of embodiment 71, wherein the anti-CEACAM1 antibody is CM-24 (MK-6018).
73. The method of embodiment 31, wherein the immune checkpoint inhibitor is a CEA antagonist.
74. The method of embodiment 73, wherein the CEA antagonist is an anti-CEA antibody or antigen binding fragment thereof.
75. The method of embodiment 74, wherein the anti-CEA antibody is cergutuzumab amunaleukin (RG7813, RO-6895882) or RG7802 (RO6958688).
76. The method of embodiment 31, wherein the immune checkpoint inhibitor is a CD47 antagonist.
77. The method of embodiment 76, wherein the CD47 antagonist is an anti-CD47 antibody or antigen binding fragment thereof.
78. The method of embodiment 77, wherein the anti-CD47 antibody is HuF9-G4, CC-90002, TTI-621, ALX148, NI-1701, NI-1801, SRF231, or Effi-DEM.
79. The method of embodiment 31, wherein the immune checkpoint inhibitor is a PVRIG antagonist.
80. The method of embodiment 79, wherein the PVRIG antagonist is an anti-PVRIG antibody or antigen binding fragment thereof.
81. The method of embodiment 80, wherein the anti-PVRIG antibody is COM701 (CGEN-15029).
82. The method of embodiment 31, wherein the immune checkpoint inhibitor is a TDO antagonist.
83. The method of embodiment 82, wherein the TDO antagonist is a 4-(indol-3-yl)-pyrazole derivative, a 3-indol substituted derivative, or a 3-(indol-3-yl)-pyridine derivative.
84. The method of embodiment 31, wherein the immune checkpoint inhibitor is a dual IDO and TDO antagonist.
85. The method of embodiment x84 wherein the dual IDO and TDO antagonist is a small molecule.
86. The method of embodiment 31, wherein the immune checkpoint inhibitor is a VISTA antagonist.
87. The method of embodiment 86, wherein the VISTA antagonist is CA-170 or JNJ-61610588.
88. The method of any one of embodiments 1-29, wherein the immunotherapeutic agent is an immune checkpoint enhancer or stimulator.
89. The method of embodiment 88, wherein the immune checkpoint enhancer or stimulator is a CD28 agonist, a 4-1BB agonist, an OX40 agonist, a CD27 agonist, a CD80 agonist, a CD86 agonist, a CD40 agonist, an ICOS agonist, a CD70 agonist, or a GITR agonist.
90. The method of embodiment 89, wherein the immune checkpoint enhancer or stimulator is an OX40 agonist.
91. The method of embodiment 90, wherein the OX40 agonist is an anti-OX40 antibody or antigen binding fragment thereof.
92. The method of embodiment 91, wherein the anti-OX40 antibody is tavolixizumab (MEDI-0562), pogalizumab (MOXR0916, RG7888), GSK3174998, ATOR-1015, MEDI-6383, MEDI-6469, BMS 986178, PF-04518600, or RG7888 (MOXR0916)..
93. The method of embodiment 90, wherein the OX40 agonist is a cell based therapy.
94. The method of embodiment 93, wherein the OX40 agonist is a GINAKIT cell.
95. The method of embodiment 89, wherein the immune checkpoint enhancer or stimulator is a CD40 agonist.
96. The method of embodiment 95 wherein the CD40 agonist is an anti-CD40 antibody or antigen binding fragment thereof.
97. The method of embodiment 96, wherein the anti-CD40 antibody is ADC-1013 (JNJ-64457107), RG7876 (RO-7009789), HuCD40-M2, APX005M (EPI-0050), or Chi Lob 7/4.
98. The method of embodiment 95, wherein the CD40 agonist is a soluble CD40 ligand (CD40-L).
99. The method of embodiment 98, wherein the soluble CD40 ligand is a fusion polypeptide.
100. The method of embodiment 98 or 99, wherein the soluble CD40 ligand is a trimeric CD40-L (AVREND).
101. The method of embodiment 89, wherein the immune checkpoint enhancer or stimulator is a GITR agonist.
102. The method of embodiment 101, wherein the GITR agonist is an anti-GITR antibody or antigen binding fragment thereof.
103. The method of embodiment 102, wherein the anti-GITR antibody is BMS-986156, TRX518, GWN323, INCAGN01876, or MEDI1873.
104. The method of embodiment 101, wherein the GITR agonist is a soluble GITR ligand (GITRL).
105. The method of embodiment 104, wherein the soluble GITR ligand is a fusion polypeptide.
106. The method of embodiment 101, wherein the GITR agonist is a cell based therapy.
107. The method of embodiment 106, wherein the cell based therapy is an anti-CTLA4 mAb RNA/GITRL RNA-transfected autologous dendritic cell vaccine or a GITRL RNA-transfected autologous dendritic cell vaccine.
108. The method of embodiment 89, wherein the immune checkpoint enhancer or stimulator a 4-1BB agonist.
109. The method of embodiment 108, wherein the 4-1BB agonist is an anti-4-1BB antibody or antigen binding fragment thereof.
110. The method of embodiment 109, wherein the anti-4-1BB antibody is urelumab or PF-05082566.
111. The method of embodiment 89, wherein the immune checkpoint enhancer or stimulator is a CD80 agonist or a CD86 agonist.
112. The method of embodiment 111, wherein the CD80 agonist or the CD86 agonist is a soluble CD80 or CD86 ligand (CTLA-4).
113. The method of embodiment 112, wherein the soluble CD80 or CD86 ligand is a fusion polypeptide.
114. The method of embodiment 112 or 113, wherein the CD80 or CD86 ligand is CTLA4-Ig (CTLA4-IgG4m, RG2077, or RG1046) or abatacept (ORENCIA, BMS-188667).
115. The method of embodiment 111, wherein the CD80 agonist or the CD86 agonist is a cell based therapy.
116. The method of embodiment 115, wherein the cell based therapy is MGN1601.
117. The method of embodiment 89, wherein the immune checkpoint enhancer or stimulator is a CD28 agonist.
118. The method of embodiment 117, wherein the CD28 agonist is an anti-CD28 antibody or antigen binding fragment thereof.
119. The method of embodiment 118, wherein the anti-CD28 antibody is TGN1412.
120. The method of embodiment 117, wherein the CD28 agonist is a cell based therapy.
121. The method of embodiment 120, wherein the cell based therapy is JCAR015 (anti-CD19-CD28-zeta modified CAR CD3+ T lymphocyte); CD28CAR/CD137CAR-expressing T lymphocyte; allogeneic CD4+ memory Th1-like T cells/microparticle-bound anti-CD3/anti-CD28; anti-CD19/CD28/CD3zeta CAR gammaretroviral vector-transduced autologous T lymphocytes KTE-C19; anti-CEA IgCD28TCR-transduced autologous T lymphocytes; anti-EGFRvIII CAR-transduced allogeneic T lymphocytes; autologous CD123CAR-CD28-CD3zeta-EGFRt-expressing T lymphocytes; autologous CD171-specific CAR-CD28 zeta-4-1-BB-EGFRt-expressing T lymphocytes; autologous CD19CAR-CD28-CD3zeta-EGFRt-expressing Tcm-enriched T cells; autologous PD-1-targeted chimeric switch receptor-modified T lymphocytes (chimera with CD28); CD19CAR-CD28-CD3zeta-EGFRt-expressing Tcm-enriched T lymphocytes; CD19CAR-CD28-CD3zeta-EGFRt-expressing Tn/mem-enriched T lymphocytes; CD19CAR-CD28zeta-4-1BB-expressing allogeneic T lymphocytes; CD19CAR-CD3zeta-4-1BB-CD28-expressing autologous T lymphocytes; CD28CAR/CD137CAR-expressing T lymphocytes; CD3/CD28 costimulated vaccine-primed autologous T lymphocytes; or iC9-GD2-CD28-OX40-expressing T lymphocytes.
122. The method of embodiment 89, wherein the immune checkpoint enhancer or stimulator is a CD27 agonist.
123. The method of embodiment 122, wherein the CD27 agonist is an anti-CD27 antibody or antigen binding fragment thereof.
124. The method of embodiment 123, wherein the anti-CD27 antibody is varlilumab (CDX-1127).
125. The method of embodiment 89, wherein the immune checkpoint enhancer or stimulator is a CD70 agonist.
126. The method of embodiment 125, wherein the CD70 agonist is an anti-CD70 antibody or antigen binding fragment thereof.
127. The method of embodiment 126, wherein the anti-CD70 antibody is ARGX-110.
128. The method of embodiment 89, wherein the immune checkpoint enhancer or stimulator is an ICOS agonist.
129. The method of embodiment 128, wherein the ICOS agonist is an anti-ICOS antibody or antigen binding fragment thereof.
130. The method of embodiment 129, wherein the anti-ICOS antibody is BMS986226, MEDI-570, GSK3359609, or JTX-2011.
131. The method of embodiment 128, wherein the ICOS agonist is a soluble ICOS ligand.
132. The method of embodiment 131, wherein the soluble ICOS ligand is a fusion polypeptide.
133. The method of embodiment 131 or 132, wherein the soluble ICOS ligand is AMG 750.
134. The method of embodiment 89, wherein the immunotherapeutic agent is an anti-CD73 antibody or antigen binding fragment thereof.
135. The method of embodiment 134, wherein the anti-CD73 antibody is MEDI9447.
136. The method of any one of embodiments 1-28, wherein the immunotherapeutic agent is a TLR9 agonist.
137. The method of embodiment 136, wherein the TLR9 agonist is agatolimod sodium.
138. The method of any one of embodiments 1-28, wherein the immunotherapeutic agent is a cytokine.
139. The method of embodiment 138, wherein the cytokine is a chemokine, an interferon, an interleukin, lymphokine, or a member of the tumor necrosis factor family.
140. The method of embodiment 138 or 139, wherein the cytokine is IL-2, IL-15, or interferon-gamma.
141. The method of any one of embodiments 1-28, wherein the immunotherapeutic agent is a TGF-β antagonist.
142. The method of embodiment 141, wherein the TGF-β antagonist is fresolimumab (GC-1008), NIS793, IMC-TR1 (LY3022859), ISTH0036, trabedersen (AP 12009), recombinant transforming growth factor-beta-2, autologous HPV-16/18 E6/E7-specific TGF-beta-resistant T lymphocytes, or TGF-beta-resistant LMP-specific cytotoxic T-lymphocytes.
143. The method of any one of embodiments 1-28, wherein the immunotherapeutic agent is an iNOS antagonist.
144. The method of embodiment 143, wherein the iNOS antagonist is N-Acetyle-cysteine (NAC), aminoguanidine, L-nitroarginine methyl ester, or S,S-1,4-phenylene-bis(1,2-ethanediyl)bis-isothiourea).
145. The method of any one of embodiments 1-28, wherein the immunotherapeutic agent is a SHP-1 antagonist.
146. The method of any one of embodiments 1-28, wherein the immunotherapeutic agent is a CSF1R (colony stimulating factor 1 receptor) antagonist.
147. The method of embodiment 146, wherein the CSF1R antagonist is an anti-CSF1R antibody or antigen binding fragment thereof.
148. The method of embodiment 147, wherein the anti-CSF1R antibody is emactuzumab.
149. The method of any one of embodiments 1-28, wherein the immunotherapeutic agent is an agonist of a TNF family member.
150. The method of embodiment 149, wherein the agonist of the TNF family member is ATOR 1016, ABBV-621, or Adalimumab.
151. The method of any one of embodiments 1-28, wherein the immunotherapeutic agent is aldesleukin, tocilizumab, or MEDI5083.
152. The method of any one of embodiments 1-28, wherein the immunotherapeutic agent is a CD160 (NK1) agonist.
153. The method of embodiment 152, wherein the CD160 (NK1) agonist is an anti-CD160 antibody or antigen binding fragment thereof.
154. The method of embodiment 152 or 153, wherein the anti-CD160 antibody is BY55.
155. The method of any one of embodiments 1-154, wherein the LAG-3 inhibitor, PD-1 pathway inhibitor, and the immunotherapeutic agent are formulated for intravenous administration.
156. The method of any one of embodiments 1-155, wherein the LAG-3 inhibitor, PD-1 pathway inhibitor, and the immunotherapeutic agent are formulated together.
157. The method of any one of embodiments 1-155, wherein the LAG-3 inhibitor, PD-1 pathway inhibitor, and the immunotherapeutic agent are formulated separately.
158. The method of any one of embodiments 1-157, wherein the malignant tumor is selected from the group consisting of a liver cancer, bone cancer, pancreatic cancer, skin cancer, cancer of the head or neck, breast cancer, lung cancer, cutaneous or intraocular malignant melanoma, renal cancer, uterine cancer, ovarian cancer, colorectal cancer, colon cancer, rectal cancer, cancer of the anal region, stomach cancer, testicular cancer, uterine cancer, carcinoma of the fallopian tubes, carcinoma of the endometrium, carcinoma of the cervix, carcinoma of the vagina, carcinoma of the vulva, non-Hodgkin's lymphoma, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, cancers of the childhood, lymphocytic lymphoma, cancer of the bladder, cancer of the kidney or ureter, carcinoma of the renal pelvis, neoplasm of the central nervous system (CNS), primary CNS lymphoma, tumor angiogenesis, spinal axis tumor, brain stem glioma, pituitary adenoma, Kaposi's sarcoma, epidermoid cancer, squamous cell cancer, environmentally induced cancers including those induced by asbestos, hematologic malignancies including, for example, multiple myeloma, B-cell lymphoma, Hodgkin lymphoma/primary mediastinal B-cell lymphoma, non-Hodgkin's lymphomas, acute myeloid lymphoma, chronic myelogenous leukemia, chronic lymphoid leukemia, follicular lymphoma, diffuse large B-cell lymphoma, Burkitt's lymphoma, immunoblastic large cell lymphoma, precursor B-lymphoblastic lymphoma, mantle cell lymphoma, acute lymphoblastic leukemia, mycosis fungoides, anaplastic large cell lymphoma, T-cell lymphoma, and precursor T-lymphoblastic lymphoma, and any combination thereof.
159. The method of embodiment 158, wherein the malignant tumor is non-small cell lung cancer (NSCLC), a virally-related cancer related tumor, or gastric adenocarcinoma.
160. The method of any one of embodiments 1-157, wherein the malignant tumor is melanoma, gastric cancer, gastroesophageal junction cancer, non-small cell lung cancer, bladder cancer, head and neck squamous cell carcinoma, or renal cell cancer.
161. The method of any one of embodiments 1-157, wherein the tumor is lung cancer, melanoma, squamous cell carcinoma of the head and neck, renal cancer, gastric cancer, or hepatocellular carcinoma.
162. The method of any one of embodiments 1-161, wherein the anti-LAG-3 antibody or antigen binding fragment thereof and the immunotherapeutic agent are administered as a first line of treatment.
163. The method of any one of embodiments 1-161, wherein the LAG-3 inhibitor, PD-1 pathway inhibitor, and the immunotherapeutic agent are administered as a second line of treatment.
164. The method of any one of embodiments 1-163, wherein the malignant tumor is refractory to first line treatment.
165. The method of any one of embodiments 1-164, further comprising the administration of at least one additional therapeutic agent.
166. The method of embodiment 165, wherein the at least one additional therapeutic agent is a chemotherapeutic agent.

## Claims

1. An anti-LAG-3 antibody or an antigen binding fragment thereof for use in treating a malignant tumor in a human patient in combination with an anti-PD-1 antibody or an antigen binding fragment thereof and an anti-CTLA-4 antibody or an antigen binding fragment thereof.

2. An anti-LAG-3 antibody or an antigen binding fragment thereof for use in treating a malignant tumor in a human patient in combination with an anti-PD-L1 antibody or an antigen binding fragment thereof and an anti-CTLA-4 antibody or an antigen binding fragment thereof.

3. An anti-LAG-3 antibody or an antigen binding fragment thereof for use in treating a malignant tumor in a human patient in combination with a small molecule drug that is a PD-1 pathway inhibitor and an anti-CTLA-4 antibody or an antigen binding fragment thereof.

4. An anti-LAG-3 antibody or an antigen binding fragment thereof for use in treating a malignant tumor in a human patient in combination with a small molecule drug that is a PD-1 pathway inhibitor and a soluble CTLA-4 polypeptide that is a CTLA-4 antagonist.

5. An anti-LAG-3 antibody or an antigen binding fragment thereof for use in treating a malignant tumor in a human patient in combination with an anti-PD-1 antibody or an antigen binding fragment thereof and a soluble CTLA-4 polypeptide that is a CTLA-4 antagonist.

6. An anti-LAG-3 antibody or an antigen binding fragment thereof for use in treating a malignant tumor in a human patient in combination with an anti-PD-L1 antibody or an antigen binding fragment thereof and a soluble CTLA-4 polypeptide that is a CTLA-4 antagonist.


SEQUENCES

<110> BRISTOL-MYERS SQUIBB COMPANY
      KORMAN, ALAN J.
      LONGBERG, NILS
      SELBY, MARK J.
      JACKSON, JEFFREY

<120> COMPOSITIONS COMPRISING A COMBINATION OF AN ANTI-LAG-3 ANTIBODY,
      A PD-1 PATHWAY INHIBITOR, AND AN IMMUNOTHERAPEUTIC AGENT

<130> 3338.069PC02/ELE/PAC/E-H

<150> 62/513,812
<151> 2017-06-01

<150> 62/512,618
<151> 2017-05-30

<160> 45

<170> PatentIn version 3.5

<210> 1
<211> 447
<212> PRT
<213> Artificial Sequence

<220>
<223> Heavy Chain Amino Acid Sequence; Anti-LAG-3 mAb (BMS-986016)

<400> 1

Gln Val Gln Leu Gln Gln Trp Gly Ala Gly Leu Leu Lys Pro Ser Glu
1               5                   10                  15

Thr Leu Ser Leu Thr Cys Ala Val Tyr Gly Gly Ser Phe Ser Asp Tyr
            20                  25                  30

Tyr Trp Asn Trp Ile Arg Gln Pro Pro Gly Lys Gly Leu Glu Trp Ile
        35                  40                  45

Gly Glu Ile Asn His Arg Gly Ser Thr Asn Ser Asn Pro Ser Leu Lys
    50                  55                  60

Ser Arg Val Thr Leu Ser Leu Asp Thr Ser Lys Asn Gln Phe Ser Leu
65                  70                  75                  80

Lys Leu Arg Ser Val Thr Ala Ala Asp Thr Ala Val Tyr Tyr Cys Ala
                85                  90                  95

Phe Gly Tyr Ser Asp Tyr Glu Tyr Asn Trp Phe Asp Pro Trp Gly Gln
            100                 105                 110

Gly Thr Leu Val Thr Val Ser Ser Ala Ser Thr Lys Gly Pro Ser Val
        115                 120                 125

Phe Pro Leu Ala Pro Cys Ser Arg Ser Thr Ser Glu Ser Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Lys Thr Tyr Thr Cys Asn Val Asp His Lys
        195                 200                 205

Pro Ser Asn Thr Lys Val Asp Lys Arg Val Glu Ser Lys Tyr Gly Pro
    210                 215                 220

Pro Cys Pro Pro Cys Pro Ala Pro Glu Phe Leu Gly Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Asp Val Ser Gln Glu Asp Pro Glu
            260                 265                 270

Val Gln Phe Asn Trp Tyr Val Asp Gly Val Glu Val His Asn Ala Lys
        275                 280                 285

Thr Lys Pro Arg Glu Glu Gln Phe Asn Ser Thr Tyr Arg Val Val Ser
    290                 295                 300

Val Leu Thr Val Leu His Gln Asp Trp Leu Asn Gly Lys Glu Tyr Lys
305                 310                 315                 320

Cys Lys Val Ser Asn Lys Gly Leu Pro Ser Ser Ile Glu Lys Thr Ile
                325                 330                 335

Ser Lys Ala Lys Gly Gln Pro Arg Glu Pro Gln Val Tyr Thr Leu Pro
            340                 345                 350

Pro Ser Gln Glu Glu Met Thr Lys Asn Gln Val Ser Leu Thr Cys Leu
        355                 360                 365

Val Lys Gly Phe Tyr Pro Ser Asp Ile Ala Val Glu Trp Glu Ser Asn
    370                 375                 380

Gly Gln Pro Glu Asn Asn Tyr Lys Thr Thr Pro Pro Val Leu Asp Ser

385                 390                 395                 400

Asp Gly Ser Phe Phe Leu Tyr Ser Arg Leu Thr Val Asp Lys Ser Arg
                405                 410                 415

Trp Gln Glu Gly Asn Val Phe Ser Cys Ser Val Met His Glu Ala Leu
            420                 425                 430

His Asn His Tyr Thr Gln Lys Ser Leu Ser Leu Ser Leu Gly Lys
        435                 440                 445

<210> 2
<211> 214
<212> PRT
<213> Artificial Sequence

<220>
<223> Light Chain Amino Acid Sequence; Anti-LAG-3 mAb (BMS-986016)

<400> 2

Glu Ile Val Leu Thr Gln Ser Pro Ala Thr Leu Ser Leu Ser Pro Gly
1               5                   10                  15

Glu Arg Ala Thr Leu Ser Cys Arg Ala Ser Gln Ser Ile Ser Ser Tyr
            20                  25                  30

Leu Ala Trp Tyr Gln Gln Lys Pro Gly Gln Ala Pro Arg Leu Leu Ile
        35                  40                  45

Tyr Asp Ala Ser Asn Arg Ala Thr Gly Ile Pro Ala Arg Phe Ser Gly
    50                  55                  60

Ser Gly Ser Gly Thr Asp Phe Thr Leu Thr Ile Ser Ser Leu Glu Pro
65                  70                  75                  80

Glu Asp Phe Ala Val Tyr Tyr Cys Gln Gln Arg Ser Asn Trp Pro Leu
                85                  90                  95

Thr Phe Gly Gln Gly Thr Asn Leu Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser

165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210> 3
<211> 120
<212> PRT
<213> Artificial Sequence

<220>
<223> Heavy Chain Variable Region (VH) Amino Acid Sequence; Anti-LAG-3
      mAb (BMS-986016)

<400> 3

Gln Val Gln Leu Gln Gln Trp Gly Ala Gly Leu Leu Lys Pro Ser Glu
1               5                   10                  15

Thr Leu Ser Leu Thr Cys Ala Val Tyr Gly Gly Ser Phe Ser Asp Tyr
            20                  25                  30

Tyr Trp Asn Trp Ile Arg Gln Pro Pro Gly Lys Gly Leu Glu Trp Ile
        35                  40                  45

Gly Glu Ile Asn His Arg Gly Ser Thr Asn Ser Asn Pro Ser Leu Lys
    50                  55                  60

Ser Arg Val Thr Leu Ser Leu Asp Thr Ser Lys Asn Gln Phe Ser Leu
65                  70                  75                  80

Lys Leu Arg Ser Val Thr Ala Ala Asp Thr Ala Val Tyr Tyr Cys Ala
                85                  90                  95

Phe Gly Tyr Ser Asp Tyr Glu Tyr Asn Trp Phe Asp Pro Trp Gly Gln
            100                 105                 110

Gly Thr Leu Val Thr Val Ser Ser
        115                 120

<210> 4
<211> 360
<212> DNA
<213> Artificial Sequence

<220>
<223> Heavy Chain Variable Region (VH) Nucleotide Sequence; Anti-LAG-3
      mAb (BMS-986016)

<400> 4
caggtgcagc tacagcagtg gggcgcagga ctgttgaagc cttcggagac cctgtccctc      60

acctgcgctg tctatggtgg gtccttcagt gattactact ggaactggat ccgccagccc     120

ccagggaagg ggctggagtg gattggggaa atcaatcatc gtggaagcac caactccaac     180

ccgtccctca agagtcgagt caccctatca ctagacacgt ccaagaacca gttctccctg     240

aagctgaggt ctgtgaccgc cgcggacacg gctgtgtatt actgtgcgtt tggatatagt     300

gactacgagt acaactggtt cgacccctgg ggccagggaa ccctggtcac cgtctcctca     360


<210> 5
<211> 107
<212> PRT
<213> Artificial Sequence

<220>
<223> Light Chain Variable Region (VL) Amino Acid Sequence; Anti-LAG-3
      mAb (BMS-986016)

<400> 5

Glu Ile Val Leu Thr Gln Ser Pro Ala Thr Leu Ser Leu Ser Pro Gly
1               5                   10                  15


Glu Arg Ala Thr Leu Ser Cys Arg Ala Ser Gln Ser Ile Ser Ser Tyr
            20                  25                  30


Leu Ala Trp Tyr Gln Gln Lys Pro Gly Gln Ala Pro Arg Leu Leu Ile
        35                  40                  45


Tyr Asp Ala Ser Asn Arg Ala Thr Gly Ile Pro Ala Arg Phe Ser Gly
    50                  55                  60


Ser Gly Ser Gly Thr Asp Phe Thr Leu Thr Ile Ser Ser Leu Glu Pro
65                  70                  75                  80


Glu Asp Phe Ala Val Tyr Tyr Cys Gln Gln Arg Ser Asn Trp Pro Leu
                85                  90                  95


Thr Phe Gly Gln Gly Thr Asn Leu Glu Ile Lys
            100                 105


<210> 6
<211> 321
<212> DNA
<213> Artificial Sequence

<220>
<223> Light Chain Variable Region (VL) Nucleotide Sequence; Anti-LAG-3
      mAb (BMS-986016)

<400> 6
gaaattgtgt tgacacagtc tccagccacc ctgtctttgt ctccagggga aagagccacc      60

ctctcctgca gggccagtca gagtattagc agctacttag cctggtacca acagaaacct      120

ggccaggctc ccaggctcct catctatgat gcatccaaca gggccactgg catcccagcc      180

aggttcagtg gcagtgggtc tgggacagac ttcactctca ccatcagcag cctagagcct      240

gaagattttg cagtttatta ctgtcagcag cgtagcaact ggcctctcac ttttggccag      300

gggaccaacc tggagatcaa a                                                321

<210> 7
<211> 5
<212> PRT
<213> Artificial Sequence

<220>
<223> Heavy Chain CDR1 Amino Acid Sequence; Anti-LAG-3 mAb (BMS-986016)

<400> 7

Asp Tyr Tyr Trp Asn
1               5

<210> 8
<211> 16
<212> PRT
<213> Artificial Sequence

<220>
<223> Heavy Chain CDR2 Amino Acid Sequence; Anti-LAG-3 mAb (BMS-986016)

<400> 8

Glu Ile Asn His Arg Gly Ser Thr Asn Ser Asn Pro Ser Leu Lys Ser
1               5                   10                  15

<210> 9
<211> 12
<212> PRT
<213> Artificial Sequence

<220>
<223> Heavy Chain CDR3 Amino Acid Sequence; Anti-LAG-3 mAb (BMS-986016)

<400> 9

Gly Tyr Ser Asp Tyr Glu Tyr Asn Trp Phe Asp Pro
1               5                   10

<210> 10
<211> 11
<212> PRT
<213> Artificial Sequence

<220>
<223> Light Chain CDR1 Amino Acid Sequence; Anti-LAG-3 mAb (BMS-986016)

<400> 10

Arg Ala Ser Gln Ser Ile Ser Ser Tyr Leu Ala
1               5                   10

<210> 11
<211> 7
<212> PRT
<213> Artificial Sequence

<220>
<223> Light Chain CDR2 Amino Acid Sequence; Anti-LAG-3 mAb (BMS-986016)

<400> 11

Asp Ala Ser Asn Arg Ala Thr
1               5


<210> 12
<211> 9
<212> PRT
<213> Artificial Sequence

<220>
<223> Light Chain CDR3 Amino Acid Sequence; Anti-LAG-3 mAb (BMS-986016)

<400> 12

Gln Gln Arg Ser Asn Trp Pro Leu Thr
1               5


<210> 13
<211> 525
<212> PRT
<213> Artificial Sequence

<220>
<223> Human LAG-3 Amino Acid Sequence

<400> 13

Met Trp Glu Ala Gln Phe Leu Gly Leu Leu Phe Leu Gln Pro Leu Trp
1               5                   10                  15

Val Ala Pro Val Lys Pro Leu Gln Pro Gly Ala Glu Val Pro Val Val
            20                  25                  30

Trp Ala Gln Glu Gly Ala Pro Ala Gln Leu Pro Cys Ser Pro Thr Ile
        35                  40                  45

Pro Leu Gln Asp Leu Ser Leu Leu Arg Arg Ala Gly Val Thr Trp Gln
    50                  55                  60

His Gln Pro Asp Ser Gly Pro Pro Ala Ala Ala Pro Gly His Pro Leu
65                  70                  75                  80

Ala Pro Gly Pro His Pro Ala Ala Pro Ser Ser Trp Gly Pro Arg Pro
                85                  90                  95

Arg Arg Tyr Thr Val Leu Ser Val Gly Pro Gly Gly Leu Arg Ser Gly
            100                 105                 110

Arg Leu Pro Leu Gln Pro Arg Val Gln Leu Asp Glu Arg Gly Arg Gln
        115                 120                 125

Arg Gly Asp Phe Ser Leu Trp Leu Arg Pro Ala Arg Arg Ala Asp Ala
    130                 135                 140

Gly Glu Tyr Arg Ala Ala Val His Leu Arg Asp Arg Ala Leu Ser Cys
145                 150                 155                 160

Arg Leu Arg Leu Arg Leu Gly Gln Ala Ser Met Thr Ala Ser Pro Pro
                165                 170                 175

Gly Ser Leu Arg Ala Ser Asp Trp Val Ile Leu Asn Cys Ser Phe Ser
            180                 185                 190

Arg Pro Asp Arg Pro Ala Ser Val His Trp Phe Arg Asn Arg Gly Gln
        195                 200                 205

Gly Arg Val Pro Val Arg Glu Ser Pro His His His Leu Ala Glu Ser
    210                 215                 220

Phe Leu Phe Leu Pro Gln Val Ser Pro Met Asp Ser Gly Pro Trp Gly
225                 230                 235                 240

Cys Ile Leu Thr Tyr Arg Asp Gly Phe Asn Val Ser Ile Met Tyr Asn
                245                 250                 255

Leu Thr Val Leu Gly Leu Glu Pro Pro Thr Pro Leu Thr Val Tyr Ala
            260                 265                 270

Gly Ala Gly Ser Arg Val Gly Leu Pro Cys Arg Leu Pro Ala Gly Val
        275                 280                 285

Gly Thr Arg Ser Phe Leu Thr Ala Lys Trp Thr Pro Pro Gly Gly Gly
    290                 295                 300

Pro Asp Leu Leu Val Thr Gly Asp Asn Gly Asp Phe Thr Leu Arg Leu
305                 310                 315                 320

Glu Asp Val Ser Gln Ala Gln Ala Gly Thr Tyr Thr Cys His Ile His
                325                 330                 335

Leu Gln Glu Gln Gln Leu Asn Ala Thr Val Thr Leu Ala Ile Ile Thr
            340                 345                 350

Val Thr Pro Lys Ser Phe Gly Ser Pro Gly Ser Leu Gly Lys Leu Leu
        355                 360                 365

Cys Glu Val Thr Pro Val Ser Gly Gln Glu Arg Phe Val Trp Ser Ser

370                 375                 380

Leu Asp Thr Pro Ser Gln Arg Ser Phe Ser Gly Pro Trp Leu Glu Ala
385                 390                 395                 400

Gln Glu Ala Gln Leu Leu Ser Gln Pro Trp Gln Cys Gln Leu Tyr Gln
                405                 410                 415

Gly Glu Arg Leu Leu Gly Ala Ala Val Tyr Phe Thr Glu Leu Ser Ser
            420                 425                 430

Pro Gly Ala Gln Arg Ser Gly Arg Ala Pro Gly Ala Leu Pro Ala Gly
        435                 440                 445

His Leu Leu Leu Phe Leu Thr Leu Gly Val Leu Ser Leu Leu Leu Leu
    450                 455                 460

Val Thr Gly Ala Phe Gly Phe His Leu Trp Arg Arg Gln Trp Arg Pro
465                 470                 475                 480

Arg Arg Phe Ser Ala Leu Glu Gln Gly Ile His Pro Pro Gln Ala Gln
                485                 490                 495

Ser Lys Ile Glu Glu Leu Glu Gln Glu Pro Glu Pro Glu Pro Glu Pro
            500                 505                 510

Glu Pro Glu Pro Glu Pro Glu Pro Glu Pro Glu Gln Leu
        515                 520                 525

<210> 14
<211> 8
<212> PRT
<213> Artificial Sequence

<220>
<223> LAG-3 Epitope

<400> 14

Pro Gly His Pro Leu Ala Pro Gly
1               5

<210> 15
<211> 8
<212> PRT
<213> Artificial Sequence

<220>
<223> LAG-3 Epitope

<400> 15

His Pro Ala Ala Pro Ser Ser Trp
1               5

<210> 16
<211> 8
<212> PRT
<213> Artificial Sequence

<220>
<223> LAG-3 Epitope

<400> 16

Pro Ala Ala Pro Ser Ser Trp Gly
1               5


<210> 17
<211> 440
<212> PRT
<213> Artificial Sequence

<220>
<223> Heavy Chain Amino Acid Sequence; Anti-PD-1 mAb (BMS936558)

<400> 17

Gln Val Gln Leu Val Glu Ser Gly Gly Gly Val Val Gln Pro Gly Arg
1               5                   10                  15

Ser Leu Arg Leu Asp Cys Lys Ala Ser Gly Ile Thr Phe Ser Asn Ser
            20                  25                  30

Gly Met His Trp Val Arg Gln Ala Pro Gly Lys Gly Leu Glu Trp Val
        35                  40                  45

Ala Val Ile Trp Tyr Asp Gly Ser Lys Arg Tyr Tyr Ala Asp Ser Val
    50                  55                  60

Lys Gly Arg Phe Thr Ile Ser Arg Asp Asn Ser Lys Asn Thr Leu Phe
65                  70                  75                  80

Leu Gln Met Asn Ser Leu Arg Ala Glu Asp Thr Ala Val Tyr Tyr Cys
                85                  90                  95

Ala Thr Asn Asp Asp Tyr Trp Gly Gln Gly Thr Leu Val Thr Val Ser
            100                 105                 110

Ser Ala Ser Thr Lys Gly Pro Ser Val Phe Pro Leu Ala Pro Cys Ser
        115                 120                 125

Arg Ser Thr Ser Glu Ser Thr Ala Ala Leu Gly Cys Leu Val Lys Asp
    130                 135                 140

Tyr Phe Pro Glu Pro Val Thr Val Ser Trp Asn Ser Gly Ala Leu Thr
145                 150                 155                 160

Ser Gly Val His Thr Phe Pro Ala Val Leu Gln Ser Ser Gly Leu Tyr

                165                 170                 175

Ser Leu Ser Ser Val Val Thr Val Pro Ser Ser Ser Leu Gly Thr Lys
            180                 185                 190

Thr Tyr Thr Cys Asn Val Asp His Lys Pro Ser Asn Thr Lys Val Asp
        195                 200                 205

Lys Arg Val Glu Ser Lys Tyr Gly Pro Pro Cys Pro Pro Cys Pro Ala
    210                 215                 220

Pro Glu Phe Leu Gly Gly Pro Ser Val Phe Leu Phe Pro Pro Lys Pro
225                 230                 235                 240

Lys Asp Thr Leu Met Ile Ser Arg Thr Pro Glu Val Thr Cys Val Val
                245                 250                 255

Val Asp Val Ser Gln Glu Asp Pro Glu Val Gln Phe Asn Trp Tyr Val
            260                 265                 270

Asp Gly Val Glu Val His Asn Ala Lys Thr Lys Pro Arg Glu Glu Gln
        275                 280                 285

Phe Asn Ser Thr Tyr Arg Val Val Ser Val Leu Thr Val Leu His Gln
    290                 295                 300

Asp Trp Leu Asn Gly Lys Glu Tyr Lys Cys Lys Val Ser Asn Lys Gly
305                 310                 315                 320

Leu Pro Ser Ser Ile Glu Lys Thr Ile Ser Lys Ala Lys Gly Gln Pro
                325                 330                 335

Arg Glu Pro Gln Val Tyr Thr Leu Pro Pro Ser Gln Glu Glu Met Thr
            340                 345                 350

Lys Asn Gln Val Ser Leu Thr Cys Leu Val Lys Gly Phe Tyr Pro Ser
        355                 360                 365

Asp Ile Ala Val Glu Trp Glu Ser Asn Gly Gln Pro Glu Asn Asn Tyr
    370                 375                 380

Lys Thr Thr Pro Pro Val Leu Asp Ser Asp Gly Ser Phe Phe Leu Tyr
385                 390                 395                 400

Ser Arg Leu Thr Val Asp Lys Ser Arg Trp Gln Glu Gly Asn Val Phe
                405                 410                 415

Ser Cys Ser Val Met His Glu Ala Leu His Asn His Tyr Thr Gln Lys
            420                 425                 430

Ser Leu Ser Leu Ser Leu Gly Lys
        435                 440

<210> 18
<211> 213
<212> PRT
<213> Artificial Sequence

<220>
<223> Light Chain Amino Acid Sequence; Anti-PD-1 mAb (BMS936558)

<400> 18

Glu Ile Val Leu Thr Gln Ser Pro Ala Thr Leu Ser Leu Ser Pro Gly
1               5                   10                  15

Glu Arg Ala Thr Leu Ser Cys Arg Ala Ser Gln Ser Val Ser Ser Tyr
            20                  25                  30

Leu Ala Trp Tyr Gln Gln Lys Pro Gly Gln Ala Pro Arg Leu Leu Ile
        35                  40                  45

Tyr Asp Ala Ser Asn Arg Ala Thr Gly Ile Pro Ala Arg Phe Ser Gly
    50                  55                  60

Ser Gly Ser Gly Thr Asp Phe Thr Leu Thr Ile Ser Ser Leu Glu Pro
65                  70                  75                  80

Glu Asp Phe Ala Val Tyr Tyr Cys Gln Gln Ser Ser Asn Trp Pro Arg
                85                  90                  95

Thr Phe Gly Gln Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Leu Ser Lys Ala Asp Tyr Glu Lys His Lys Val Tyr Ala
            180                 185                 190

Cys Glu Val Thr His Gln Gly Leu Ser Ser Pro Val Thr Lys Ser Phe
        195                 200                 205

Asn Arg Gly Glu Cys
    210

<210> 19
<211> 113
<212> PRT
<213> Artificial Sequence

<220>
<223> Heavy Chain Variable Region (VH) Amino Acid Sequence; Anti-PD-1
      mAb (BMS936558)

<400> 19

<210> 20
<211> 339
<212> DNA
<213> Artificial Sequence

<220>
<223> Heavy Chain Variable Region (VH) Nucleotide Sequence; Anti-PD-1
      mAb (BMS936558)

<400> 20
caggtgcagc tggtggagtc tgggggaggc gtggtccagc ctgggaggtc cctgagactc      60

gactgtaaag cgtctggaat caccttcagt aactctggca tgcactgggt ccgccaggct     120

ccaggcaagg ggctggagtg ggtggcagtt atttggtatg atggaagtaa aagatactat     180

gcagactccg tgaagggccg attcaccatc tccagagaca attccaagaa cacgctgttt     240

ctgcaaatga acagcctgag agccgaggac acggctgtgt attactgtgc gacaaacgac 300

gactactggg gccagggaac cctggtcacc gtctcctca 339

<210> 21
<211> 107
<212> PRT
<213> Artificial Sequence

<220>
<223> Light Chain Variable Region (VL) Amino Acid Sequence; Anti-PD-1
      mAb (BMS936558)

<400> 21

<210> 22
<211> 321
<212> DNA
<213> Artificial Sequence

<220>
<223> Light Chain Variable Region (VL) Nucleotide Sequence; Anti-PD-1
      mAb (BMS936558)

<400> 22
gaaattgtgt tgacacagtc tccagccacc ctgtctttgt ctccagggga aagagccacc 60

ctctcctgca gggccagtca gagtgttagt agttacttag cctggtacca acagaaacct 120

ggccaggctc ccaggctcct catctatgat gcatccaaca gggccactgg catcccagcc 180

aggttcagtg gcagtgggtc tgggacagac ttcactctca ccatcagcag cctagagcct 240

gaagattttg cagtttatta ctgtcagcag agtagcaact ggcctcggac gttcggccaa 300

gggaccaagg tggaaatcaa a 321

<210> 23
<211> 5
<212> PRT
<213> Artificial Sequence

<220>
<223> Heavy Chain CDR1 Amino Acid Sequence; Anti-PD-1 mAb (BMS936558)

<400> 23

Asn Ser Gly Met His
1               5

<210> 24
<211> 17
<212> PRT
<213> Artificial Sequence

<220>
<223> Heavy Chain CDR2 Amino Acid Sequence; Anti-PD-1 mAb (BMS936558)

<400> 24

Val Ile Trp Tyr Asp Gly Ser Lys Arg Tyr Tyr Ala Asp Ser Val Lys
1               5                   10                  15

Gly

<210> 25
<211> 4
<212> PRT
<213> Artificial Sequence

<220>
<223> Heavy Chain CDR3 Amino Acid Sequence; Anti-PD-1 mAb (BMS936558)

<400> 25

Asn Asp Asp Tyr
1

<210> 26
<211> 11
<212> PRT
<213> Artificial Sequence

<220>
<223> Light Chain CDR1 Amino Acid Sequence; Anti-PD-1 mAb (BMS936558)

<400> 26

Arg Ala Ser Gln Ser Val Ser Ser Tyr Leu Ala
1               5                   10

<210> 27
<211> 7
<212> PRT
<213> Artificial Sequence

<220>
<223> Light Chain CDR2 Amino Acid Sequence; Anti-PD-1 mAb (BMS936558)

<400> 27

Asp Ala Ser Asn Arg Ala Thr
1               5


<210> 28
<211> 9
<212> PRT
<213> Artificial Sequence

<220>
<223> Light Chain CDR3 Amino Acid Sequence; Anti-PD-1 mAb (BMS936558)

<400> 28

Gln Gln Ser Ser Asn Trp Pro Arg Thr
1               5


<210> 29
<211> 2106
<212> DNA
<213> Artificial Sequence

<220>
<223> Complete Homo sapiens PD-1 sequence

<400> 29
agtttccctt ccgctcacct ccgcctgagc agtggagaag gcggcactct ggtggggctg      60

ctccaggcat gcagatccca caggcgccct ggccagtcgt ctgggcggtg ctacaactgg     120

gctggcggcc aggatggttc ttagactccc cagacaggcc ctggaacccc cccaccttct     180

tcccagccct gctcgtggtg accgaagggg acaacgccac cttcacctgc agcttctcca     240

acacatcgga gagcttcgtg ctaaactggt accgcatgag ccccagcaac cagacggaca     300

agctggccgc cttccccgag gaccgcagcc agcccggcca ggactgccgc ttccgtgtca     360

cacaactgcc caacgggcgt gacttccaca tgagcgtggt cagggcccgg cgcaatgaca     420

gcggcaccta cctctgtggg gccatctccc tggcccccaa ggcgcagatc aaagagagcc     480

tgcgggcaga gctcagggtg acagagagaa gggcagaagt gcccacagcc caccccagcc     540

cctcacccag gccagccggc cagttccaaa ccctggtggt tggtgtcgtg ggcggcctgc     600

tgggcagcct ggtgctgcta gtctgggtcc tggccgtcat ctgctcccgg gccgcacgag     660

ggacaatagg agccaggcgc accggccagc ccctgaagga ggacccctca gccgtgcctg     720

tgttctctgt ggactatggg gagctggatt tccagtggcg agagaagacc ccggagcccc     780

ccgtgccctg tgtccctgag cagacggagt atgccaccat tgtctttcct agcggaatgg     840

gcacctcatc ccccgcccgc aggggctcag ccgacggccc tcggagtgcc cagccactga     900

ggcctgagga tggacactgc tcttggcccc tctgaccggc ttccttggcc accagtgttc     960

tgcagaccct ccaccatgag cccgggtcag cgcatttcct caggagaagc aggcagggtg    1020

caggccattg caggccgtcc aggggctgag ctgcctgggg gcgaccgggg ctccagcctg 1080

cacctgcacc aggcacagcc ccaccacagg actcatgtct caatgcccac agtgagccca 1140

ggcagcaggt gtcaccgtcc cctacaggga gggccagatg cagtcactgc ttcaggtcct 1200

gccagcacag agctgcctgc gtccagctcc ctgaatctct gctgctgctg ctgctgctgc 1260

tgctgctgcc tgcggcccgg ggctgaaggc gccgtggccc tgcctgacgc cccggagcct 1320

cctgcctgaa cttgggggct ggttggagat ggccttggag cagccaaggt gcccctggca 1380

gtggcatccc gaaacgccct ggacgcaggg cccaagactg ggcacaggag tgggaggtac 1440

atggggctgg ggactcccca ggagttatct gctccctgca ggcctagaga agtttcaggg 1500

aaggtcagaa gagctcctgg ctgtggtggg cagggcagga aacccctccc acctttacac 1560

atgcccaggc agcacctcag gccctttgtg gggcagggaa gctgaggcag taagcgggca 1620

ggcagagctg gaggcctttc aggccagcca gcactctggc ctcctgccgc cgcattccac 1680

cccagcccct cacaccactc gggagaggga catcctacgg tcccaaggtc aggagggcag 1740

ggctggggtt gactcaggcc cctcccagct gtggccacct gggtgttggg agggcagaag 1800

tgcaggcacc tagggccccc catgtgccca ccctgggagc tctccttgga acccattcct 1860

gaaattattt aaaggggttg gccgggctcc caccagggcc tgggtgggaa ggtacaggcg 1920

ttcccccggg gcctagtacc cccgcgtggc ctatccactc ctcacatcca cacactgcac 1980

ccccactcct ggggcagggc caccagcatc caggcggcca gcaggcacct gagtggctgg 2040

gacaagggat cccccttccc tgtggttcta ttatattata attataatta aatatgagag 2100

catgct 2106

<210> 30
<211> 1344
<212> DNA
<213> Artificial Sequence

<220>
<223> Heavy Chain Nucleotide Sequence; Anti-LAG-3 mAb (BMS-986016)

<400> 30
caggtgcagc tacagcagtg gggcgcagga ctgttgaagc cttcggagac cctgtccctc 60

acctgcgctg tctatggtgg gtccttcagt gattactact ggaactggat ccgccagccc 120

ccagggaagg ggctggagtg gattggggaa atcaatcatc gtggaagcac caactccaac 180

ccgtccctca agagtcgagt caccctatca ctagacacgt ccaagaacca gttctccctg 240

aagctgaggt ctgtgaccgc cgcggacacg gctgtgtatt actgtgcgtt tggatatagt 300

gactacgagt acaactggtt cgacccctgg ggccagggaa ccctggtcac cgtctcctca 360

gctagcacca agggcccatc cgtcttcccc ctggcgccct gctccaggag cacctccgag 420

agcacagccg ccctgggctg cctggtcaag gactacttcc ccgaaccggt gacggtgtcg 480

tggaactcag gcgccctgac cagcggcgtg cacaccttcc cggctgtcct acagtcctca 540

ggactctact ccctcagcag cgtggtgacc gtgccctcca gcagcttggg cacgaagacc 600

tacacctgca acgtagatca caagcccagc aacaccaagg tggacaagag agttgagtcc 660

aaatatggtc ccccatgccc accatgccca gcacctgagt tcctgggggg accatcagtc 720

ttcctgttcc ccccaaaacc caaggacact ctcatgatct cccggacccc tgaggtcacg 780

tgcgtggtgg tggacgtgag ccaggaagac cccgaggtcc agttcaactg gtacgtggat 840

ggcgtggagg tgcataatgc caagacaaag ccgcgggagg agcagttcaa cagcacgtac 900

cgtgtggtca gcgtcctcac cgtcctgcac caggactggc tgaacggcaa ggagtacaag 960

tgcaaggtct ccaacaaagg cctcccgtcc tccatcgaga aaaccatctc caaagccaaa 1020

gggcagcccc gagagccaca ggtgtacacc ctgcccccat cccaggagga gatgaccaag 1080

aaccaggtca gcctgacctg cctggtcaaa ggcttctacc ccagcgacat cgccgtggag 1140

tgggagagca atgggcagcc ggagaacaac tacaagacca cgcctcccgt gctggactcc 1200

gacggctcct tcttcctcta cagcaggcta accgtggaca agagcaggtg gcaggagggg 1260

aatgtcttct catgctccgt gatgcatgag gctctgcaca accactacac acagaagagc 1320

ctctccctgt ctctgggtaa atga 1344

<210> 31
<211> 645
<212> DNA
<213> Artificial Sequence

<220>
<223> Light Chain Nucleotide Sequence; Anti-LAG-3 mAb (BMS-986016)

<400> 31
gaaattgtgt tgacacagtc tccagccacc ctgtctttgt ctccagggga aagagccacc 60

ctctcctgca gggccagtca gagtattagc agctacttag cctggtacca acagaaacct 120

ggccaggctc ccaggctcct catctatgat gcatccaaca gggccactgg catcccagcc 180

aggttcagtg gcagtgggtc tgggacagac ttcactctca ccatcagcag cctagagcct 240

gaagattttg cagtttatta ctgtcagcag cgtagcaact ggcctctcac ttttggccag 300

gggaccaacc tggagatcaa acgtacggtg gctgcaccat ctgtcttcat cttcccgcca 360

tctgatgagc agttgaaatc tggaactgcc tctgttgtgt gcctgctgaa taacttctat 420

cccagagagg ccaaagtaca gtggaaggtg gataacgccc tccaatcggg taactcccag 480

gagagtgtca cagagcagga cagcaaggac agcacctaca gcctcagcag caccctgacg 540

ctgagcaaag cagactacga gaaacacaaa gtctacgcct gcgaagtcac ccatcagggc 600

ctgagctcgc ccgtcacaaa gagcttcaac aggggagagt gttag 645

<210> 32
<211> 6
<212> PRT
<213> Artificial Sequence

<220>
<223> Motif

<400> 32

Met Tyr Pro Pro Pro Tyr
1               5


<210> 33
<211> 450
<212> PRT
<213> Artificial Sequence

<220>
<223> Heavy Chain Amino Acid Sequence; Anti-ICOS mAb (BMS986226)

<400> 33

Glu Val Gln Leu Val Glu Ser Gly Gly Gly Leu Val Lys Pro Gly Gly
1               5                   10                  15

Ser Leu Arg Leu Ser Cys Ala Ala Ser Gly Phe Thr Phe Ser Asp Tyr
            20                  25                  30

Phe Met His Trp Val Arg Gln Ala Pro Gly Lys Gly Leu Glu Trp Val
        35                  40                  45

Gly Val Ile Asp Thr Lys Ser Phe Asn Tyr Ala Thr Tyr Tyr Ser Asp
    50                  55                  60

Leu Val Lys Gly Arg Phe Thr Ile Ser Arg Asp Asp Ser Lys Asn Thr
65                  70                  75                  80

Leu Tyr Leu Gln Met Asn Ser Leu Lys Thr Glu Asp Thr Ala Val Tyr
                85                  90                  95

Tyr Cys Thr Ala Thr Ile Ala Val Pro Tyr Tyr Phe Asp Tyr Trp Gly
            100                 105                 110

Gln Gly Thr Leu Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205

Lys Pro Ser Asn Thr Lys Val Asp Lys Arg Val Glu Pro Lys Ser Cys
    210                 215                 220

Asp Lys Thr His Thr Cys Pro Pro Cys Pro Ala Pro Glu Leu Leu Gly
225                 230                 235                 240

Gly Pro Ser Val Phe Leu Phe Pro Pro Lys Pro Lys Asp Thr Leu Met
                245                 250                 255

Ile Ser Arg Thr Pro Glu Val Thr Cys Val Val Val Asp Val Glu His
            260                 265                 270

Glu Asp Pro Glu Val Lys Phe Asn Trp Tyr Val Asp Gly Val Glu Val
        275                 280                 285

His Asn Ala Lys Thr Lys Pro Arg Glu Glu Gln Tyr Asn Ser Thr Tyr
    290                 295                 300

Arg Val Val Ser Val Leu Thr Val Leu His Gln Asp Trp Leu Asn Gly
305                 310                 315                 320

Lys Glu Tyr Lys Cys Lys Val Ser Asn Lys Ala Leu Pro Ala Pro Ile
                325                 330                 335

Glu Lys Thr Ile Ser Lys Ala Lys Gly Gln Pro Arg Glu Pro Gln Val
            340                 345                 350

Tyr Thr Leu Pro Pro Ser Arg Glu Glu Met Thr Lys Asn Gln Val Ser
        355                 360                 365

Leu Thr Cys Leu Val Lys Gly Phe Tyr Pro Ser Asp Ile Ala Val Glu
    370                 375                 380

Trp Glu Ser Asn Gly Gln Pro Glu Asn Asn Tyr Lys Thr Thr Pro Pro
385                 390                 395                 400

Val Leu Asp Ser Asp Gly Ser Phe Phe Leu Tyr Ser Lys Leu Thr Val
                405                 410                 415

Asp Lys Ser Arg Trp Gln Gln Gly Asn Val Phe Ser Cys Ser Val Met
            420                 425                 430

His Glu Ala Leu His Asn His Tyr Thr Gln Lys Ser Leu Ser Leu Ser
        435                 440                 445

Pro Gly

    450

<210> 34
<211> 213
<212> PRT
<213> Artificial Sequence

<220>
<223> Light Chain Amino Acid Sequence; Anti-ICOS mAb (BMS986226)

<400> 34

Asp Ile Gln Met Thr Gln Ser Pro Ser Ser Leu Ser Ala Ser Val Gly
1               5                   10                  15

Asp Arg Val Thr Ile Thr Cys Gln Ala Ser Gln Asp Ile Ser Asn Tyr
            20                  25                  30

Leu Ser Trp Tyr Gln Gln Lys Pro Gly Lys Ala Pro Lys Leu Leu Ile
        35                  40                  45

Tyr Tyr Thr Asn Leu Leu Ala Glu Gly Val Pro Ser Arg Phe Ser Gly
    50                  55                  60

Ser Gly Ser Gly Thr Asp Phe Thr Phe Thr Ile Ser Ser Leu Gln Pro
65                  70                  75                  80

Glu Asp Ile Ala Thr Tyr Tyr Cys Gln Gln Tyr Tyr Asn Tyr Arg Thr
                85                  90                  95

Phe Gly Pro Gly Thr Lys Val Asp Ile Lys Arg Thr Val Ala Ala Pro
            100                 105                 110

Ser Val Phe Ile Phe Pro Pro Ser Asp Glu Gln Leu Lys Ser Gly Thr
        115                 120                 125

Ala Ser Val Val Cys Leu Leu Asn Asn Phe Tyr Pro Arg Glu Ala Lys
    130                 135                 140

Val Gln Trp Lys Val Asp Asn Ala Leu Gln Ser Gly Asn Ser Gln Glu
145                 150                 155                 160

Ser Val Thr Glu Gln Asp Ser Lys Asp Ser Thr Tyr Ser Leu Ser Ser
                165                 170                 175

Thr Leu Thr Leu Ser Lys Ala Asp Tyr Glu Lys His Lys Val Tyr Ala
            180                 185                 190

Cys Glu Val Thr His Gln Gly Leu Ser Ser Pro Val Thr Lys Ser Phe
        195                 200                 205

Asn Arg Gly Glu Cys

    210

<210> 35
<211> 121
<212> PRT
<213> Artificial Sequence

<220>
<223> Heavy Chain Variable Region (VH) Amino Acid Sequence; Anti-ICOS
      mAb

<400> 35

Glu Val Gln Leu Val Glu Ser Gly Gly Gly Leu Val Lys Pro Gly Gly
1               5                   10                  15

Ser Leu Arg Leu Ser Cys Ala Ala Ser Gly Phe Thr Phe Ser Asp Tyr
            20                  25                  30

Phe Met His Trp Val Arg Gln Ala Pro Gly Lys Gly Leu Glu Trp Val
        35                  40                  45

Gly Val Ile Asp Thr Lys Ser Phe Asn Tyr Ala Thr Tyr Tyr Ser Asp
    50                  55                  60

Leu Val Lys Gly Arg Phe Thr Ile Ser Arg Asp Asp Ser Lys Asn Thr
65                  70                  75                  80

Leu Tyr Leu Gln Met Asn Ser Leu Lys Thr Glu Asp Thr Ala Val Tyr
                85                  90                  95

Tyr Cys Thr Ala Thr Ile Ala Val Pro Tyr Tyr Phe Asp Tyr Trp Gly
            100                 105                 110

Gln Gly Thr Leu Val Thr Val Ser Ser
        115                 120


<210> 36
<211> 106
<212> PRT
<213> Artificial Sequence

<220>
<223> Light Chain Variable Region (VL) Amino Acid Sequence; Anti-ICOS
      mAb

<400> 36

Asp Ile Gln Met Thr Gln Ser Pro Ser Ser Leu Ser Ala Ser Val Gly
1               5                   10                  15

Asp Arg Val Thr Ile Thr Cys Gln Ala Ser Gln Asp Ile Ser Asn Tyr
            20                  25                  30

Leu Ser Trp Tyr Gln Gln Lys Pro Gly Lys Ala Pro Lys Leu Leu Ile

35                  40                  45

Tyr Tyr Thr Asn Leu Leu Ala Glu Gly Val Pro Ser Arg Phe Ser Gly
    50                  55                  60

Ser Gly Ser Gly Thr Asp Phe Thr Phe Thr Ile Ser Ser Leu Gln Pro
65                  70                  75                  80

Glu Asp Ile Ala Thr Tyr Tyr Cys Gln Gln Tyr Tyr Asn Tyr Arg Thr
                85                  90                  95

Phe Gly Pro Gly Thr Lys Val Asp Ile Lys
            100                 105

<210> 37
<211> 10
<212> PRT
<213> Artificial Sequence

<220>
<223> Heavy Chain CDR1 Amino Acid Sequence; Anti-ICOS mAb (BMS986226)

<400> 37

Gly Phe Thr Phe Ser Asp Tyr Phe Met His
1               5                   10

<210> 38
<211> 19
<212> PRT
<213> Artificial Sequence

<220>
<223> Heavy Chain CDR2 Amino Acid Sequence; Anti-ICOS mAb (BMS986226)

<400> 38

Val Ile Asp Thr Lys Ser Phe Asn Tyr Ala Thr Tyr Tyr Ser Asp Leu
1               5                   10                  15

Val Lys Gly

<210> 39
<211> 10
<212> PRT
<213> Artificial Sequence

<220>
<223> Heavy Chain CDR3 Amino Acid Sequence; Anti-ICOS mAb (BMS986226)

<400> 39

Thr Ile Ala Val Pro Tyr Tyr Phe Asp Tyr
1               5                   10

<210> 40

<211> 11
<212> PRT
<213> Artificial Sequence

<220>
<223> Light Chain CDR1 Amino Acid Sequence; Anti-ICOS mAb (BMS986226)

<400> 40

Gln Ala Ser Gln Asp Ile Ser Asn Tyr Leu Ser
1               5                   10



<210> 41
<211> 7
<212> PRT
<213> Artificial Sequence

<220>
<223> Light Chain CDR2 Amino Acid Sequence; Anti-ICOS mAb (BMS986226)

<400> 41

Tyr Thr Asn Leu Leu Ala Glu
1               5



<210> 42
<211> 8
<212> PRT
<213> Artificial Sequence

<220>
<223> Light Chain CDR3 Amino Acid Sequence; Anti-ICOS mAb (BMS986226)

<400> 42

Gln Gln Tyr Tyr Asn Tyr Arg Thr
1               5



<210> 43
<211> 8
<212> PRT
<213> Artificial Sequence

<220>
<223> Light Chain CDR3 Amino Acid Sequence; Anti-ICOS mAb (BMS986226)

<400> 43

Gln Gln Tyr Tyr Asn Tyr Arg Thr
1               5



<210> 44
<211> 525
<212> PRT
<213> Artificial Sequence

<220>
<223> Lymphocyte Activation Gene 3 Protein Amino Acid Sequence (Homo
      Sapiens, NP_002277)

<400> 44

Met Trp Glu Ala Gln Phe Leu Gly Leu Leu Phe Leu Gln Pro Leu Trp
1               5                   10                  15

Val Ala Pro Val Lys Pro Leu Gln Pro Gly Ala Glu Val Pro Val Val
            20                  25                  30

Trp Ala Gln Glu Gly Ala Pro Ala Gln Leu Pro Cys Ser Pro Thr Ile
        35                  40                  45

Pro Leu Gln Asp Leu Ser Leu Leu Arg Arg Ala Gly Val Thr Trp Gln
    50                  55                  60

His Gln Pro Asp Ser Gly Pro Pro Ala Ala Ala Pro Gly His Pro Leu
65                  70                  75                  80

Ala Pro Gly Pro His Pro Ala Ala Pro Ser Ser Trp Gly Pro Arg Pro
                85                  90                  95

Arg Arg Tyr Thr Val Leu Ser Val Gly Pro Gly Gly Leu Arg Ser Gly
            100                 105                 110

Arg Leu Pro Leu Gln Pro Arg Val Gln Leu Asp Glu Arg Gly Arg Gln
        115                 120                 125

Arg Gly Asp Phe Ser Leu Trp Leu Arg Pro Ala Arg Arg Ala Asp Ala
    130                 135                 140

Gly Glu Tyr Arg Ala Ala Val His Leu Arg Asp Arg Ala Leu Ser Cys
145                 150                 155                 160

Arg Leu Arg Leu Arg Leu Gly Gln Ala Ser Met Thr Ala Ser Pro Pro
                165                 170                 175

Gly Ser Leu Arg Ala Ser Asp Trp Val Ile Leu Asn Cys Ser Phe Ser
            180                 185                 190

Arg Pro Asp Arg Pro Ala Ser Val His Trp Phe Arg Asn Arg Gly Gln
        195                 200                 205

Gly Arg Val Pro Val Arg Glu Ser Pro His His His Leu Ala Glu Ser
    210                 215                 220

Phe Leu Phe Leu Pro Gln Val Ser Pro Met Asp Ser Gly Pro Trp Gly
225                 230                 235                 240

Cys Ile Leu Thr Tyr Arg Asp Gly Phe Asn Val Ser Ile Met Tyr Asn
                245                 250                 255

Leu Thr Val Leu Gly Leu Glu Pro Pro Thr Pro Leu Thr Val Tyr Ala
            260                 265                 270

Gly Ala Gly Ser Arg Val Gly Leu Pro Cys Arg Leu Pro Ala Gly Val
        275                 280                 285

Gly Thr Arg Ser Phe Leu Thr Ala Lys Trp Thr Pro Pro Gly Gly Gly
    290                 295                 300

Pro Asp Leu Leu Val Thr Gly Asp Asn Gly Asp Phe Thr Leu Arg Leu
305                 310                 315                 320

Glu Asp Val Ser Gln Ala Gln Ala Gly Thr Tyr Thr Cys His Ile His
                325                 330                 335

Leu Gln Glu Gln Gln Leu Asn Ala Thr Val Thr Leu Ala Ile Ile Thr
            340                 345                 350

Val Thr Pro Lys Ser Phe Gly Ser Pro Gly Ser Leu Gly Lys Leu Leu
        355                 360                 365

Cys Glu Val Thr Pro Val Ser Gly Gln Glu Arg Phe Val Trp Ser Ser
    370                 375                 380

Leu Asp Thr Pro Ser Gln Arg Ser Phe Ser Gly Pro Trp Leu Glu Ala
385                 390                 395                 400

Gln Glu Ala Gln Leu Leu Ser Gln Pro Trp Gln Cys Gln Leu Tyr Gln
                405                 410                 415

Gly Glu Arg Leu Leu Gly Ala Ala Val Tyr Phe Thr Glu Leu Ser Ser
            420                 425                 430

Pro Gly Ala Gln Arg Ser Gly Arg Ala Pro Gly Ala Leu Pro Ala Gly
        435                 440                 445

His Leu Leu Leu Phe Leu Ile Leu Gly Val Leu Ser Leu Leu Leu Leu
    450                 455                 460

Val Thr Gly Ala Phe Gly Phe His Leu Trp Arg Arg Gln Trp Arg Pro
465                 470                 475                 480

Arg Arg Phe Ser Ala Leu Glu Gln Gly Ile His Pro Pro Gln Ala Gln
                485                 490                 495

Ser Lys Ile Glu Glu Leu Glu Gln Glu Pro Glu Pro Glu Pro Glu Pro
            500                 505                 510

Glu Pro Glu Pro Glu Pro Glu Pro Glu Pro Glu Gln Leu
        515                 520                 525

<210> 45
<211> 288
<212> PRT
<213> Artificial Sequence

<220>
<223> PD-1 Amino Acid Sequence (Homo Sapiens, AAC51773.1)

<400> 45

Met Gln Ile Pro Gln Ala Pro Trp Pro Val Val Trp Ala Val Leu Gln
1               5                   10                  15

Leu Gly Trp Arg Pro Gly Trp Phe Leu Asp Ser Pro Asp Arg Pro Trp
            20                  25                  30

Asn Pro Pro Thr Phe Phe Pro Ala Leu Leu Val Val Thr Glu Gly Asp
        35                  40                  45

Asn Ala Thr Phe Thr Cys Ser Phe Ser Asn Thr Ser Glu Ser Phe Val
    50                  55                  60

Leu Asn Trp Tyr Arg Met Ser Pro Ser Asn Gln Thr Asp Lys Leu Ala
65                  70                  75                  80

Ala Phe Pro Glu Asp Arg Ser Gln Pro Gly Gln Asp Cys Arg Phe Arg
                85                  90                  95

Val Thr Gln Leu Pro Asn Gly Arg Asp Phe His Met Ser Val Val Arg
            100                 105                 110

Ala Arg Arg Asn Asp Ser Gly Thr Tyr Leu Cys Gly Ala Ile Ser Leu
        115                 120                 125

Ala Pro Lys Ala Gln Ile Lys Glu Ser Leu Arg Ala Glu Leu Arg Val
    130                 135                 140

Thr Glu Arg Arg Ala Glu Val Pro Thr Ala His Pro Ser Pro Ser Pro
145                 150                 155                 160

Arg Pro Ala Gly Gln Phe Gln Thr Leu Val Val Gly Val Val Gly Gly
                165                 170                 175

Leu Leu Gly Ser Leu Val Leu Leu Val Trp Val Leu Ala Val Ile Cys
            180                 185                 190

Ser Arg Ala Ala Arg Gly Thr Ile Gly Ala Arg Arg Thr Gly Gln Pro
        195                 200                 205

Leu Lys Glu Asp Pro Ser Ala Val Pro Val Phe Ser Val Asp Tyr Gly
    210                 215                 220

Glu Leu Asp Phe Gln Trp Arg Glu Lys Thr Pro Glu Pro Pro Val Pro
225                 230                 235                 240

Cys Val Pro Glu Gln Thr Glu Tyr Ala Thr Ile Val Phe Pro Ser Gly
                245                 250                 255

Met Gly Thr Ser Ser Pro Ala Arg Arg Gly Ser Ala Asp Gly Pro Arg
            260                 265                 270

Ser Ala Gln Pro Leu Arg Pro Glu Asp Gly His Cys Ser Trp Pro Leu
        275                 280                 285